# EUROPEAN PATENT APPLICATION

(11) **EP 4 736 919 A2**
(43) Date of publication of application: **06.05.2026**
(21) Application number: 26166895.8
(22) Date of filing: 12.01.2022
(51) Int. Cl.: A61M 16/06

(54) **PATIENT INTERFACE**

(30) Priority: 19.01.2021 US 202163139190 P; 14.06.2021 US 202163202504 P; 17.12.2021 US 202163291037 P
(62) Divisional of application: 22742939.6
(71) Applicant: Fisher & Paykel Healthcare Limited, East Tamaki, Auckland 2013 (NZ)
(72) Inventor: GORDON, James Alexander, 2013 Auckland (NZ); ZOELLNER, Sascha Kristopher, 2013 Auckland (NZ); NIHOTTE, Joseph Jules, 2013 Auckland (NZ); PERERA, Ashani Melisha, 2013 Auckland (NZ); GAO, Vicky Dan, 2013 Auckland (NZ); DUTHIE, Neil Gray, 2013 Auckland (NZ); SHAHRI, Saachi, 2013 Auckland (NZ); STRAUSS, Frederic Walter Williamson, 2013 Auckland (NZ); WHARMBY, Christopher, 2013 Auckland (NZ); GRAHAM, Ryan Anthony, 2013 Auckland (NZ); VAN SCHALKWYK, Andre, Auckland, 2013 (GB)
(74) Representative: Boult Wade Tennant LLP

(57) **Abstract**

The disclosure relates to a patient interface for non-invasive ventilation. The patient interface is configured to seal about the mouth and nares of a patient and includes an outer wall defining an interior volume which includes a first chamber having one or more oral openings to communicate gas with the mouth and a second chamber having one or more nare openings to communicate gas with the nares. The patient interface further includes a dividing wall that separates the first chamber from the second chamber. The patient interface further includes one or more flow directors which enable gas to flow into the second chamber from the first chamber or into the first chamber from the second chamber. The one or more flow directors are configured to direct the gas flow through the one or more nare openings.

## Description

### TECHNICAL FIELD

The present invention relates to a patient interface for delivering respiratory gas to a patient. In particular, the present invention relates to a non-invasive patient interface.

### BACKGROUND

One current treatment for respiratory diseases, such as thoracic restrictive diseases, acute respiratory failure, advanced neuromuscular diseases, chronic obstructive pulmonary disease (COPD - which includes emphysema, refractory asthma and chronic bronchitis), is non-invasive ventilation (NIV). There is some evidence to suggest that NIV therapy may be useful for assisting respiration after intubation, including reducing the chances of re-intubation. The NIV treatment applies a positive airway pressure to the lungs throughout the inhalation and exhalation cycle so as to splint the airways open. This improves the flow of respiratory gas into and out of the lungs.

However, one side effect of the positive pressure applied in current NIV treatments is that the therapy pressures applied can make patients uncomfortable and, therefore, less willing to undergo the treatment. A follow-on effect of the positive pressure is that it requires the patient interface to be secured firmly to the patient to avoid leakages and, thereby ensure that the pressure is maintained in the patient interface and the respiratory system. Such firm application of the interface can cause pressure sores, particularly for patients that are semi-conscious or unconscious and, therefore, are unable to provide feedback on any soreness caused by the pressure of the patient interface on their skin.

The NIV treatment gives rise to two challenges, namely compliance (the extent to which patients are willing to submit to the treatment) and pressure sores. In addition to these challenges, a further challenge for patients with obstructive respiration diseases is flushing carbon dioxide out of anatomical dead space. Specifically, the end of the exhalation cycle is characterised by a reduction in pressure of the exhaled respiratory gas. This means that the carbon dioxide-loaded respiratory gas remains in the throat, nose and mouth of the patient and is pulled back into the lungs at the commencement of the inhalation cycle. Replacing the carbon dioxide-loaded respiratory gas in these regions with respiratory gas that includes higher levels of oxygen than the carbon dioxide-loaded respiratory gas therefore assists patients in achieving sufficient respiration.

It is desirable to provide a patient interface that improves patient comfort and that reduces pressure sores.

It is also desirable to provide a patient interface that assists with flushing anatomical dead space.

### SUMMARY OF THE DISCLOSURE

The present invention will now be described by way of a set of embodiments. However, it will be appreciated that the invention may be defined by combining the features of two or more of the embodiments.

According to a first aspect, there is provided a non-invasive patient interface which is configured to seal about the mouth and nares of a patient, the patient interface including:
(a) an outer wall defining an interior volume which includes a first chamber having one or more oral openings to communicate gas with the mouth and a second chamber having one or more nare openings to communicate gas with the nares; and
(b) a dividing wall that separates the first chamber from the second chamber; and
(c) one or more flow directors which enable gas to flow into the second chamber from the first chamber and which flow directors are configured to direct the gas flow through the one or more nare openings.

The one or more flow directors may extend from the dividing wall.

The one or more flow directors may be located in a central region of the dividing wall such that each flow director is spaced from a perimeter of the dividing wall.

The one or more flow directors may extend from the dividing wall in to the first chamber or into the second chamber or into both the first and the second chambers.

The one or more flow directors may extend toward the one or more nare openings.

The dividing wall may be joined with the outer wall to separate the first chamber from the second chamber.

The dividing wall may join with the outer wall at a location spaced from the one or more nare openings.

The dividing wall may join with the outer wall across the entire width of the

The dividing wall may join with the outer wall between the at least one or more nare openings and the at least one or more oral openings.

The dividing wall may join with the outer wall closer to the one or more oral openings than the one or more nare openings.

The outer wall may include a wall portion between the at least one or more nare openings and the at least one or more oral openings and the join between the dividing wall and the wall portion may be located in a lower half of the wall portion. The width of the wall portion may be defined between the laterally outermost ends of the one or more nare openings.

The one or more flow directors may define a gas flow path from the first chamber to the second chamber and the flow directors surround the gas flow path.

The one or more flow directors may have a sealed joint with the dividing wall.

Each of the one or more flow directors may have a second opening in the second chamber recessed from the one or more nare openings.

Each of the one or more flow directors may have a second opening within the second chamber and the second opening is spaced from the one or more nare openings.

The second opening is defined by a rim that may be contoured so that at least a portion of the rim has a substantially consistent spacing from the one or more nare openings.

The portion of the rim that may be substantially consistently spaced from the one or more nare openings is adjacent to the outer wall between the at least one or more nare openings and the at least one or more oral openings.

The rim of each second opening may extend further from the dividing wall at a laterally outer side of the flow director than the rim extends from the dividing wall at a laterally inner side of the flow director.

The rim of each second opening may be recessed further from the nare opening at a laterally inner side of the flow director than the rim is recessed at the laterally outer side of the flow director.

The rim of each second opening may be furthest recessed from the nare opening at a point that is between the laterally inner side of the flow director and the laterally outer side of the flow director.

At least a portion of the rim of the second opening may be concentric with at least a portion of the rim of the nare opening.

The patient interface may include at least two flow directors that are spaced apart by a gap through which respiratory gas from the first chamber and exhaled respiratory gas from the nares can flow into the second chamber.

The rim of each flow director may extend from the dividing wall less on the same side as the gap than the rim extends on the side remote from the gap.

The flow directors may be configured with the lateral outer side of the rims adjacent to a lateral rim of the nare opening having regard to a direction of gas flow from the flow director.

The or each flow director may be configured with the lateral outer side of the respective rim adjacent to a lateral rim of the nare opening having regard to a direction of gas flow from the respective flow director.

The flow directors may be configured with the lateral outer side of the rim aligned with a lateral rim of the nare opening having regard to a direction of gas flow from the flow director.

Alternatively, each of the one or more flow directors may have an second opening flush with the one or more nare openings.

The one or more flow directors may be spaced from the outer wall.

The one or more flow directors may be linked to the outer wall.

According to a second aspect, there is provided a non-invasive patient interface which is configured to seal about the mouth and nares of a patient, the patient interface including:
(a) an outer wall defining an interior volume of the patient interface, the outer wall having a patient-engagement surface including one or more oral openings which communicate gas with the mouth and one or more nare openings which communicate gas with the nares; and
(b) a dividing wall that separates the interior volume into a first chamber having the one or more oral openings and a second chamber having the one or more nare openings; and
(c) one or more flow directors extending from the dividing wall which enable gas to flow into the second chamber from the first chamber and which flow directors are configured to direct the gas flow through the one or more nare openings; and
wherein the flow directors are spaced apart by a spacing element which maintains a spacing between the flow directors.

Each flow director may have an second opening defined by an second opening rim and wherein the second opening rims are spaced from the patient-engagement surface.

The second opening rims may be recessed from the patient-engagement surface

Each flow director may comprise a body defining a flow channel which extends between the second opening in the second chamber and an inlet which opens into the first chamber and wherein the body is spaced from the patient-engagement surface.

The spacing element may be disposed between the flow directors.

The spacing element may be linked to the dividing wall.

The spacing element may link the flow directors to each other.

The spacing element may comprise a rib or web.

The spacing element may be spaced from the outer wall of the patient interface.

The spacing element may include a concave curved surface extending between the flow directors.

The features disclosed in respect of the first aspect regarding the dividing wall and the flow directors are equally applicable to this aspect.

In a third aspect, there is provided a non-invasive patient interface which is configured to seal about the mouth and nares of a patient, the patient interface including:
(a) an outer wall defining an interior volume of the patient interface, the outer wall having one or more oral openings which communicate gas with the mouth and one or more nare openings which communicate gas with the nares;
(b) a dividing wall that separates the interior volume into a first chamber having the one or more oral openings and a second chamber having the one or more nare openings; and
wherein the dividing wall includes one or more spaced apart flow directors which enable gas to flow into the second chamber from the first chamber and which flow directors are configured to direct the gas flow through the nare openings.

The one or more spaced apart flow directors may be linked to the outer wall.

The patient interface may include at least two flow directors that are spaced apart by a gap through which respiratory gas from the first chamber and exhaled respiratory gas from the nares can flow into the second chamber.

Each of the one or more flow directors may have a second opening within the second chamber and afirst opening which opens into the first chamber.

Each flow director may define an independent gas flow path.

The body of one or more of the flow directors may be linked to the outer wall so that the gas flow path is defined in part by the body and in part by the outer wall.

The one or more flow directors may be linked to the outer wall at or adjacent to the one or more nare openings.

The one or more flow directors may be linked to the outer wall laterally outwardly of the one or more nare openings.

The one or more flow directors may be linked to the outer wall distally of the one or more nare openings.

The one or more flow directors may be linked to the outer wall in the first chamber.

The one or more flow directors may be linked to the outer wall in the first chamber and in the second chamber.

The body of one or more of the flow directors may be linked to the outer wall between the at least one or more nare openings and the at least one or more oral openings.

The body of one or more of the flow directors may be linked to the outer wall on a side of the one or nare openings that is opposite to the wall portion.

The body of one or more of the flow directors may be linked to the outer wall at or adjacent to the one or more nare openings.

The patient interface may include at least two flow directors that are spaced apart and part of each of the flow director bodies joins with the outer wall on a laterally outer side of a lateral rim of the one or more nare openings and another part of each of the flow director bodies may join with the outer wall on a laterally inwardly side of the lateral rim of the one or more nare openings.

Each body may have a lateral wall and a medial wall both of which extend from spaced apart locations at the wall portion and which meet each other away from the wall portion.

The lateral wall and the medial wall may extend from the dividing wall to meet with the outer wall.

The lateral wall may join with the outer wall on a laterally outer side of the lateral rim of the one or more nare openings so that the rim of the at least one or more nare openings forms part of the rim of the flow director.

The medial wall may join with the outer wall on a laterally inward side of the lateral rim of the one or more nare openings so that part of the medial wall forms part of the rim of the flow director.

The rim of the second opening formed by the medial wall may be closer to the dividing wall than the rim of the second opening formed by the lateral rim of the one or more nare openings.

The body may be linked to the outer wall by a linking element.

The second opening from the one or more flow directors may be defined in part by the at least one or more nare openings.

The second opening of each flow director may be configured to direct respiratory gas laterally inwardly.

The features disclosed in respect of the first aspect are equally applicable to the second and third aspects. Additionally, the features disclosed in respect of the second aspect are equally applicable to the third aspect. Furthermore, the application of features from one aspect does not exclude application of features from another aspect.

The following disclosure applies to each of the first, second and third aspects disclosed above.

The dividing wall may include one or more preferential deformation regions to accommodate at least some deformation force created by patient contact with the patient-engagement surface in preference to deformation of the flow directors.

The flow directors may be spaced from the one or more preferential deformation regions.

The one or more preferential deformation regions may decouple one portion of the dividing wall from another portion of the dividing wall such that a force applied to one portion is greater than the force experienced by the decoupled portion.

The preferential deformation region may decouple one portion of the dividing wall from another portion of the dividing wall such that the two portions can move relative to each other.

The two portions of the dividing wall may be shaped to resist deformation.

The outer wall or the dividing wall may include deformation resistant sections that translate deformation forces into the preferential deformation regions such that deformation of the dividing wall is substantially confined to the preferential deformation regions.

The one or more preferential deformation regions may be interposed between the flow directors and a resilient region.

The one or more preferential deformation regions may comprise a first resilient region and a deformation panel disposed between the first resilient region and the flow directors.

The deformation panel may have a thickness that is less than the thickness of the first resilient region.

The deformation panel may comprise a first wall projecting from the first resilient region and a second wall connecting the first wall with a second resilient region of the dividing wall that includes the one or more flow directors and wherein the first and second walls meet along a linking line.

The first and second walls may be adapted to deform in a predetermined sequence.

The predetermined sequence may include the first wall being folded towards the first resilient region.

The predetermined sequence may include the second wall buckling to accommodate the reduction in spacing between the first resilient region and the region of the dividing wall that includes the one or more flow directors.

The second wall may be configured to induce the buckling when the distance between the region of the dividing wall that includes the one or more flow directors and the linking line is less than the length of the second wall.

The second wall may have a curved profile from the linking line to the region of the dividing wall that includes the one or more flow directors to induce buckling of the second wall.

The second wall may increase in thickness from the connecting portion to the second resilient region to cause initial folding of the first wall during deformation and subsequent buckling in the second wall.

The deformation panel may have a wall thickness that is selected to induce deformation of the deformation panel in preference to the first and second resilient regions.

The first resilient region may have a thickness that is at least three times the thickness of the first wall.

The second wall may have a profile from the first wall to the region of the dividing wall that includes the one or more flow directors to induce a rolling movement in the second wall to accommodate deformation in the deformation region.

The second wall may increase in thickness from the first wall to the flow director region to cause initial folding of the first wall during deformation and subsequent rolling in the second wall starting from an intersection between the second wall and the first wall.

A notional line extending from an intersection line between the first resilient region and the first wall may converge at a pivot point with another notional line extending along the linking line between the first wall and the second wall.

The first resilient region may comprise a first thickened region of the dividing wall.

The first thickened region may comprise a resilient lip adjoining the deformation panel.

The deformation of the one or more preferential deformation regions may involve a reduction in the spacing between the first resilient region and the region of the dividing wall that includes the one or more flow directors and may involve an associated deformation of the deformation panel to accommodate the reduction in spacing.

The patient interface may include one or more deformation resistant sections that are configured to bear at least some deformation forces applied to the patient-engagement surface such that the one or more flow directors bear less of the deformation forces than the deformation resistant sections.

The one or more deformation resistant sections may be configured to reduce the extent to which the flow directors deform due to deformation forces applied to the patient-engagement surface.

The flow directors or dividing wall may include the one or more deformation resistant sections.

The one or more deformation resistant sections may increase the resistance to deformation of a region of the dividing wall that includes the one or more flow directors.

The dividing wall may include one or more deformation resistant sections between the patient-engagement surface and the preferential deformation region.

The one or more deformation resistant sections may be in the region of the dividing wall that includes the one or more flow directors.

One or more of the deformation resistant sections may comprise ribs associated with the dividing wall.

One or more of the deformation resistant sections may be integrally formed with the dividing wall.

One or more of the deformation resistant sections may comprise portions of the dividing wall that have a wall thickness that is greater than the wall thickness of other portions of the dividing wall.

One or more of the deformation resistant sections may be configured to direct deformation forces that are applied to the patient-engagement surface into the preferential deformation region.

One or more of the deformation resistant sections may extend from or adjacent to the patient-engagement surface and extend within the region of the dividing wall that includes the one or more flow directors to direct deformation forces into the preferential deformation region.

One or more of the deformation resistant sections may be wider at an end at or adjacent to the patient-engagement surface than at an end remote from the patient-engagement surface.

One or more of the deformation resistant sections may comprise a deformation translation rib formed on an underside of the dividing wall.

One or more of the deformation resistant sections may be configured to cause the flow directors to track deformation of the patient-engaging surface.

One or more of the deformation resistant sections may be configured on the underside and topside of the dividing wall to overlap at least in part.

One or more of the deformation resistant sections may be located where the flow directors join with the dividing wall.

One or more of the deformation resistant sections which are located where the flow directors join with the dividing wall may be linked to the patient-engaging surface.

One or more of the deformation resistant sections which are located where the flow directors join with the dividing wall may be linked to the patient-engaging surface by one or more other deformation resistant sections.

One or more of the deformation resistant sections which are located where the flow directors join with the dividing wall may reinforce the region of the dividing wall that includes the one or more flow directors so that at least some of the deformation force applied to the patient-engaging surface is transferred to the preferential deformation region.

One or more of the deformation resistant sections which are located where the flow directors join with the dividing wall may be disposed on the topside or the underside of the dividing wall and may be linked to the patient-engaging surface by one or more other deformation resistant sections disposed on the underside or the topside, respectively.

One or more of the deformation resistant sections may overlap with the spacing element so that the flow directors track movement of the one or more deformation resistant sections which overlap with the spacing element.

The deformation resistant sections which overlap with the spacing element may extend substantially orthogonally with respect to each other.

One or more of the deformation resistant sections may be configured on the underside and topside of the dividing wall to transfer deformation of the patient-engaging surface into deformation of the preferential deformation region.

The deformation resistant section may be configured to overlap at least in part with another of the one or more deformation resistant sections that are located where the flow directors meet the dividing wall.

The flow directors may be configured to accelerate respiratory gas from the first opening to the second opening.

The second openings of the flow directors may have a combined area that is less than the area of the one or more nare openings.

The second openings of the flow directors may be disposed at laterally outer sides of the one or more nare openings.

Each flow director may comprise a body which has a cross-sectional area that reduces in the direction from the inlet to the outlet.

Each flow director may comprise a body which has a cross-sectional area that is substantially constant from the inlet to the outlet.

The body may include an lower body portion and a upper body portion and wherein the upper body portion has a cross-sectional area that is less than the cross-sectional profile of the lower body portion.

The lower body portion may have a conical profile.

The lower body may have a constant profile.

The upper body portion may have a conical profile.

The upper body portion may have a circular, elliptical or oval-shaped profile in cross-section generally parallel to the dividing wall where the flow director is located.

The body may have a conical profile or a stepped profile in cross-section generally orthogonal to the dividing wall where the flow director is located.

The body may have a tapering profile in cross-section generally orthogonal to the dividing wall where the flow director is located.

The patient interface may have an inlet to the first chamber for respiratory gas.

The patient interface may have an exhaust vent that is configured to communicate respiratory gas from the patient interface to externally of the patient interface.

The exhaust vent may be configured to communicate respiratory gas from the second chamber to externally of the patient interface.

The exhaust vent may be configured to communicate respiratory gas from the first chamber and the second chamber to externally of the patient interface.

The outer wall may be provided by a cushion module comprising a resilient seal member and a housing and wherein the seal member and the housing together form the first and second chambers.

The seal member may include the patient-engagement surface.

The inlet may be formed in the housing

The exhaust vent may be formed in the housing

The dividing wall may be disposed between the inlet and the exhaust vent

The dividing wall may be disposed between the one or more oral openings and the one or more nare openings.

The seal member may include a nare-sealing portion that includes the one or more nare openings and the seal member further includes a mouth-sealing portion that includes the one or more oral openings.

The seal member may be a resilient material and may be connected to a mask housing to form a unitary structure.

The seal member may be a resilient material and may be mechanically locked to the mask housing to form a unitary structure.

The seal member may be over moulded onto the mask housing to mechanically lock and/or chemically bond with the mask housing.

The patient interface may further include a mask frame.

The mask frame may be removably connectable to the mask housing.

The mask frame may be removably connectable by co-operable snap-fit formations on the mask housing and on the mask frame.

The mask frame may be permanently connectable to the housing by co-operable formations on the mask housing and the mask frame.

The mask frame may be permanently connectable to the housing by ultrasonically welding the mask housing and the mask frame.

The mask frame and the mask housing may be integrally formed.

The mask frame or the mask housing may further comprise headgear connectors for connecting a headgear to the patient interface.

The patient interface may further include a conduit connector and a rotator cuff that rotatably links the conduit connector to the mask frame to form a flow path for respiratory gas from a conduit to the first chamber.

In a fourth aspect, there is provided a non-invasive patient interface which is configured to seal about the mouth and nares of a patient, the patient interface including:
(a) an outer wall defining an interior volume which includes a first chamber having one or more oral openings to communicate gas with the mouth and a second chamber having one or more nare openings to communicate gas with the nares; and
(b) a dividing wall that separates the first chamber from the second chamber; and
(c) one or more flow directors which enable gas to flow into the second chamber from the first chamber or into the first chamber from the second chamber.

The one or more flow directors enable gas to flow into the second chamber from the first chamber and are configured to direct the gas flow through the one or more nare openings.

The dividing wall is configured to reduce movement of the flow directors toward each other.

The dividing wall is configured to reduce collapse of the flow directors.

The dividing wall is configured to reduce buckling of the flow directors.

The dividing wall is configured to reduce collapse or buckling of the flow directors inwardly toward each other.

The dividing wall includes lateral side portions which are disposed laterally outwardly of the flow directors and which join with the outer wall.

The lateral side portions join with the outer wall at a position that is spaced from the top of the outer wall.

The lateral side portions join the outer wall at a level below the lowest level of the one or more nasal apertures having regard to an upright orientation of the patient interface.

The lateral side portions join the outer wall along a line that extends at least partly above and partly below the lowest level of the one or more nasal apertures and that does not extend above the highest level of the one or more nasal apertures, having regard to an upright orientation of the patient interface.

Part of the lateral side portions join the outer wall along a line that extends between the highest and lowest levels of the one or more nasal apertures and that does not extend above the highest level of the one or more nasal apertures, having regard to an upright orientation of the patient interface.

Part of the lateral side portions join the outer wall along a line that extends above the highest level of the one or more nasal apertures, having regard to an upright orientation of the patient interface.

The lateral side portions join the outer wall at a level lower than or substantially equal to an uppermost level of the flow directors, having regard to an upright orientation of the patient interface.

The dividing wall includes a deformation region that is configured to deform in preference to the flow directors.

The deformation region is configured to deform also in preference to the lateral side portions.

The dividing wall is configured to reduce laterally inward travel of the lateral side portions upon deformation of the deformation region.

The flow directors are disposed between a patient-contact portion of the outer wall and the deformation region and the lateral side portions are disposed laterally outwardly of the flow directors.

The lateral side portions include parts of the deformation region that are disposed laterally outwardly of the flow directors.

The lateral side portions are proximal of the deformation region.

The lateral side portions join, at least in part, with portions of the outer wall which have a wall thickness that is greater than the wall thickness of patient-contact portions of the outer wall.

The lateral side portions join, at least in part, with portions of the outer wall which have a wall thickness that is selected to resist deformation of the outer wall.

The outer wall comprises a housing that forms at least part of a coupling to a gas supply and a seal member and wherein the dividing wall extends at least partly along a notional line that is contiguous with a line extending across the top of the housing, having regard to an upright orientation of the patient interface.

The inclination of the lateral side portions where they join with the outer wall, having regard to an upright orientation of the patient interface, is horizontal or is an acute angle relative to horizontal.

The inclination of the lateral side portions where they join with the outer wall, having regard to an upright orientation of the patient interface, is horizontal or is less than 45° relative to horizontal.

The inclination of the lateral side portions where they join with the outer wall, having regard to an upright orientation of the patient interface, is horizontal or is less than 30° relative to horizontal.

The inclination of the lateral side portions where they join with the outer wall, having regard to an upright orientation of the patient interface, is horizontal or is less than 15° relative to horizontal.

The lateral side wall joins with the outer wall at an angle in the second chamber that is obtuse.

Each flow director has a first opening to the first chamber and a second opening to the second chamber.

The first opening has a shape with a major axis which is longer than an orthogonal minor axis.

The major axis is within a plane of the dividing wall and is oriented at least 45° from a vertical mid-plane through the patient interface.

The major axes of the respective first openings intersect at a point that is on a proximal side of a notional straight line that passes through the centres of the first openings.

The major axes of the respective first openings intersect at a point that is proximal of a patient-contact portion of the outer wall.

An angle between the major axes where they intersect is less than 180°.

The angle between the major axes where they intersect is in the range of 45° to less than 180°.

The angle between the major axes where they intersect is in the range of 90° to 150°.

The angle between the major axes where they intersect is in the range of 110° to 150°.

The major axes of the respective first openings intersect at a point that is on a distal side of a notional straight line that passes through the centres of the first openings.

The first openings are disposed between the deformation region and a patient-contact portion of the outer wall and extend a distance that is less than the distance between the patient-contact portion of the outer wall portion and the deformation region.

The first openings are disposed between the deformation region and a patient-contact portion of the outer wall between the one or more nare openings and the one or more oral openings and extend a distance that is less than the distance between the patient-contact portion of the outer wall portion and the deformation region.

The first openings are disposed on the dividing wall.

The flow director forming each first opening is spaced from the deformation region.

Each first opening has an elliptical shape.

The orientation of the major and minor axes is retained throughout the flow director.

The dimension of the major axis or the minor axis or both major and minor axes at the second opening are different from the dimension of the major axis or the minor axis or both major and minor axes at the first opening.

The dimension of the major axis or the minor axis or both major and minor axes at the second opening are less than the dimension of the major axis or the minor axis or both major and minor axes at the first opening.

The dimension of the major axis or the minor axis or both major and minor axes vary through the flow director.

The dimension of the major axis or the minor axis or both major and minor axes decrease from the first opening to the second opening.

The dimension of the major axis or the minor axis or both major and minor axes vary to define a constriction.

The second opening of each flow director is spaced from the outer wall to enable gas flow between the first chamber and the second chamber, and wherein the second openings are positioned such that a gas stream flowing from the first chamber into the second chamber through the flow directors would impinge at least partly on the outer wall when the patient interface is not fitted to a patient.

The area, shape or shape and area of the nare openings change when the patient interface is fitted to a patient.

The area, shape or shape and area of the one or more nare openings change when the patient interface is fitted to a patient such that a gas stream passing through the flow director would be directed through the one or more nare openings without impinging upon the outer wall.

The flow directors and the outer wall are arranged such that the outer wall extends over part of each second opening when the patient interface is not fitted.

The outer wall extends over part of the second openings when viewed from vertically above a mid-point between the second openings, having regard to an upright orientation of the patient interface, when the patient interface is not fitted to a patient.

The part of the second opening over which the outer wall extends is a laterally outer part of the second opening.

At least part of each second opening is outside a notional columnar volume having the profile of and extending vertically through the one or more nare openings, having regard to an upright orientation of the patient interface, when the patient interface is not fitted to a patient.

At least part of each second opening is obscured by the outer wall when the patient interface is viewed from vertically above a mid-point between the second openings having regard to an upright orientation of the patient interface when the patient interface is not fitted to a patient.

When the patient interface is fitted to a patient, laterally outer edges of the one or more nare openings are disposed laterally outwardly of the second openings of the respective flow directors.

The outer wall is configured to enable the one or more nare openings to extend laterally outwardly.

The outer wall is configured to enable the one or more nare openings to extend laterally outwardly so that the outer wall does not extend over part of each second opening when the patient interface is viewed from vertically above a mid-point between the second openings having regard to an upright orientation of the patient interface when the patient interface is fitted to a patient.

The patient-contact surface of the outer wall is configured to enable the outer wall which extends over the second openings of the flow directors to shift laterally outwardly so that the outer wall no longer extends over the second openings when the patient interface is fitted to a patient.

The patient-contact surface is curved to receive the underside of a patient's nose when the patient interface is fitted to a patient and the curvature reduces when the patient interface is fitted.

The patient-contact surface includes a valley-shape configured to receive the underside of a patient's nose and a floor of the valley-shape, which includes the one or more nare opening, adopts a flatter valley-shape when the patient interface is fitted to a patient.

The dividing wall includes the flow directors which enable gas flow between the first chamber and the second chamber, each flow director has a base which joins with the dividing wall and which defines a first opening from the first chamber, a body extending from the base, a rim remote from the base and which rim defines a second opening which opens into the second chamber and a gas flow channel extending from the first opening in the base to the second opening.

The body includes a reinforced segment that is configured to resist deformation of the shape of the outlet under a deformation force.

The reinforced segment extends wholly or partly around the body.

The reinforced segment has a wall thickness that is greater than the wall thickness of other parts of the body.

The reinforced segment comprises a rib.

The body is formed of a first material and the reinforced segment is formed from a second material which is different from the first material and which is less compliant than the first material.

The reinforced segment extends around at least part of a distal side of the body.

The reinforced segment is located on one side of a major axis of the flow director profile generally parallel to the dividing wall.

The reinforced segment is disposed around the body at a set spacing from the rim.

The reinforced segment is disposed around the body at a set spacing from the base.

The reinforced segment is disposed around the body at a varying spacing from the rim.

The reinforcing segment is disposed adjacent to the rim.

The reinforcing segment is disposed on an exterior of the body.

The body includes a lower body portion and an upper body portion.

The reinforced segment extends wholly or partly around the lower body portion.

The reinforced segment extends wholly or partly around the upper body portion.

The reinforced segment extends from the lower body portion and at least partly around the upper body portion.

The upper body portion has a wall thickness that is less than the wall thickness of the lower body portion.

The base of each flow director is elongate in the lateral direction away from a vertical mid-plane through the patient interface, having regard to an upright orientation of the patient interface, the body is off-set from a lateral centerline through the base so that the body is closer to the laterally inward side of the base than the laterally outward side of the base.

The laterally-outer side of the base extends laterally outwardly further from the body than a laterally-inward side of the base extends laterally inwardly from the body.

The laterally-outer side of the base extends laterally outwardly from the body at least two times, at least three times, at least four times or at least five times further than the laterally-inward side of the base extends laterally inwardly from the body.

The base has a footprint which extends laterally beyond a corresponding lateral edge of the one or more nare openings.

The gas flow channel has a longitudinal axis that is off-set laterally inwardly from a lateral centerline through the base, which centreline is parallel to the vertical mid-plane through the patient interface, so that the gas flow channel is closer to the laterally inward side of the base than the laterally outward side of the base.

The base comprises a wall thickness that is larger than the wall thickness of the dividing wall adjacent the base.

The base comprises a thickened pad which extends laterally outwardly of the body.

The thickened pad decreases in wall thickness away from the body.

The thickened pad has a wedge-shaped profile in the laterally outward direction.

The base wraps, at least in part, around the body.

The base includes a laterally-outer side that is configured to resist displacement of the outlets toward each other.

The laterally-outer side of the base is configured as a reinforced link between the dividing wall and the body so that flexing of the dividing wall that causes displacement and changes in orientation of the body is resisted.

The laterally outer side of the base tapers from the dividing wall to the body.

The taper of the laterally-outer side of the base has a fillet.

The laterally-outer side of the base includes a radial wall thickness, having regard to the longitudinal axis of the gas flow channel that is greater than the radial wall thickness at any point on the remainder of the body.

The base of each flow director comprises a block which includes the gas flow channel and which has a thickness that resists deformation of the body.

The base is configured to resist displacement and changes in orientation of the body by transferring deformation forces imparted on the outer wall to the deformation region.

The bases of the flow directors are linked together to form a common block from which separate bodies extend.

The common block has a footprint which extends laterally beyond corresponding lateral edges of the one or more nare openings.

The common block has inclined sides which extend from the lateral side portions of the dividing wall to the bodies.

A region of the common block which is bound by the inclined sides is tapered downwardly in the distal direction.

The region is tapered to be substantially parallel to the one or more nare openings.

The inclined side of the common block comprises a fillet-shape.

The fillet-shape is continuous about the lateral side of the common block.

The fillet-shape is continuous about proximal and distal sides of the common block.

The fillet shape is continuous about proximal and distal sides of the common block, including the spacing portion.

The wall thickness of the flow director decreases from the base towards the rim.

The body includes a transition that is intermediate the base and the rim.

The transition delineates the upper body portion from the lower body portion.

The upper body portion includes a wall thickness that is less than the wall thickness of the lower body portion.

The wall thickness of the lower body portion is constant and the wall thickness of the upper body portion decreases from the transition to the rim.

The wall thickness of the body varies from the transition to the rim.

The wall thickness of the body decreases from the transition to the rim.

The decrease in wall thickness of the body from the transition to the rim is constant.

The decrease in wall thickness of the body from the transition to the rim is not constant.

The transition is inclined relative to the dividing wall.

The transition is inclined so that a distal side of the lower body portion extends further from the base than a proximal side of the lower body portion extends from the base.

A plane defined by the transition is generally parallel to the one or more nare openings, or the outer wall above the flow director, or the one or more nare openings and the outer wall above the flow director, having regard to an upright orientation of the patient interface.

The rim of the upper body portion is generally parallel to the one or more nare openings, or the outer wall above the flow director, or the one or more nare openings and the outer wall above the flow director, having regard to an upright orientation of the patient interface.

The lower body portion has a wall thickness selected to resist deformation more than the deformation region resists deformation so that the deformation region preferentially deforms in response to a deformation force applied to a patient-contact portion of the seal member.

An external surface of a distal side of the flow director has a curved profile in the direction of the longitudinal axis of the flow director.

The decrease in wall thickness of the flow director from the transition to the rim is due to the external surface of the flow director tapering inwardly toward an internal surface of the flow director.

The transition comprises, at least in part, a step which is intermediate the base and the rim.

The body below the step has a first wall thickness and the body above the step has a second wall thickness and wherein the second wall thickness is less than the first wall thickness.

The step extends around at least the distal side of the flow director.

The step extends around the distal and laterally outer sides of the flow director.

The step extends substantially all the way around the flow director.

The step extends all the way around the flow director.

The step comprises an inclined surface which connects the lower body portion to the upper body portion.

The step is formed on the exterior of the flow director.

Having regard to the longitudinal axis of the flow director, the radial wall thickness of the lower body portion varies about the flow director.

Having regard to the longitudinal axis of the flow director, the radial wall thickness of the lower body portion varies with the spacing of the rim from the base.

Having regard to the longitudinal axis of the flow director, the radial wall thickness of the lower body portion is larger where the rim is spaced further from the base than where the rim is spaced less from the base..

Having regard to the longitudinal axis of the flow director, the radial wall thickness of the lower body portion is least where the spacing between the rim and the base is the least and increases with increasing spacing between the rim and the base.

The radial wall thickness of the lower body portion is constant around the flow director.

The radial wall thickness of the upper body portion is constant around the flow director.

The dividing wall includes a spacing rib disposed between the bodies of the flow directors.

The spacing rib joins with the bodies at a position spaced from the rim.

The spacing rib joins with the base and bodies of the flow directors up to a position spaced from the rim.

The spacing rib joins with the base and the lower body portions only of the flow directors.

The flow directors include a step between the lower body portion and the upper body portion and the spacing rib which joins with the step.

The spacing rib has a flattened upper surface which joins with the step.

The spacing rib joins with the base and the lower body portions and the steps of the flow directors only.

The spacing rib includes a fillet-shaped transition joint with the flow directors.

The spacing rib is disposed in a plane which intersects the longitudinal axes of the flow directors.

The spacing rib has a wall thickness in the distal-proximal direction that is 5 to 20 % of the dimension of the flow director in the distal-proximal direction.

The spacing rib has a wall thickness in the distal-proximal direction that is 20 to 40 % of the dimension of the flow director in the distal-proximal direction.

The spacing rib has a wall thickness in the distal-proximal direction that is 40 to 60 % of the dimension of the flow director in the distal-proximal direction.

The spacing rib overlaps with a reinforcement section of the dividing wall which is arranged to transfer deformation forces applied to a patient-contact portion of the outer wall to the deformation region.

The dividing wall includes a respective brace member disposed between each flow director and the outer wall and which brace member maintains a spacing between the outer wall and the respective flow director.

Each brace members joins with the outer wall at the outer wall portion between the one or more nare openings and the one or more oral openings.

Each brace member joins with the outer wall portion and with the lower body portion only.

Each brace member joins with the outer wall portion and with the lower body portion and the step portion only.

Each brace member joins with the outer wall portion and with the common block.

Each brace member joins with the outer wall portion and with a respective block of the flow directors.

Each brace member has a lateral width which is 20 to 70 % of the lateral width of the flow director.

The brace member has a polygonal profile.

The brace member has a trapezoidal profile.

The brace member has a semi-circular profile.

The brace member has a circle-segment profile.

The brace member has a curved profile.

A proximal portion of the dividing wall between the spacing rib and the wall portion of the seal and between the lateral side portions of the dividing wall may have a wall thickness that is greater than the wall thickness of lateral side portions of the dividing wall.

The wall thickness of the proximal portion may be at least 1.5 times the wall thickness of the lateral side portions.

The wall thickness of the proximal portion may be in the range of 1.5 to 8 times the wall thickness of the lateral side portions.

The wall thickness of the proximal portion may be in the range of 1.5 to 3 times the wall thickness of the lateral side portions.

The wall thickness of the proximal portion may be greater than the wall thickness of a distal portion of the dividing wall between the spacing rib and the deformation panel and between the lateral side portions of the dividing wall.

The wall thickness of the proximal portion may be different at different locations. The wall thickness of the proximal portion may vary across the proximal portion in the proximal-distal direction or in the lateral direction or in both directions.

The spacing rib may be positioned distally of a centre of the second opening of the flow directors.

The spacing rib may be aligned, in the proximal-distal direction, with a distal portion of the rim which defines the second opening.

The main portion of the dividing wall may have a curved profile in the proximal-distal direction.

The curved profile may extend across substantially the entire lateral dimension of the main portion of the dividing wall.

The curved profile may extend through the proximal and distal portions of the dividing wall.

The curved profile may be concave in the proximal-distal direction and relative to the nare opening.

The proximal portion and the distal portion may have profiles that are inclined relative to one another in the proximal-distal direction such that the angle between them on the side of the dividing wall within the second chamber is less than 180°.

The profile of the proximal portion may be curved.

The profile of the distal portion may be curved.

The profile of the proximal portion may be concave relative to the nare opening.

The profile of the distal portion may be concave relative to the nare opening.

A distal portion of the dividing wall between the spacing rib and the deformation region and between the lateral side portions of the dividing wall may have a wall thickness that is greater than the wall thickness of lateral side portions of the dividing wall.

The wall thickness of the distal portion may be at least 1.5 times the wall thickness of the lateral side portions.

The wall thickness of the distal portion may be in the range of 1.5 to 8 times the wall thickness of the lateral side portions.

The wall thickness of the distal portion may be in the range of 1.5 to 3 times the wall thickness of the lateral side portions.

The wall thickness of the distal portion may vary in the proximal-distal direction.

The wall thickness of the distal portion may vary in the lateral direction.

The wall thickness of the distal portion may be the same as the wall thickness of the proximal portion.

The wall thickness of the distal portion may be greater than the wall thickness of the proximal portion.

The distal portion may include one or more taper regions where the wall thickness of the distal portion decreases in the direction of an adjoining lateral side portion or in the direction of the adjoining deformation panel to a thickness that is the same as the respective adjoining lateral side portion or the adjoining deformation panel.

One or more of the taper regions may be disposed on the side of the dividing wall facing the first chamber.

One or more of the taper regions may be disposed on the side of the dividing wall facing the second chamber.

One or more of the tapering regions may be disposed on the side of the dividing wall facing the second chamber and one or more of the tapering regions may be disposed on the side of the dividing wall facing the first chamber.

The spacing rib may include a taper region disposed between the proximal portion and the distal portion.

The base of the or each flow director may include a tapering region where the wall thickness of the distal portion decreases to a thickness that matches the wall thickness of the body portion of the one or more flow directors.

The distal portion may have a lateral width that tapers inwardly in the distal direction.

The patient interface may include a tether between the body of a respective flow director and the dividing wall.

The tether may be configured to resist changes in the orientation between the body of a respective flow director and the dividing wall.

The tether may link the body to the distal portion at positions on the body and on the distal portion spaced from the base of the respective flow director.

The tether may join with the flow director along the body and the base and joins with the distal portion from the base to the position spaced from the base.

The tether may be a rib extending between the body of a respective flow director and the distal portion.

The tether may be positioned on a distal side of the flow director.

The tether may be oriented in the proximal-distal direction.

The tether may be an extension of the body in the distal direction and which extension has a wall thickness in the proximal-distal direction that is greater than the wall thickness of other parts of the body.

The extension may taper inwardly in the distal direction in a cross-section parallel to the plane of the distal portion.

The patient interface may include one or more ties that connect the deformation panel to the first resilient region.

The deformation panel may extend from the first resilient region to define a gap into which the main portion travels upon deformation of the deformation panel and wherein the or each respective tie extends across the gap and joins with the first resilient region and the deformation panel.

The gap may be formed between the main portion and the first resilient region.

The or each respective tie may be configured to resist movement of the distal portion towards a nare sealing portion of the outer wall.

The or each respective tie may be configured to limit travel of the dividing wall away from the first resilient region.

The or each respective tie may be a panel which connects with the deformation panel and the first resilient region.

The panel may be configured to collapse under compression and is configured to resist extension under tension.

The panel may be configured to collapse under compression in the proximal-distal direction and is configured to resist extension under tension in the proximal-distal direction and/or or in the direction toward the nare sealing portion.

The panel may have a wall thickness that is the same as the wall thickness of the first wall of the deformation panel.

The panel may have a wall thickness that is the less than the wall thickness of the first wall of the deformation panel.

The or each tie may be perpendicular to the first wall of the deformation panel.

The or each respective tie may connect the distal portion and the first resilient region.

The or each respective tie may join with the distal portion, the deformation panel and the first resilient region.

The or each respective tie may connect the first resilient region and the reinforcement section associated with the distal portion.

The or each respective tie may connect the deformation panel and the first resilient region.

The tie may include a free edge exposed to the oral cavity.

The free edge may be a straight line extending between the first resilient region and the deformation panel or the distal portion or the reinforcement section associated with the distal portion.

The or each respective tie may include a point of weakness to induce collapse of the tie at that point under compressive forces.

The or each respective tie may include a point of weakness to induce collapse of the tie at that point as the distal portion travels toward the first resilient region.

The point of weakness may be a reduction in the wall thickness of the or each respective tie.

The free edge may extend between the first resilient region and the deformation panel or the distal portion or the reinforcing rib associated with the distal portion and the free edge includes the point of weakness.

The point of weakness in the free edge may be a notch disposed between the deformation panel and the first resilient region.

The notch has a V-shape.

The patient interface may include two ties and each tie is aligned in the proximal-distal direction with the distal portion.

The patient interface may include two ties and each tie is aligned in the proximal-distal direction with a respective lateral side portion.

The two ties may be parallel to each other in the proximal-distal direction.

The two ties may diverge from one another in the distal direction.

The two ties may converge toward one another in the distal direction.

The free edge of the or each respective tie may extend to a respective flow director.

The free edge of the or each respective tie may merge with a respective flow director.

The free edge may merge with the base of the respective flow director.

The dividing wall may include a reinforcing structure extending across at least the distal portion of the dividing wall and adjacent to the deformation panel.

The reinforcing structure may extend across the distal portion and the lateral side portions of the dividing wall and is adjacent to the deformation panel.

The reinforcing structure may be configured to resist deformation of the distal portion and the lateral side portions.

The reinforcing structure may be a lateral reinforcing rib extending across the distal portion and the lateral side portions at a position that is on a distal side of the or each flow director.

The reinforcing structure may be disposed with a spacing between the reinforcing structure and the deformation panel and the spacing is constant along the reinforcing structure.

The spacing may vary along the reinforcing structure.

The spacing between the reinforcing structure and the deformation panel may be greater across the distal portion than the spacing between the reinforcing structure and the deformation panel across the lateral portions.

The spacing between the reinforcing structure and the deformation panel may decrease in the lateral direction.

The spacing between the reinforcing structure and the deformation panel may be greatest in a central location.

The lateral reinforcing rib may be on the side of the dividing wall exposed to the second chamber or may be on the side of the dividing wall exposed to the first chamber.

The reinforcing structure may include a first lateral reinforcing rib on the side of the dividing wall exposed to the second chamber and includes a second lateral reinforcing rib on the side of the dividing wall exposed to the first chamber.

The first and second reinforcing ribs may be aligned on each side of the dividing wall in the proximal-distal direction.

The first and second reinforcing ribs may be off-set on each side of the dividing wall in the proximal-distal direction.

The spacing from the deformation panel for the first reinforcing rib may be different to the spacing from the deformation panel for the second reinforcing rib.

The spacing from the deformation panel for the first reinforcing rib may be constant and the spacing from the deformation panel for the second reinforcing rib varies.

The spacing from the deformation panel for the second reinforcing rib may be constant and the spacing from the deformation panel for the first reinforcing rib varies.

The lateral reinforcing rib may have a profile that is consistent along the length of the lateral reinforcing rib.

The lateral reinforcing rib may have a profile that varies along the length of the lateral reinforcing rib.

The lateral reinforcing rib may have a profile that tapers in height above the dividing wall in the lateral direction.

The lateral reinforcing rib may be spaced from the or each respective flow director.

The lateral reinforcing rib may be linked to the or each respective flow director.

The patient interface may include one or more deformation region ties in the first chamber and which connect the distal portion to the outer wall.

The one or more deformation region ties may be configured to inhibit inversion of the dividing wall.

The or each respective deformation region tie may extend between the side of the dividing wall that is exposed to the first chamber and the outer wall of the seal member.

The or each respective deformation region tie may extend from the outer wall to the distal portion.

The or each respective deformation region tie may connect with the dividing wall between the deformation panel and the flow directors or a respective one of the flow directors.

The or each deformation region tie may be spaced from the deformation panel.

The or each respective deformation region tie may be spaced from the flow directors.

The or each respective deformation region ties may connect with the dividing wall between the flow directors and the deformation panel.

Each respective deformation region tie may merge with a respective one of flow directors.

The or each respective deformation region tie may extend from the dividing wall and the outer wall and has a free edge in the first chamber.

The free edge of the or each deformation region tie may be a straight line.

The or each respective deformation region tie may have a flat planar structure.

The interface may include two deformation region ties and an angle between a proximal surface of each respective deformation region tie on the proximal side is greater than 140°.

The angle between the proximal surface of each respective deformation region tie on the proximal side may be greater than 160°.

The two deformation region ties may extend to positions on the distal portion that are spaced apart.

The two deformation region ties may extend to the same position on the distal portion.

The two deformation region ties may extend towards each other on the distal portion.

The two deformation region ties may extend to the reinforcement section.

The or each respective deformation region tie may include a first portion that connects with the outer wall and a second portion that extends to and connects with the main portion.

The or each respective deformation region tie may have two connection points, one connection point is with the main portion and the other connection point is with the outer wall at a level below the distal portion, having regard to an upright orientation of the patient interface.

The or each respective deformation region tie may be a cord or wire or cable.

The first portion has a flat planar structure and the second portion may be curved so that an angle, on the proximal side, between tangent lines at the respective positions to which the second portions extend is between 140° to 180°.

The or each respective deformation region tie may extend to a level below a lowermost level of the dividing wall, having regard to an upright orientation of the patient interface.

The or each respective deformation region tie may extend to a level below an uppermost level of the oral opening, having regard to an upright orientation of the patient interface.

The deformation region tie may extend from the distal portion to the outer wall at a position that is located distally of the proximal most portion of the one or more nare openings.

The free edge may extend from the outer wall at a position that is located distally of the reinforcement section.

The free edge may extend from the outer wall at a position that is located distally of the one or more oral openings.

The free edge may extend from the outer wall at a position that is located distally of the spacing element.

The deformation region ties may be configured to permit travel of the main portion of the dividing wall in the proximal-distal direction and to inhibit inversion of the dividing wall.

The flow directors may be configured to provide a gas flow rate through one flow director that is greater than a gas flow rate through another flow director.

One of the respective flow directors may be configured with a first resistance to gas flow and another of the respective flow directors is configured with a second resistance to gas flow and the first resistance to gas flow is lower than the second resistance to gas flow.

The first opening or the second opening or both the first opening and the second opening of the one of the respective flow directors may be larger than the first opening or the second opening or both the first opening and the second opening of the another of the respective flow directors.

A ratio of the area of the first opening of the one of the respective flow directors to the area of first opening of the another of the respective flow directors may be in the range of 1:1 to 1:0.1.

A ratio of the area of the second opening of the one of the respective flow directors to the area of the second opening of the another of the respective flow directors may be in the range of 1:1 to 1:0.1.

A ratio of the area of the second opening of the one of the respective flow directors to the area of the second opening of the another of the respective flow directors may be 1:0.33.

A ratio of the area of the second opening of the one of the respective flow directors to the area of the second opening of the another of the respective flow directors may be in the range of 1:0.5 to 1:0.2.

A ratio of the combined area of the first opening and the second opening of the one of the respective flow directors to the combined area of the first opening and the second opening of the another of the respective flow directors may be in the range of 1:1 to 1:0.1.

The patient interface may include only one flow director.

The one flow director may be configured to deliver a flow of respiratory gas to one nare.

The one flow director may be configured to deliver an accelerated flow of respiratory gas to create a flushing flow.

The one flow director may be configured to enable flushing of deadspace in the nasal cavity by delivering the flushing flow to the one nare.

The flow director may be in the form of any one of the flow directors disclosed above.

The flow director may be configured to direct a flow of gas through the nare opening and into one of the patient's nares.

The second opening of the flow director may be off-set laterally from a mid-plane through the patient interface.

The second opening of the flow director may be spaced laterally from a mid-plane through the patient interface.

The first opening of the flow director may be off-set laterally from a mid-plane through the patient interface.

The first opening of the flow director may be spaced laterally from a mid-plane through the patient interface.

The dividing wall on the opposite side of the mid-plane may include a first lateral portion adjacent to the mid-plane, the first lateral portion having a first wall thickness, and includes a second lateral portion extending laterally outwardly from the first lateral portion, the second lateral portion having a second wall thickness, and wherein the first wall thickness is greater than the second wall thickness.

A transition between the first lateral portion and the second lateral portion may be laterally spaced from the mid-plane by a distance that is substantially the same as the distance that the laterally-most positioned part of the flow director is spaced from the mid-plane.

A transition between the first lateral portion and the second lateral portion may be laterally spaced from the mid-plane by a distance that is greater than the distance that the laterally-most positioned part of the flow director is spaced from the mid-plane.

A transition between the first lateral portion and the second lateral portion may be laterally spaced from the mid-plane by a distance that is less than the distance that the laterally-most positioned part of the flow director is spaced from the mid-plane.

The patient interface may include one or more nare openings that seal about the nares of a patient and includes a channel extending from the first chamber to the nare opening or to one of the nare openings and which channel is configured to align with one of the patient's nares and the remainder of the one nare opening or another of the nare openings is configured to align with the other of the patient's nares.

The channel may be configured to enable gas from the other of the patient's nares to flow into the second chamber.

The channel may be configured to enable gas from the channel to flow into the second chamber.

The channel may be configured to deliver gas flow only to one of the patient's nares.

The channel may be configured to deliver gas flow to both of the patient's nares.

The channel may be configured to deliver first and second gas flows to the respective patient's nares and the first and second gas flows are unequal.

The first gas flow may be greater than the second gas flow.

The channel may be defined in part by a perimeter wall extending between the dividing wall and the nare opening or to the one of the nare openings and in part by a partition extending between the dividing wall and a position recessed from the nare opening.

The recessed position of the partition from the nare opening may enable gas from the channel to flow into the second chamber when the patient interface is fitted to a patient.

The cross-sectional area of the channel may reduce from the dividing wall to the nare opening or to one of the nare openings to accelerate gas as it travels through the channel.

The channel may be tapered from the dividing wall to the nare opening or to one of the nare openings to accelerate gas as it travels through the channel.

The profile of the channel is constant from the dividing wall to the nare opening or to one of the nare openings.

The channel and the one or more nare openings may be configured to deliver a flushing flow of gas to the first nare when the interface is fitted to a patient and which flushing flow causes flushing of the nasal cavity and causes gas flushed through the nasal cavity to flow through the other nare and through the remainder of the one nare opening or another of the nare openings and into the second chamber.

Each deformation region tie may be a panel having a wall thickness that decreases from the dividing wall to the free edge.

The or each respective flow director may comprise a base, a lower body portion, a rim which forms a second opening that opens into the second chamber and a transition extending between the lower body portion and the rim.

The lower body portion may have a wall thickness and the rim has another wall thickness that is less than the wall thickness of the lower body portion and wherein transition includes a tapering wall thickness.

The spacing rib may join with the lower body portion and the transition at a position spaced from the rim.

The spacing rib may be disposed distally of a notional line connecting the centres of the second openings of the respective flow directors.

The base or lower body portion of one or more flow directors may join with the wall portion.

The inner wall of the flow director may taper inwardly toward the second opening to form an inwardly tapered channel between the first opening and the second opening.

The wall thickness of the rim may be greater than the wall thickness of the lateral side portion, the distal portion or the proximal portion of the dividing wall.

The patient interface may include:
(a) one or more deformation region ties that extends between the side of the dividing wall that is exposed to the first chamber and the outer wall of the seal member;
(b) the main portion of the dividing wall has a concave profile in the proximal-distal direction relative to the nare opening; and
(c) the or each respective flow director comprises a base, a lower body portion, a rim which forms a second opening that opens into the second chamber and a transition extending between the lower body portion and the rim.

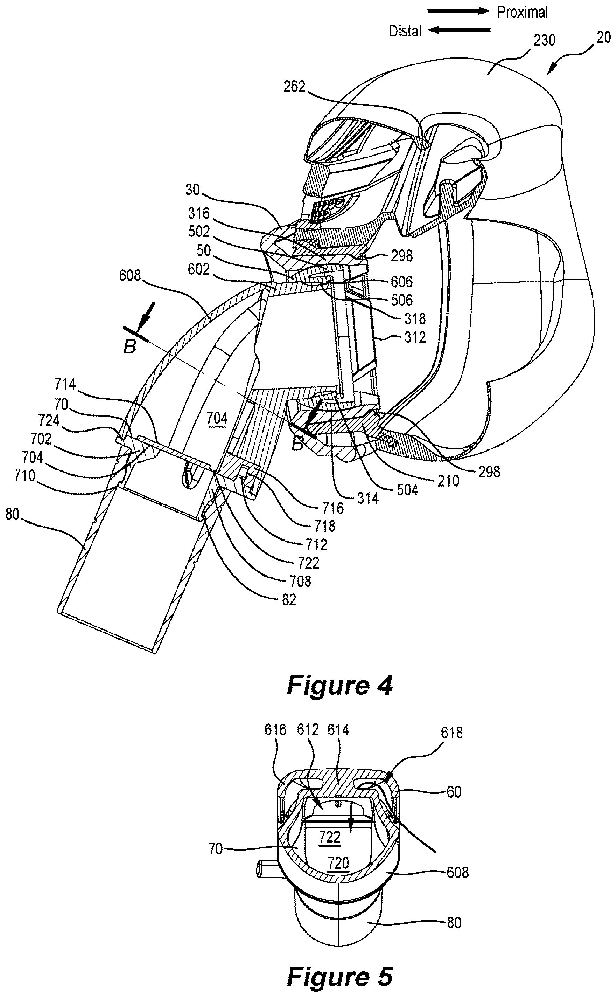

Throughout the above disclosure, the following description and the claims, the terms "proximal" and "distal" and their grammatical variants are references to the respective proximal direction and the distal direction. Proximal direction references indicate a direction that is toward a patient as if the patient interface were fitted to a patient. Distal references indicate a direction that is away from the patient as if the patient interface were fitted to a patient. These directions are shown in Figures 4, 34 and 40. The same directionality applies to all of the drawings and all of the embodiments disclosed in this specification. Use of the terms "proximal" and "distal" is not to be taken as indicating a state when the patient interface is fitted to a patient, unless the context indicates otherwise.

The flow directors of the fourth aspect may include any one or more of the features of the flow directors outlined in respect of the first, second and third aspects. The flow directors of the fourth aspect may also be combined with the cushion modules disclosed above in respect of the first, second and thirds aspects. The following description includes embodiments of the first, second, third and fourth aspects disclosed above. It will be understood that the embodiments of flow directors associated with the fourth aspect may be combined with embodiments of cushion modules associated with the first, send and third aspects. Furthermore, the flow directors of the fourth aspect may include any one or more of the features of the flow directors outlined in respect of the first, second and third aspects. The same applies to all other features described in the following description, namely, the embodiment described below and associated with one of the aspects disclosed above may be combined with any of the other embodiments described below in respect of the same aspect or a different aspect disclosed above.

Throughout the above disclosure, the following description and the claims, the term "chamber" is taken to mean a structure of the patient interface that encloses a volume and which structure has one or more respiratory gas inlets and one or more respiratory gas outlets.

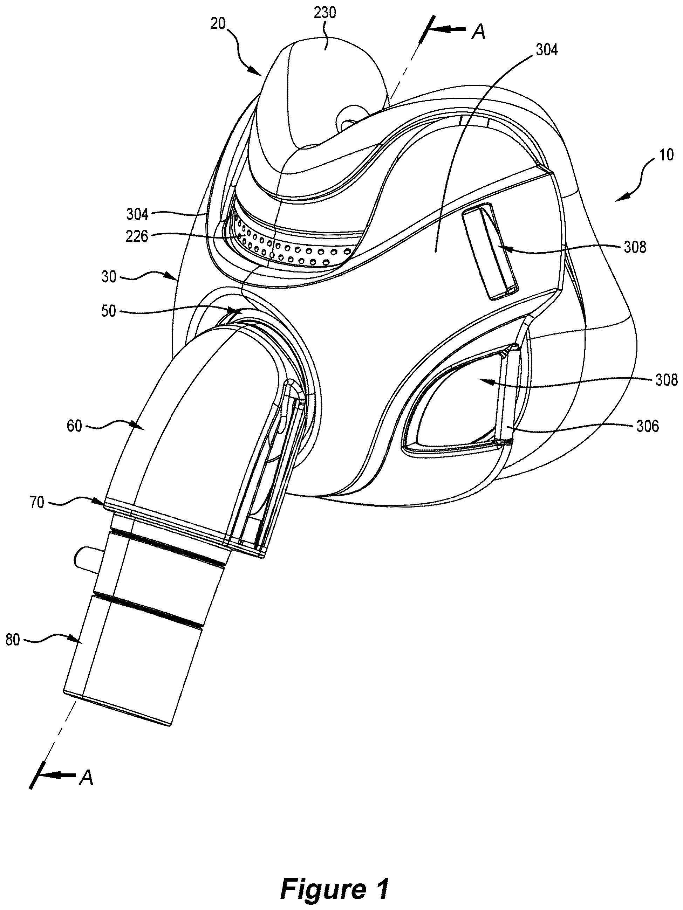

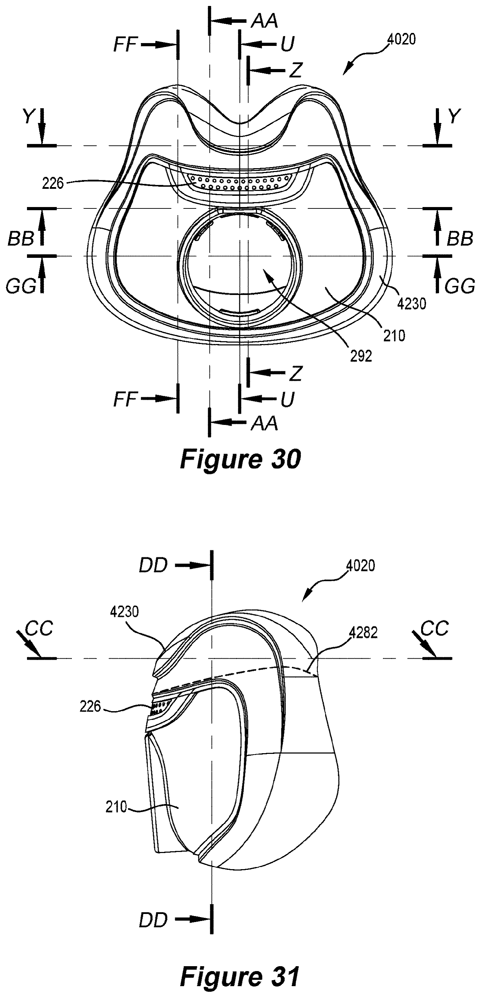

Throughout the above disclosure, the following description and the claims, the term "upright orientation" is taken to mean the orientation of the patient interface when a mid-plane between the lateral sides of the patient interface is oriented vertically and when the distal-most point of the wall portion on the mid-plane and a below-mouth point of the oral opening on the mid-plane are vertically aligned. The mid-plane is the plane of the cross-section line A-A in Figure 1 and is also the plane of the cross-section line U-U in Figure 30.

Although various features are disclosed above in relation to one or more aspect, it will be appreciated that one or more features of one aspect may be combined with other aspects to arrive at additional embodiments. It follows that disclosure of features in the preceding statements should not be interpreted as meaning that the features are limited in application to the aspects in respect of which they are disclosed. For example, the deformation region may be incorporated into the patient interface of any aspect described above. As a further example, the housing disclosed above may be incorporated into the patient interface of any one of the previous aspects.

Ordinal references (e.g. first, second, third) to aspects disclosed above serve to differentiate aspects from one another only. The ordinal references are not to be interpreted as the order of importance of the aspects.
The following numbered clauses show further illustrative examples only:
1. A non-invasive patient interface which is configured to seal about the mouth and nares of a patient, the patient interface including:
   (a) an outer wall defining an interior volume which includes a first chamber having one or more oral openings to communicate gas with the mouth and a second chamber having one or more nare openings to communicate gas with the nares; and
   (b) a dividing wall that separates the first chamber from the second chamber; and
   (c) one or more flow directors which enable gas to flow into the second chamber from the first chamber and which flow directors are configured to direct the gas flow through the one or more nare openings.
2. A non-invasive patient interface which is configured to seal about the mouth and nares of a patient, the patient interface including:
   (a) an outer wall defining an interior volume of the patient interface, the outer wall having a patient-engagement surface including one or more oral openings which communicate gas with the mouth and one or more nare openings which communicate gas with the nares; and
   (b) a dividing wall that separates the interior volume into a first chamber having the one or more oral openings and a second chamber having the one or more nare openings; and
   (c) one or more flow directors extending from the dividing wall which enable gas to flow into the second chamber from the first chamber and which flow directors are configured to direct the gas flow through the one or more nare openings; and
   wherein the flow directors are spaced apart by a spacing element which maintains a spacing between the flow directors.
3. A non-invasive patient interface which is configured to seal about the mouth and nares of a patient, the patient interface including:
   (a) an outer wall defining an interior volume of the patient interface, the outer wall having one or more oral openings which communicate gas with the mouth and one or more nare openings which communicate gas with the nares;
   (b) a dividing wall that separates the interior volume into a first chamber having the one or more oral openings and a second chamber having the one or more nare openings; and
   wherein the dividing wall includes one or more spaced apart flow directors which enable gas to flow into the second chamber from the first chamber and which flow directors are configured to direct the gas flow through the nare openings.
4. A non-invasive patient interface which is configured to seal about the mouth and nares of a patient, the patient interface including:
   (a) an outer wall defining an interior volume which includes a first chamber having one or more oral openings to communicate gas with the mouth and a second chamber having one or more nare openings to communicate gas with the nares; and
   (b) a dividing wall that separates the first chamber from the second chamber; and
   (c) one or more flow directors which enable gas to flow into the second chamber from the first chamber or into the first chamber from the second chamber.

### BRIEF DESCRIPTION OF THE DRAWINGS

The aspects of the patient interface disclosed above are described in detail below by reference to embodiments, which serve as examples only, and with reference to the accompanying drawings, in which:
Figure 1 is an oblique view of a patient interface and gas conduit connector according to one embodiment;
Figure 2 is an exploded oblique view of the patient interface and the gas conduit connector of Figure 1;
Figure 3 is a rear view of the patient interface and the gas conduit connector of Figure 1;
Figure 4 is a side cross-section view of the patient interface and the gas conduit connector along the line A-A in Figure 1;
Figure 5 is a cross-section of the gas conduit connector along the line B-B in Figure 4;
Figure 6 is a front plan view of a housing which form part of the patient interface of Figure 1;
Figure 7 is an oblique front view of a cushion module of the patient interface in Figure 1;
Figure 8 is an oblique side cross-section view of a cushion module only of the patient interface along the line C-C in Figure 7;
Figure 9 is a magnified view of the region marked D in the cross-section view in Figure 8;
Figure 10 an oblique cross-section view of the cushion module along the line E-E in Figure 7;
Figure 11 is a cross-section view of the cushion module along the line F-F in Figure 7;
Figure 12 is an oblique rear view of an alternative cushion module;
Figure 13 is a side cross-section view along the line G-G in Figure 12;
Figure 14 is a magnified and oblique view of the region marked H in the cross-section view of Figure 13;
Figure 15 is an underside view of the dividing wall in the cushion module of Figure 12;
Figure 16 is an oblique rear view of a further alternative cushion module;
Figure 17 is a side cross-section view of the cushion module in Figure 16 along the line J-J;
Figure 18 is a magnified and oblique view of the region marked K in the cross-section view of Figure 17;
Figure 19 is another side cross-section view of the cushion module in Figure 16 along the line L-L;
Figure 20 is a front cross-section view of the cushion module in Figure 16 along the line M-M;
Figure 21 is an underside view of the dividing wall in the cushion module of Figure 16;
Figure 22 is a magnified and oblique view of the underside of the dividing wall in the direction marked N in Figure 21;
Figure 23 is a top cross-section view of the cushion module in Figure 16 along the line P-P;
Figure 24 is an oblique rear view of a further alternative cushion module;
Figure 25 is a top cross-section view of the cushion module in Figure 24 along the line Q-Q;
Figure 26 is a side cross-section view of the cushion module in Figure 24 along the line R-R;
Figure 27 is a magnified and oblique view of the region marked S in the cross-section view of Figure 26;
Figure 28 is an underside view of the dividing wall in the cushion module of Figure 24;
Figure 29 is a magnified and oblique view of the underside of the dividing wall in the direction marked T in Figure 21;
Figure 30 is a front plan view of a further alternative cushion module;
Figure 31 is a side plan view of the alternative cushion module shown in Figure 30;
Figure 32 is a top plan view of the alternative cushion module shown in Figure 30;
Figure 33 is a rear plan view of the alternative cushion module shown in Figure 30;
Figure 34 is a cross-sectional view along the line U-U in Figure 30;
Figure 35 is a magnified view of the region marked W in the cross-section view of Figure 34;
Figure 36 is a magnified view of a cross-section view along the line X-X in Figure 32;
Figure 37 is an oblique cross-sectional view along the line Y-Y in Figure 30;
Figure 38 is an oblique cross-sectional view along the line Z-Z in Figure 30;
Figure 39 is a cross-sectional view along the line AA-AA in Figure 30;
Figure 40 is an underneath view of a cross-section along the line BB-BB in Figure 30;
Figure 41 is a magnified cross-sectional view of the cushion module in Figure 30 along the line U-U and with an alternative flow director and spacing rib;
Figure 42 is a magnified view of Figure 32 with flow directors in the form of the alternative flow director and spacing rib in Figure 41;
Figure 43 is a magnified cross-sectional view of the cushion module in Figure 30 along the line U-U and with another alternative flow director and another alternative spacing rib;
Figure 44 is a magnified cross-section view along the line AA-AA in Figure 30 with the alternative flow director and spacing rib in Figure 43;
Figure 45 is an oblique magnified cross-sectional view from above of the cushion module in Figure 30 along the line U-U and with another alternative flow director and spacing rib;
Figure 46 is an oblique magnified cross-section view along the line AA-AA in Figure 30 with the alternative flow director in Figure 45;
Figure 47 is a magnified cross-sectional view of the cushion module in Figure 30 along the line U-U and with another alternative flow director, spacing rib and reinforcement section;
Figure 48 is a magnified cross-section view along the line BB-BB in Figure 30 with the flow directors, spacing rib and reinforcement section in Figure 47;
Figure 49 is a magnified cross-section view along the line Y-Y in Figure 30 with the flow directors, spacing rib and reinforcement section in Figure 47;
Figure 50 is a magnified cross-sectional view of the cushion module in Figure 30 along the line U-U and with another alternative flow director and another alternative spacing rib;
Figure 51 is a magnified cross-section view along the line AA-AA in Figure 30 with the alternative flow director and spacing rib in Figure 50;
Figures 52 to 54 are magnified cross-sectional views of the cushion module in Figure 30 along the line U-U with alternative proximal portions and distal portions of the dividing wall;
Figure 55 is a magnified oblique view of a cross-section of the cushion module in Figure 31 along the line CC-CC with the flow directors, spacing rib and with the proximal portions and distal portions in Figure 54;
Figure 56 is a magnified cross-sectional view of the cushion module in Figure 31 along the line DD-DD and with further alternative flow directors;
Figure 57 is a magnified cross-sectional view of the cushion module in Figure 33 along the line EE-EE with the alternative flow directors in Figure 56;
Figure 58 is a magnified oblique view of a cross-section through the cushion module in Figure 33 along the line EE-EE and with further alternative flow directors;
Figure 59 is a magnified cross-sectional view of the cushion module in Figure 31 along the line CC-CC with the flow directors in Figure 58;
Figure 60 is a magnified cross-sectional view of the cushion module in Figure 30 along the line U-U and with the flow directors of Figure 58;
Figure 61 is a magnified oblique view of a cross-section through the cushion module in Figure 33 along the line EE-EE and with further alternative flow directors;
Figure 62 is a magnified oblique view of a cross-section through the cushion module in Figure 30 along the line FF-FF and with the alternative flow directors of Figure 61;
Figure 63 is a magnified cross-sectional view of the cushion module in Figure 30 along the line U-U and with the flow directors of Figure 61.
Figure 64 is a top plan view of a cross-sectional view along the line Y-Y in Figure 30 with further alternative flow director;
Figures 65A and 65B are magnified cross-sectional views of the cushion module in Figure 30 along the line U-U with the alternative flow directors and spacing rib of Figure 64 and with alternative proximal portions of the dividing wall;
Figure 66 is a magnified cross-sectional view of the cushion module in Figure 30 along the line U-U with the alternative flow directors and spacing rib of Figure 64 and with an alternative configuration of the proximal and distal portions of the dividing wall;
Figure 67 is a magnified cross-sectional view of the seal member only in Figure 30 along the line HH-HH and with the flow directors of Figure 64 and with an alternative distal portion of the dividing wall;
Figure 68 is an oblique view from underneath of a magnified cross-section of the seal member only in Figure 30 along the line HH-HH and with the flow directors of Figure 64 and with the alternative distal portion of Figure 67;
Figure 69 is a magnified cross-sectional view of the seal member only in Figure 30 along the line U-U and with the flow directors of Figure 64 and with the alternative distal portion of the dividing wall of Figure 67;
Figure 70 is a top plan view of a cross-section along the line Y-Y in Figure 30 with the alternative flow directors of Figure 64 and with flow director tethers;
Figure 71 is an oblique view from above of a magnified cross-section of the cushion module in Figure 30 along the line U-U and with the flow directors of Figure 64 and with the flow director tethers of Figure 70;
Figure 72 is a magnified cross-sectional view of the seal member only in Figure 30 along the line U-U and with the flow directors of Figure 64 and with a tie that extends from the deformation panel;
Figure 73 is an oblique underneath view of a cross-section of the seal member only in Figure 30 along the line BB-BB and with the tie of Figure 72;
Figure 74 is a magnified underneath plan view of a cross-section of the seal member only in Figure 30 along the line BB-BB and with the tie of Figure 72;
Figure 75 is a magnified cross-sectional view of the seal member only in Figure 30 along the line U-U and with the flow directors of Figure 64 and with an alternative tie arrangement;
Figure 76 is an oblique underneath view of a cross-section of the seal member only in Figure 30 along the line BB-BB and with the alternative tie arrangement of Figure 75;
Figure 77 is a magnified underneath plan view of a cross-section of the seal member only in Figure 30 along the line BB-BB and the alternative tie arrangement of Figure 75;
Figure 78 is a top plan view of a cross-section of the cushion module along the line Y-Y in Figure 30 with the alternative flow directors of Figure 64 and with a reinforcing structure;
Figure 79 is a magnified cross-sectional view of the cushion module in Figure 30 along the line U-U and with the flow directors of Figure 64 and with the reinforcing structure of Figure 78;
Figure 80 is a magnified cross-sectional view of the cushion module in Figure 30 along the line U-U and with the flow directors of Figure 64 and with deformation region ties;
Figure 81 is an underneath plan view of a cross-section view of the cushion module in Figure 30 along the line GG-GG and with the deformation region ties of Figure 80;
Figure 82 is an oblique underneath view of a cross-section of the seal member only in Figure 30 along the line GG-GG and with the deformation region ties of Figure 80;
Figure 83 is a magnified cross-sectional view of the cushion module in Figure 30 along the line AA-AA and with alternative flow directors and with alternative deformation region ties;
Figure 84 is a magnified cross-sectional view of the cushion module in Figure 30 along the line U-U and with the flow directors and deformation region ties of Figure 83;
Figure 85 is a top plan view of a cross-section of the cushion module in Figure 30 along the line Y-Y with an alternative arrangement of flow directors;
Figure 86 is an underneath plan view of a cross-section view of the cushion module in Figure 30 along the line GG-GG and with the arrangement of flow directors of Figure 85;
Figure 87 is an oblique view from above of a cross-section of the cushion module in Figure 30 along the line Y-Y with the alternative arrangement of flow directors of Figure 85;
Figure 88 is a top plan view of a cross-section of the cushion module in Figure 30 along the line Y-Y with a single flow director arrangement;
Figure 89 is an underneath plan view of a cross-section of the cushion module in Figure 30 along the line GG-GG and with the single flow director arrangement of Figure 88;
Figure 90 is an oblique view from above of a cross-section of the cushion module in Figure 30 along the line Y-Y with the single flow director arrangement of Figure 88;
Figure 91 is an oblique view from above of a cross-section of the cushion module in Figure 33 along the line LL-LL and with a channel from the oral chamber to the nare opening;
Figure 92 is a magnified view a cross-section of the cushion module in Figure 33 along the line KK-KK and with the channel of Figure 91; and
Figure 93 is an underneath plan view of a cross-section of the cushion module in Figure 30 along the line BB-BB and with the channel of Figure 91.

### DESCRIPTION OF EMBODIMENTS

A preferred embodiment of the present invention will now be described in the following text which includes reference numerals that correspond to features illustrated in the accompanying figures. Where possible, the same reference numeral has been used to identify the same or substantially similar features in the different embodiments. To maintain clarity of the figures, however, all reference numerals are not included in each figure.

The aspects of the patient interface disclosed above will be described in detail below by reference to embodiments of a patient interface in the general form shown in Figures 1 to 4. The embodiments described below are variations on that general form. However, it will be appreciated that the scope of the aspects should not be limited by reference to that general form or to the specific embodiments described below and, instead, the aspects should be interpreted as relating to other forms of patient interface that also deliver pressurised respiratory gas to the patient, including patient interfaces that extend across the bridge of the nose, full-face masks and full-head helmets.

The term "respiratory gas" as used throughout this specification is taken to mean a gas used in human respiration. The term "inhaled respiratory gas" as used throughout this specification is taken to mean respiratory gas that is inhaled during the inhalation phase of the breathing cycle. The term includes within its scope ambient air or air that is conditioned for treating a patient, such as having elevated humidity or oxygen levels or both compared to ambient air. The term "exhaled respiratory gas" as used throughout this specification is taken to mean respiratory gas that is exhaled from the lungs and airways of a patient. It, therefore, includes respiratory gas from the lungs and which gas occupies anatomical dead space at the end of the exhalation phase of the breathing cycle.

Having regard to Figures 1 to 4, the general form comprises a patient interface 10 which includes a cushion module 20, a frame 30 and a conduit connector 40. The conduit connector 40 comprises structural components for connecting the cushion module to a source of respiratory gas, such as a ventilator, humidifier, flow generator or wall source. In this embodiment, the patient interface 10 is in the form of a sub-nasal mask where the cushion module 20 comprises a resilient seal member 230 and a housing 210. Collectively, the resilient seal member 230 and a housing 210 form an outer wall 288 of the cushion module 20.

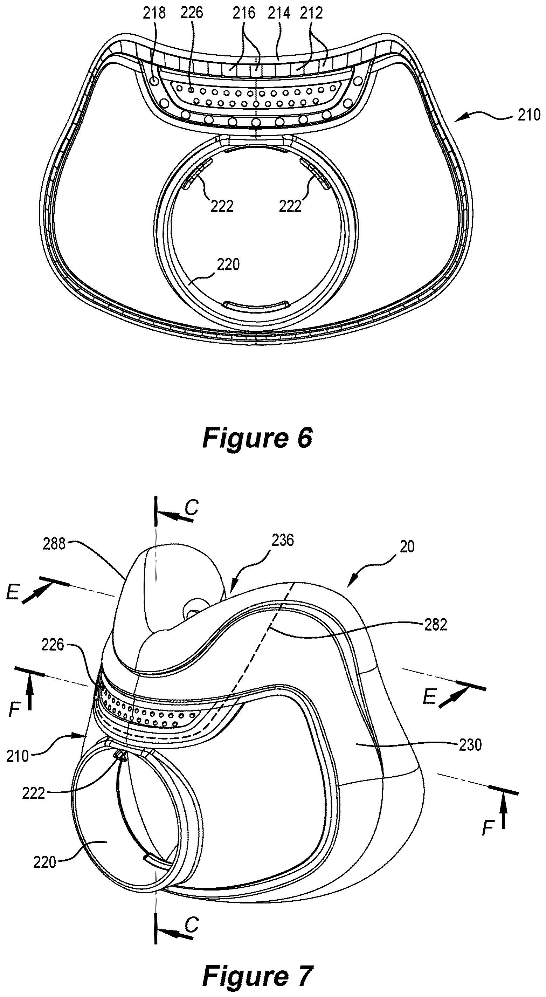

The housing 210, as shown in Figure 6 without the seal member 230, is formed of substantially rigid plastics material to provide structural support to the seal member 230. Additionally, the housing 210 provides an interface for connecting the seal member 230 to the frame 30 and the conduit connector 40.

The housing 210 includes a sleeve 220 that is sized and shaped to connect with the frame 30. The sleeve 220 forms an inlet opening 292 through which respiratory gas can be communicated from the conduit connector 40 to interior of the cushion module 20. The sleeve includes key formations 222 that interact with the frame 30 to ensure correct alignment of the frame 30 with the housing 210 when they are fitted together.

The housing 210 includes a series of tabs 212 which project outwardly around its perimeter. The outer ends of the tabs 212 are linked to a bead 214 which runs continuously across all of the tabs 212, thereby forming a series of discrete outer over-mould windows 216 between the tabs 212 and the bead 214. The seal member 230 is integrally formed with the housing by over-moulding a resilient material onto the housing 210 to fill the series of windows. Therefore, the tabs 212 and the bead 214 become embedded in the resilient material and are mechanically interlocked with the seal member 230. The seal member 230 and the housing 210, therefore, form a unitary cushion module 20 structure.

The housing 210 further includes a group of bias vent holes 226 through the housing 210. The housing further includes a series of inner over-mould windows 218 through which the seal member 230 is also over-moulded with the housing 210. The inner over-mould windows 218 are located in a region of the housing 210 that is within the perimeter formed by the outer over-mould windows 216. The bias vent holes 226 are bound on one side by the outer over-mould windows 216 and on another side by the inner over-mould windows 218. As shown in Figure 6, the bias vent holes 226 are surrounded by the outer over-mould windows 216 and the inner over-mould windows 218. The material used to form the seal member 230 flows through the inner and outer over-mould windows 216, 218 during moulding so that the material conforms to the shape of the housing 210 and the windows 216, 218 prior to solidifying or curing. Having the material extend through the windows 216, 218 results in a mechanical connection with the housing. The windows 216, 218 may take the form of apertures which extend completely through the housing 210.

The seal member 230 is formed of soft, resilient material, such as silicone, and includes an oral opening 232 and a nare opening 234. When fitted to a patient, the oral opening 232 circumscribes the patient's mouth and a patient-contact surface 290 of the outer wall forms a seal about the mouth. Accordingly, respiratory gas at elevated pressure can be delivered to the patient via the oral opening 232. The seal member 230 is formed with a nare-sealing portion 236 in the valley of which is located the nare opening 234. The patient-contact surface 290 includes the nare-sealing portion 236 and a wall portion 276 located between the oral opening 232 and the nare opening 234. The nare-sealing portion 236 is arranged to contact the underside of the patient's nose and to form a seal with the patient's nares so that respiratory gas at elevated pressure can be delivered to the patient via the nare opening 234. The nare opening 234 is located to align with the nares of the patient when the mask 10 is fitted.

Although this embodiment of the cushion module 20 includes a single oral opening 232 and a single nare opening 234, it will be appreciated that other embodiments may include more than one oral opening, more than one nare opening or multiple oral openings and multiple nare openings. In another non-illustrated embodiment, the cushion module includes a single oral opening 232 with two nare openings 234.

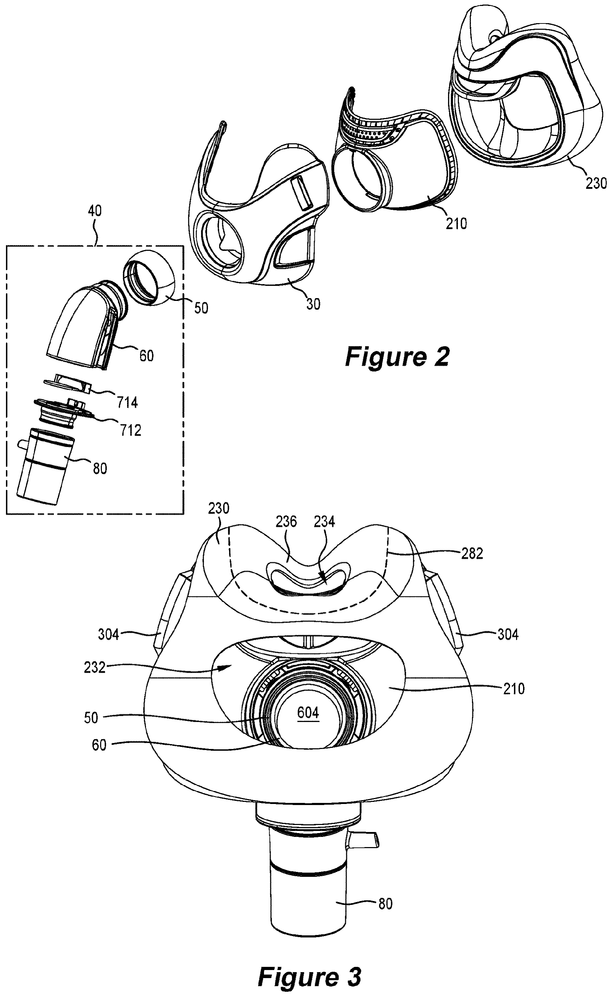

The cushion module 20 defines an interior volume which comprises a first chamber 238 and a second chamber 240. The second chamber 240 is located in an upper portion of the internal volume of the seal member 230. The first chamber 238 comprises the lower portion of the internal volume of the cushion module 20. The first and second chambers 238, 240 are separated by a dividing wall 242. The first cavity is a first chamber 238 and the second cavity is a second chamber 240. As shown in Figures 3 and 4, the oral opening 232 is associated with the first chamber 238 to enable transfer of respiratory gas between a first chamber 238 and the patient's mouth. The inlet opening 292 of the sleeve 220 is also associated with the first chamber 238. The nare opening 234 is associated with the second chamber to enable transfer of respiratory gas between a second chamber 240 and the patient's nares. The bias vent holes 226 are also associated with the second chamber 240. The bias vent holes 226, therefore, enable respiratory gas to be vented from the cushion module to an outside environment. In this embodiment, the bias vent holes 226 vent the respiratory gas to the ambient atmosphere.

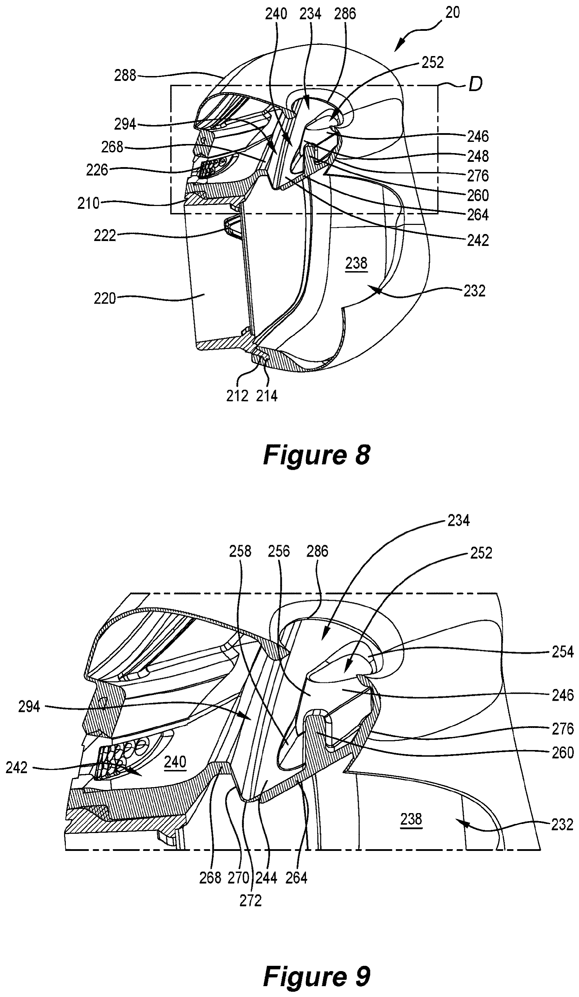

The dividing wall 242 partitions the cushion module 20 internally to define the first chamber 238 and the second chamber 240. The dividing wall 242 separates the first chamber 238 from the second chamber 240 by extending all the way across the internal volume of the cushion module 20. In other words, the perimeter of the dividing wall 242 seals with the outer wall 288. A meeting line 282, which notionally shows where the dividing wall 242 meets the outer wall 288, is shown in Figures 3 and 7. In particular, it extends all the way across between the first chamber 238 and the second chamber 240. The sealing of the first chamber from the second chamber means that the only flow of respiratory gas between the first chamber and the second chamber is through the flow directors 246. So, while the dividing wall 242 seals the first chamber 238 from the second chamber 240, the dividing wall 242 permits respiratory gas to flow from the first chamber 238 to the second chamber 240 only via a pair of flow directors 246 (see Figures 8 to 11).

The dividing wall 242 of the cushion module 20 has a broad U-shaped transverse profile. This is shown by the meeting line 282 in Figures 3 and 7 extending below the bias vent holes 226, across ridges either side of the nare sealing portion 236 and across the wall portion 276. The U-shaped profile allows for some vertical travel of dividing wall 242 (and the flow directors 246) which contributes to maintaining a spacing between the flow directors 246 and the nare opening 234 (which also travel vertically when a deformation force is applied to the nare sealing portion 236) to reduce the risk of the flow directors contacting the patient. The broad U-shaped profile of the dividing wall 242 is replaced in later embodiments described below with a flatter profile.

Figures 8 and 9 show the dividing wall 242 seals with the outer wall 288 between the nare opening 234 and the oral opening 232. In terms of the wall portion 276, this means that the dividing wall 242 seals with the wall portion 276 across the entire width of the wall portion 276. In other words, the dividing wall 242 seals with the outer wall 288 at a location spaced from the nare opening 234. It can further be seen in Figures 8 and 9 that the dividing wall 242 seals with the outer wall 288 closer to the oral opening 232 than to the nare opening 234. In other words, the seal between the dividing wall 242 and the wall portion 276 is located in a lower half of the wall portion 276.

One design consideration for the seal member 230 involves balancing patient comfort and treatment performance. Treatment performance is improved by ensuring that flow structures which direct respiratory gas into the nares, such as the flow directors 246 in this embodiment, retain their shape and remain correctly oriented when the patient interface is fitted to a patient. Some patient interfaces link the structures to the patient-contact surface 290. Often the link is in a nasal contact region of the patient contact surface 290, i.e. a nasal contact surface. In this embodiment, the dividing wall 242 is shifted lower within the internal volume of cushion module 20. The lower positioning of the dividing wall 242 shifts the join between the dividing wall 242 and the outer wall 288 away from the nasal contact surface. This is anticipated to improve patient comfort. However, it is anticipated that treatment performance can be sustained because the dividing wall 242 can be formed with additional support structures which assist to retain the shape and orientation of the flow directors 246. This means that the structures rely less on connection to the outer wall 288 for support, which is the reason for the flow directors 246 in cushion module 20 having only a small contact region with the outer wall 288. While the additional support structures incorporated into the dividing wall 246 will increase the resilience of the dividing wall 242, and increase the resilience of the connection with the outer wall, the lower location of the dividing wall 242 results in the connection between the dividing wall 242 and the outer wall 288 away from the highly sensitive nasal contact region. This shift in the location of the dividing wall 242 join with the outer wall 288 improves patient comfort compared with joins and links in the nasal contact region. That is, reducing links between structures, such as flow directors 246, and the outer wall 288 improves patient comfort because there are less structural contact points with the outer wall 288 which can form pressure points on the patient's face. The facial contact points affected by the patient interface 10 in use include highly sensitive areas, such as the septum and the philtrum. It is anticipated that patient comfort is improved by adopting the lower position of the dividing wall 242 between the nare opening 234 and the oral opening 232. It is also anticipated to be improved by reducing contact between internal structures of the cushion module 20 and the patient-contact surface 290. Furthermore, the greater volume of the second chamber 240 enables structures within the second chamber 240 (such as the flow directors 246) to be further supported and reinforced by the dividing 242 wall. As mentioned above, it is anticipated that other embodiments will have the flow directors spaced from the outer wall 288 so that the dividing wall 242 is the only link between them and the cushion module 20.

The flow directors 246 extend from the dividing wall 242 into the second chamber 240. In other embodiments, the flow directors 246 may extend from the dividing wall 242 into the first chamber 238 and the second chamber 240 or into the first chamber 238 only. In another embodiment, the flow directors 246 may not extend from either side of the dividing wall 242. For example, the dividing wall 242 may have a wall thickness that is sufficient to include a flow director 246 between the surfaces of the dividing wall 242 exposed to the first chamber 238 and to the second chamber 240 respectively.

The flow directors 246 define a gas flow path from the first chamber 238 to the second chamber 240 and the flow directors 246 surround the gas flow path. Each flow director 246 comprises a body 248 having a first opening 250 and a second opening 252. The first openings 250 open into the first chamber and the second openings 252 open into the second chamber 240. This means that the flow directors 246 are the only avenue for respiratory gas to flow between the first chamber 238 and the second chamber 240.

The flow directors 246 are disposed in a main portion 244 of the dividing wall 242. In this embodiment, the body 248 of each flow director 246 joins with the dividing wall 242. A small segment of the body 248 joins with the outer wall 288 at the wall portion 276. It follows that the first chamber 238 is separated from the second chamber 240 by the dividing wall 242 joining with the outer wall 288 and joining with the flow directors 246 and by the flow directors 246 joining with the outer wall 288. In other embodiments, the body 248 of each flow director 246 may be spaced from the outer wall 288 so that the entire perimeter of the dividing wall 242 joins with the outer wall 288. Said another way, the body 248 of each flow director 246 may be located in a central region of the dividing wall 242 such that each flow director 246 is spaced from the perimeter of the dividing wall 242. The central region may coincide with the centre of the dividing wall or may be off-set from the centre of the dividing wall. In each embodiment, however, the first chamber 238 is separated from the second chamber 240 by the dividing wall 242 and the flow directors 246.

In this embodiment, the entire seal member 230 is integrally formed and is over-moulded onto the housing 210. This integral moulding of the dividing wall 242 with the outer wall 288 forms a gas-tight joint. The same applies with the flow directors 246 joining with the dividing wall 242.

The second openings 252 are positioned to direct respiratory gas towards and through the nare opening 234. This directs respiratory gas to the patient's nares from the first chamber 238 via the second opening 252 and through the nare opening 234. Such directed respiratory gas assists breathing during the respiratory cycle and contributes to anatomical dead-space flushing in the nasal cavity of a patient. It is to be appreciated that whilst at least a portion of the gas flow directed by the flow directors 246 toward the nare opening 234 will generally flow through the nare opening 234, some of the directed gas may not flow through the nare opening 234. Furthermore, at some times during use of the patient interface the directed respiratory gas may not flow through the nare opening 234 at all. Gas flow through the nare opening 234 will depend upon the gas flow rate, gas pressure and the point in the breathing cycle. Any directed respiratory gas flow from the flow directors 246 that doesn't flow through the nare opening 234 will flow through the second chamber 240 to the bias vent 226.

The position and shape of the second openings 252 affects the flow of respiratory gas into the second chamber and through the nare opening 234. In the embodiment shown in Figures 8 to 10, each flow director 246 has an second opening 252 within the second chamber 240 and the second opening 252 is spaced from the nare opening 234. The second opening 252 is defined by a rim 254 that is contoured so that at least a portion of the rim 254 has a substantially consistent spacing from the nare opening 234. The portion of the rim 254 that is substantially consistently spaced from the nare opening 234 is adjacent to the outer wall 288 between the at least nare opening 234 and the oral openings 232. The rim 254 of each second opening 252 extends further from the dividing wall 242 at a laterally outer side of the flow director 246 than the rim 252 extends from the dividing wall 242 at a laterally inner side of the flow director 246. This means that the rim 254 is recessed further from the nare opening 234 at a laterally inner side of the flow director 246 than the rim 254 is recessed from the nare opening 234 at the laterally outer side of the flow director 246. The lower height of the rim relative to the dividing wall 242 on the laterally inner side of each flow director 246 assists with the flow of respiratory gas from the first chamber 238 into the second chamber 240. The higher height of the rim relative to the dividing wall 242 on the laterally outer side of each flow director 246 assists with directing respiratory gas from the first chamber 238 through the second chamber 240 and through the nare opening 234. In another embodiment the rim 254 is recessed furthest from the nare opening 234 at a point between the laterally outer side of the flow director 246 and the laterally inner side of the flow director 246 such that in cross section the rim 254 is concave or curved inwardly.

The recessed position of the second opening 252 relative to the nare opening 234 enables excess respiratory gas from the first chamber 238 (including exhaled respiratory gas from the mouth) to pass into the second chamber 240 and be vented to externally of the patient interface 10 through the bias vent holes 226. The nare opening 234 is defined, at the outer wall 288 of the seal member 230, by a rim 286. However, given that the second opening 252 is recessed from the rim 254, the spacing between the second opening 252 and the rim 286 enables respiratory gas to flow from the first chamber 238 to the second chamber 240 and then out through the bias vent holes 226 to ambient atmosphere. This generally occurs during part of the exhalation phase of the respiratory cycle. For example, when the patient exhales through their nose (excess respiratory gas delivered from a source to the first chamber 238 flows into the second chamber 240). In another example, it also occurs when the patient exhales through their mouth (the exhales gas flows from the mouth and into the first chamber and then into the second chamber with excess respiratory gas delivered from a source). At times during inhalation through the nose or during nasal cavity dead space flushing, the respiratory gas may flow from the first chamber 238, into the second chamber 240 and then through the nare opening 234 and into the nares of the patient.

The body 248 of each flow director 246 includes an lower body portion 256 and a upper body portion 258. The overall profile of the body 248 has a cross-sectional area that reduces in the direction between the first opening 250 to the second opening 252. In this embodiment, the body 248 includes an lower body portion 256 and a upper body portion 258. The lower body portion 256 tapers significantly from the first opening 250 to the upper body portion 258 and the upper body portion 258 tapers slightly from the lower body portion 256 to the second opening 252. In other embodiments, however, the upper body portion 258 may have a cross-sectional area that is less than the cross-sectional profile of the lower body portion 256. This may be embodied in a stepped profile for the body 248. The reduction in the cross-sectional area between the first opening 250 and the second opening 252 has the effect of accelerating respiratory gas toward the nare opening 234 so as to deliver an accelerated stream of respiratory gas to the nares. In a further embodiment, the body 248 of each flow director may have a cross-sectional area that is constant between the first opening 250 and the second opening 252.

In this embodiment, the lower body portion 256 and the upper body portion 258 have a conical profile. In alternative embodiments, the upper body portion 258 may have a circular, elliptical or oval-shaped profile in cross-section generally parallel to the dividing wall where the flow director is located. Furthermore, the body 248 may have a conical profile, tapering profile or a stepped profile in cross-section generally orthogonal to the dividing wall 242 where the flow director 246 is located.

In the embodiment shown in Figures 8 to 19, the patient interface 10 includes two flow directors 246 that are spaced apart. That is, the two flow directors 246 comprise two separate structures. They do not share a common wall which defines the flow path through each of the flow directors 246. The flow directors 246 are spaced apart by a gap through which respiratory gas from the first chamber 238 and exhaled respiratory gas from the nares entering the second chamber via the nare opening 234 can flow into the second chamber. The rim 254 of each flow director 246 extends from the dividing wall 242 less on the same side of the flow director 246 as the gap than the rim 254 extends on the side of the flow director 246 that is remote from the gap. In this embodiment, the flow directors 246 are configured with the lateral outer side of the rims 254 adjacent to a laterally outer rim 286 of the nare opening 234 having regard to a direction of gas flow from the flow director 246. More specifically, the rim 254 is located laterally inwardly of the laterally outer rim 286 of the nare opening 234. However, in other embodiments, the rim 254 may be located laterally outwardly of the laterally outer rim 286 of the nare opening 234 or the lateral outer side of the rim 254 may be aligned with a laterally outer rim 286 of the nare opening 234 having regard to a direction of gas flow from the flow director 246.

The spacing apart of the flow directors 246 to the laterally outer side of the nare opening 234 is anticipated to improve patient comfort due to reduced contact points with the outer wall 288. The reduced contact points, as explained above, will reduce the number or size of pressure points on a patient's face. Additionally, the lateral spacing of the contact points will shift contact points away from the sensitive septum and philtrum areas. The anticipated improvement in comfort should contribute to greater compliance with treatments and should reduce pressure sores. Furthermore, it is believed that the lateral position of each of the two flow directors 246 with regards to the nare opening 234 will improve the likelihood of the flow directors 246 aligning with the nares of the patient and therefore delivering sufficient flow. This is because the laterally spaced position of the flow directors 246 lowers the chance that a patient with a wide or large septum (which would be located centrally with respect to the nare opening 234) will impede flow from exiting the flow directors 246.

A bead 262 circumscribes the rim 286 on an inner surface of the seal member 230. The bead 262 comprises, in this embodiment, a region of increased wall thickness as shown in Figure 4. The increased thickness of the rim 286 increases the resistance of the rim 286 to prevent excessive outwards deformation, otherwise known as a blow-out which can occur when the patient interface 10 receives high levels of pressurised respiratory gas from a gas source. The increased thickness of the rim 286 also increases the resistance of the rim 286 to undesired deformation which may occur when the patient interface 10 is fitted to the patient.

The dividing wall 242 is configured to preferentially deform in a way that reduces the likelihood of the second chamber 240 and the nare opening 234 being occluded.

In this embodiment, the dividing wall 242 comprises the main portion 244 and a first resilient region 268 which are linked by a deformation panel 294. In this embodiment, the first resilient region 268 has the form of a lip. The deformation panel 294 comprises a first wall 270 and a second wall 272 . The first wall 270 which projects from the first resilient region 268 (see Figure 9), the second wall 272 extends from the main portion 244 between the flow directors 246 and the second wall 272 and joins with the first wall 270. The second wall 272 has a curved profile which extends from the first wall 270 to the main portion 244. The undeformed configuration of the first wall 270 and the second wall 272 is shown in Figure 9.

The deformation panel 294 structurally decouples the first resilient region 268 from the main portion 244. The decoupling occurs because the deformation panel 294 accommodates a reduction in distance between the first resilient region 268 and the main portion 244.

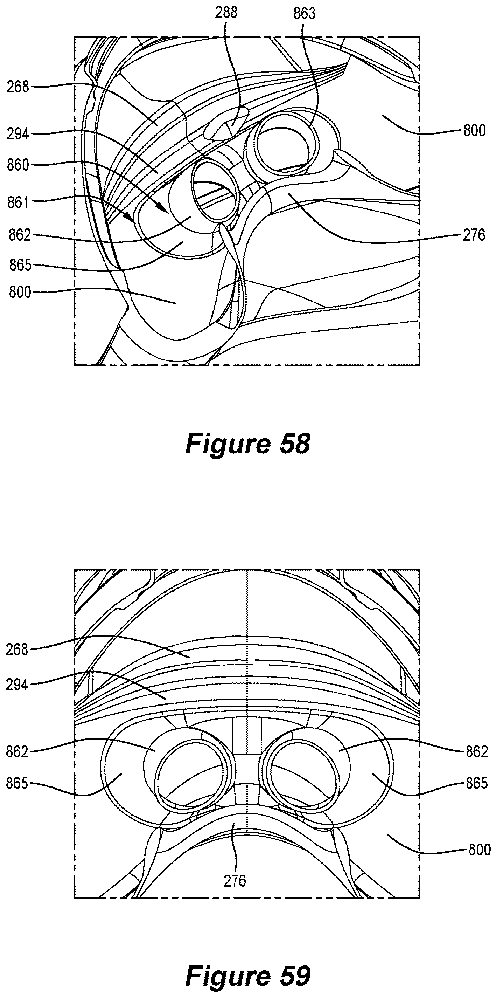

Deformation of the deformation panel 294 occurs in two stages. The main portion 244 includes the flow directors 246 and, therefore, is relatively resistant to deformation in the area of the flow directors 246 compared to the first and second walls 270, 272. Accordingly, the deformation panel 294 will deform more readily than the remainder of the main portion 244. The first stage of deformation involves a force being applied to the patient contact surface 290 such that the main portion 244 is displaced toward the first resilient region 268. This causes the first wall 270 to fold about its line of connection with the first resilient region 268 until it contacts, or is located adjacent an underside of the first resilient region 268. In the second stage of deformation, as the main portion 244 continues to be displaced towards the first resilient region 268, the second wall 272 and the section of the main portion 244 buckle and translate over the first wall 270 until the flow directors 246 contact, or are located adjacent to, the first resilient region 268. This buckling and translating motion may be referred to as "rolling". The bucking occurs on account of the curvature of the second wall 272. This sequence of deflection is shown and described in international patent application PCT/NZ202/050072, with reference in particular to Figures 59A to 59D and associated description. The content of the international application is incorporated in this specification by this reference so they are read as a single disclosure.

In an alternative embodiment, the dividing wall 242 may be configured so that deformation of the deformation panel 294 is limited to the first stage of deformation described above. For example, the second wall 272 may be omitted or the second wall may be reduced in size or have an altered shape which avoids the "rolling" motion. In a further alternative, the first wall 270 may be extend further from the first resilient region 268 so that the first stage of deformation occurs for all displacements of the main portion 244 toward the first resilient region 268.

During use, when the mask 10 is fitted, force will be applied to the patient contact surface 290 to account for differing facial geometries, headgear preferences and pressure settings. These forces and the locations they are applied will differ. The configuration described above, however, focusses the forces and deflection into the deformation panel 294 to provide a predictable collapse and rebound movement. The predictable buckling pattern achieved via the preferable deformable region allows the mask 10 to be designed in such a way that, when forces are applied to the seal, the resulting deformation and compression that occurs in the elastomeric material that forms the seal member 230 happens in such a way that the nare opening 234 and the second openings 252 from the flow directors 246 are likely to remain unobstructed. Furthermore, the positioning of the nare opening 234 and the second openings 252 relative to each other will be substantially maintained during deformation. Without this preferential deformation, collapse of the dividing wall 242 would be unpredictable, potentially leading to inconsistent flow through the second openings 252 or inconsistent positioning of the second openings 252 relative to the nare opening 234 and/or patents nares. This would cause inconsistencies in the therapy achieved, comfort, fitting procedure and in overall performance both between uses for the same patient and between different patients.

In a variation of this embodiment, the seal member 230 may have more than one preferential deformation region. For example, the additional preferential deformation regions may be incorporated into the dividing wall 242 or may be incorporated into the seal member 230 at other locations that enable the second chamber 240 and/or nare opening 234 and the second openings 252 to substantially retain their shape.

The geometries of the first and second walls 270, 272 and their thicknesses are selected so that the cushion module 20 can accommodate a wide range of facial geometries and deformation forces associated with application and use of the patient interface. However, it is possible for different cushion modules to be produced to fit specific ranges of facial geometries which fall toward the ends of the facial geometry spectrum.

While the first resilient region 268 is formed with greater wall thicknesses to provide greater resilience compared to the deformation panel 294, this is done so that a single material can be used to form the seal member 230. However, it will be appreciated that the first resilient region 268 may be stiffened by alternative means provided that the deformation panel 294 preferentially deforms when a force is applied to the seal member 230. For example, the first resilient region 268 may be formed of more resilient materials (such as a different grade of silicone or a plastic material) or may have a different structure.

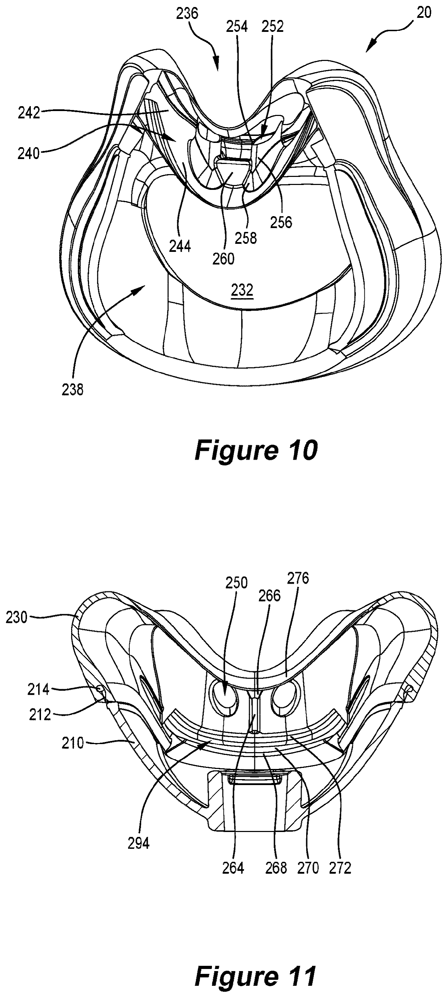

The transfer of deformation forces into the deformation panel 294 is contributed to by one or more reinforcement sections 264 formed in the dividing wall 242. In this embodiment, the reinforcement section 264 comprises a thickened wall section located on the underside of the dividing wall 242 as shown in Figure 11 and shown in cross-section in Figures 8 and 9. The reinforcement section 264 takes the form of a rib with a force-spreading end 266 that links to a wall portion 276 of the outer wall 288. The force spreading end 266 tapers outwardly in the direction of the wall portion 276. The spreading distributes a contact area of the reinforcement section 264 over a larger area of the wall portion 276. This creates a larger contact point over which the force is spread. Reducing the flexibility of the wall portion 276 affects patient comfort by providing a hard, localised contact point in the patient contact surface 290. However, spreading the contact point improves patient comfort by reducing the contact pressure. The reinforcement section 264 directs forces applied to the wall portion 276 into the dividing wall 242 where the deflection force is absorbed by the deformation panel 294. In doing so, the reinforcement section 264 substantially retains the position of the dividing wall 242 (and the flow directors 246) relative to the nare opening 234. Another way of understanding the effect of the reinforcement section 264 is to understand that it braces the main portion 244 in position relative to the wall portion 276. This enables a patient treatment to continue with little interference to the flow of respiratory gas (a) through the flow directors 246, (b) through the nare opening 234 and (c) through the second chamber 240 during deformation of the patient contacting portion of the outer wall 288.

The function of the reinforcement section 264 is assisted by a spacing rib 260 (Figure 10). In this embodiment, the spacing rib 260 has the form of a rib which connects both flow directors 246 and connects to the main portion 244 at a position that overlaps the reinforcement section 264 (see Figure 9). The spacing rib 260 extends part way up each flow director 246. In this embodiment, the spacer extends from the dividing wall and up the lower body portion 256. Connection of the spacing rib 260 to the flow director 246 reinforces the flow director 246 and makes it more resilient to deformation. Connection of the spacing rib 260 to each flow director 246 also ensures that any forces applied to one flow director 246 are transferred and spread to the other flow director 246 which may reduce localised deformation. The connection of the spacing rib 260 to the flow director 246 also reduces the likelihood of the flow director 246 buckling inwardly or outwardly. Reducing the likelihood of buckling assists to retain the alignment of the flow directors 246 in a direction that, in use, directs a flow of respiratory gas toward the nare opening 234.

Additionally, the position of the spacing rib 260 and the reinforcement section 264 means that deflection of the wall portion 276 translates through the reinforcement section 264 and the spacing rib 260 and, therefore, causes the flow directors 246 to track movement of the wall portion 276 and the nare opening 234. The spacing rib 260 also serves to maintain the position of the second openings 252 relative to each other and reduces the likelihood of the second openings 252 collapsing inwardly or outwardly during deformation. In this embodiment, the spacing rib 260 is integrally formed with the bodies 248 of the flow directors 246. However, in other embodiment, the spacing rib 260 may be located between the flow directors 246, but not joined to them. Alternatively, the spacing rib 260 may join the flow directors 246 but may not be linked to the main portion 244. While this arrangement does not overlap with the reinforcement section 264, the spacing rib 260 still acts to maintain the relative spacing of the second openings 252. In another alternative, the spacing rib 260 or the reinforcement section 264 may be omitted. The structure of the flow directors and either the spacing rib 260 or the reinforcement section 264 act to make the main portion more resilient to deformation so that, without one or other of the spacing rib 260 or the reinforcement section 264, deformation forces are expected to be transferred into the preferential deformation region. Accordingly, the flow directors 246 are expected to retain their shape and position relative to the nare opening 234 when subject to deformation forces.

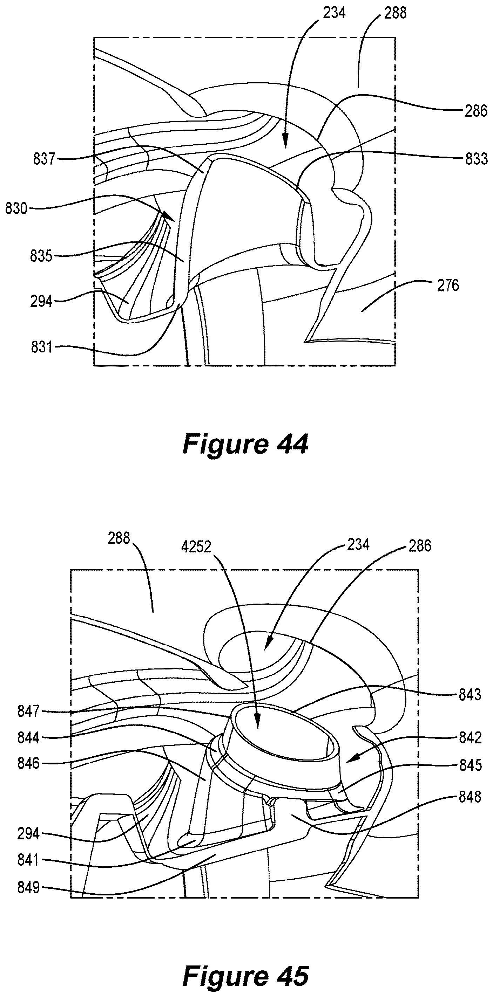

The way in which the cushion module 20 delivers respiratory gas to a patient and removes excess respiratory gas and removes exhaled respiratory gas is similar to the method described in the international patent application PCT/NZ202/050072, with reference in particular to Figures 45 to 47 and the associated description. By way of summary in reference to the cushion module 20, the supply of respiratory gas to the patient is as follows. An accelerated stream of respiratory gas is delivered to a patient to provide anatomical dead space flushing. This involves delivering respiratory gas at an elevated pressure to the first chamber 238 of the cushion module 20. The first chamber 238 supplies the respiratory gas to the mouth of a patient via the oral opening 232, and to the second chamber 240 via the flow directors 246. The second chamber 240 supplies the respiratory gas to the nares of the patient via the nare opening 234. The flow of respiratory gas towards the nares is accelerated through the flow directors 246. The flow directors 246 reduce in cross-sectional area toward the nare opening 234. The accelerated respiratory gas can then be delivered to the nares of a patient. The accelerated flow of respiratory gas occurs at the same time that respiratory gas is available for delivery to the mouth from the first chamber 238.

In an additional embodiment the flow directors 246 do not reduce in cross-sectional area towards the second opening 252, however the flow directors 246 still accelerate gas from the first opening 250 towards the second opening 252 due to a restriction of the gas entering the flow directors 246 from the first chamber 238.

Broadly speaking, the patient interface 10 operates such that when the patient interface 10 is fitted to a patient and the patient's mouth is open there is:
- a first flow path from the first chamber 238 into the patient's mouth through the oral opening 232, and subsequently into their oral cavity then nasal cavity, out one or both of the nares and into the second chamber 240 via the one or more nare openings 234 and out of the patient interface via the bias vent 226, and
- a second flow path from the first chamber 238 through the flow directors 246 into the second chamber 240 and out of the patient interface through the bias vent 226.
When the patient interface 10 is fitted to a patient and a flow of pressurised gas is supplied to the patient interface 10 there is a first pressure P1 generated in the first chamber 238 and a second pressure P2 generated in the second chamber 240. Due to a combination of a flow restriction located in the second flow path and the bias vents 226 located in the second chamber 240, there is a pressure differential formed between the pressure P1 in the first chamber 238 and the pressure P2 in the second chamber 240 with the pressure P2 in the second chamber 240 being lower than the pressure P1 in the first chamber 238. This pressure differential means pressurised gas supplied to the first chamber 238 will flow through both the first flow path and the second flow path in order to exit the patient interface via the bias vents 226 in the second chamber 240.

The balance of the resistance to flow through the first flow path and the resistance to flow through the second flow path determines the proportion of the pressurised gas supplied to the patient interface 10 that travels through each path.

The second flow path is configured to have a sufficiently high resistance to flow due to the flow restriction in the second flow path that at least a desired minimum portion of the pressurised gas flow supplied to the patient interface 10 flows along the first flow path to exit the patient interface 10. This causes flushing of the patient's anatomical deadspace in at least the throat and nasal cavity. In some embodiments, the flow restriction of the second flow path (and therefore the desired resistance to flow) is due to the restricted cross-section of the flow directors 246. In other embodiments, a restriction can be provided in another location along the second flow path to create the desired resistance to flow of the second flow path.

The patient interface 10 also generally operates such that, when the patient's mouth is closed, there is only a single flow path from the first chamber 238, through the flow directors 246, through the one or more nare openings 234 into one or both of the patients nares and into the nasal cavity, after which the gas flow decelerates and the flow direction is reversed and the gas flows through one or both or the patients nares, through the one or more nare openings 234 into the second chamber 240 and out of the patient interface 10 through the bias vent 226. Due to the restricted cross-section of the flow directors 246 and the large volume of gas flowing through them owing to the single flow path through the patient interface 10, the flow is accelerated therethrough which results in a jetting effect that is directed towards and into the patient's nares via the one or more nare openings 234. This jetting effect into the one or both nares causes flushing of the patient's anatomical deadspace.

It is to be appreciated that the above description describes certain flow paths formed during use of the patient interface 10. If the patient interface 10 were to be fitted to an anatomical model with no respiration occurring, it is believed the above would be observed. However, other flow paths may be formed in particular during respiration either through the mouth or nares. The below description provides some further details on specific situations during the respiration cycle.

While breathing through the mouth, most of the supply of respiratory gas to the first chamber 238 is inhaled by the patient via the oral opening 232. Some respiratory gas supplied to the first chamber 238 in excess of the tidal flow requirements flows from the first chamber 238 through the flow directors 246. A portion of the gas flow through the flow directors 246 may then flow through the nare opening 234 and into the nares to flush the patients' anatomical nasal dead space, while the remaining flow enters the second chamber 240 and exits the cushion module 20 via the bias vent holes 226. Additionally, or instead of the above discussed flow through the flow directors 246, a portion of the respiratory gas supplied to the first chamber 238 in excess of the tidal flow requirement may flow through the oral opening 232 and into the patient's mouth, through the oral cavity and nasal cavity and out through their nares into the second chamber 240 via the one or more nare openings 234 and subsequently out of the patient interface 10 via the bias vent holes 226 creating some flushing of the anatomical deadspace. This is due to the pressure differential formed between the first chamber and second chamber as discussed above. During exhalation, exhaled respiratory gas flows from the mouth and into the first chamber 238 where it mixes with fresh respiratory gas supplied to the first chamber 238. This mix of exhaled respiratory gas and fresh respiratory gas then flows through the flow directors 246, a portion of the gas flow through the flow directors 246 flows through the one or more nare openings 234 and into the nares to flush the patients' anatomical nasal dead space, while the majority of the exhaled and excess respiratory gas will flow via the flow directors 246 into the second chamber 240. The flow of respiratory gas into the second chamber 240 is assisted by the recessed position of the second openings 252 relative to the rim 286 of the one or more nare openings 234. The exhaled and excess respiratory gas then flow out of the second chamber 240 of the patient interface 10 via the bias vent holes 226. In a majority of cases, a majority of the flushing of the mouth and upper throat occurs towards the end of the exhalation and/or inhalation cycles when the pressure within the mouth and throat of the patient drops below the pressure of the respiratory gas supplied to the first chamber, at this point flow enters the patients mouth via the oral opening 232, travels through the patients nasal cavity exiting the nares and enters the second chamber 240 via the one or more nare opening 234 thereby flushing the patients nasal cavity, mouth and upper throat with fresh respiratory gas.

While breathing through the nose with the mouth closed, respiratory gas supplied to the first chamber 238 flows through the flow directors 246 and is directed toward the nares. The respiratory gas is inhaled through the nares. Respiratory gas in excess of the tidal flow requirements flows from the flow directors 246 and into the second chamber 240 and then out of the patient interface 10 via the bias vent holes 226. During most of the exhalation phase, the respiratory gas cannot enter the nares due to the pressure within the patient's nares being higher than the pressure of the supplied respiratory gas and, therefore, the respiratory gas flows into the second chamber 240 and then out of the patient interface 10 via the bias vent holes 226. The exhaled respiratory gas flows into the second chamber 240 via the gap between the flow directors 246 and the nare opening 234 and joins the flow of respiratory gas flowing out through the bias vent holes 226. However, toward the end of the exhalation phase, when the pressure within the nasal cavity drops below the pressure of the supplied respiratory gas the accelerated stream of respiratory gas from the flow directors 246 penetrates the nares so that anatomical dead space in the nasal cavity and upper throat are flushed with fresh respiratory gas.

While breathing through the nose with the mouth open, respiratory gas supplied to the first chamber 238 flows through the flow directors 246 and is directed toward the nares. The respiratory gas is inhaled through the nares via nare opening 234. Some respiratory gas in excess of the tidal flow requirements flows from the flow directors 246 and into the second chamber 240 and then out of the patient interface 10 via the bias vent holes 226. During exhalation through the nose with the mouth open, exhaled respiratory gas enters the second chamber 240 via the nare opening 234 and exits the patient interface 10 via bias holes 226. Concurrently fresh respiratory gas supplied to the first chamber enters the patient's mouth, travels through the patient's nasal cavity exiting the nares and enters the second chamber 240 via the nare opening 234. This flushes the patient's anatomical deadspace in their mouth, upper throat and nasal cavity with fresh respiratory gas. Furthermore, toward the end of the exhalation phase, when the pressure within the nasal cavity drops below the pressure of the respiratory gas exiting the flow directors 246, a portion of the supplied respiratory gas may flow through the flow directors 246 and enter the nares so that anatomical dead space in the nasal cavity and upper throat are flushed with fresh respiratory gas.

The dead space flushing described above replaces low oxygen-content respiratory gas in the patient's anatomical dead spaces with higher oxygen-content respiratory gas that is taken into the lungs at the start of the next inhalation cycle. The low oxygen-content respiratory gas is pushed out of the dead spaces and into the second chamber 240 where it exits the patient interface 10 via the bias flow holes 226. Flushing of the low oxygen-content (or said another way, high CO₂ content) respiratory gas into the patient interface 10 means that the low oxygen-content respiratory gas is not available for inhalation at the start of the next breathing cycle. This is enabled in part by having the pressurised respiratory gas being supplied to the first chamber 238 in combination with the bias vent holes 226 being located in the second chamber 240 such that there is a constant one-way flow through the patient interface 10 which removes any low oxygen-content respiratory gas from the patient interface 10 by pushing it out of second chamber 240 via the bias holes 226.

Said another way, the dead space flushing described above replaces high carbon dioxide-content respiratory gas in the patient's anatomical dead space with lower carbon dioxide-content respiratory gas that is taken into the lungs at the start of the next inhalation cycle. The high carbon dioxide-content respiratory gas is pushed out of the anatomical dead space and into the second chamber 240 where it exits the patient interface 10 via the bias flow holes 226. Flushing of the high carbon dioxide-content respiratory gas into the patient interface 10 means that the high carbon dioxide-content respiratory gas is not available for inhalation at the start of the next breathing cycle.

Whilst providing a patient with supplemental oxygen would increase oxygen concentrations, it doesn't necessarily reduce CO₂ levels present in the anatomical dead space as much as flushing the anatomical dead space with ambient air does. It follows that an advantage of this approach is that improved outcomes may be achieved without the need for supplemental oxygen in the respiratory gas that is supplied to the patient interface 10. This is particularly advantageous where there are shortages of supplemental oxygen. This is additionally advantageous wherein potential risks associated with supplying supplemental oxygen to a patient may be avoided by removing or reducing the need for supplemental oxygen. Furthermore, because removing the high carbon dioxide-content respiratory gas from a patient's deadspace may improve gas exchange within the lungs, it follows that this approach may provide improved outcomes that cannot be achieved with supplemental oxygen solely.

Alternative embodiments of the cushion module 20 are described below. They function in the same way as described above for the cushion module 20 in terms of delivering respiratory gas to a patient, removing excess respiratory gas and removing exhaled respiratory gas.

The embodiment described above involves supplying respiratory gas from a gas source to the first chamber 238 and venting respiratory gas from at least the second chamber. This configuration of supply and venting of respiratory gas involves the flow of respiratory gas from the first chamber 238 to the second chamber 240. In alternative embodiments, however, the housing 210 may be reconfigured to receive the supply of respiratory gas from a gas source into the second chamber 240 and to vent the respiratory gas from the first chamber 238. This configuration involves the flow of respiratory gas from the second chamber 240 to the first chamber 238 via the flow directors 246. While the embodiments of the cushion module described below are in the context of gas flow from the first chamber 238 to the second chamber 240, the same applies to the various cushion module embodiments, including variations of those embodiments, described below. That is, those cushion module embodiments may be utilized with the direction of the gas flow from the first chamber 238 to the second chamber 240 or from the second chamber 240 to the first chamber 238.

Although the description of the seal member 230 above is in the context of the features being formed integrally by a moulding process, any one or more of the features may be formed separately and then assembled with the seal member 230. For example, the main portion 244 may be formed with an opening that is adapted to receive a pre-formed flow director unit which includes two spaced part flow directors. One reason for adopting this assembly method may be to form certain features from different material. Referring to the flow director unit example, the unit may be formed of hard plastics material which has a considerably higher resistance to deformation than the same structure when formed of silicone. In a further example spacing rib 260 and/or reinforcement section 264 may be formed separately from seal member 230 and joined at a later stage.

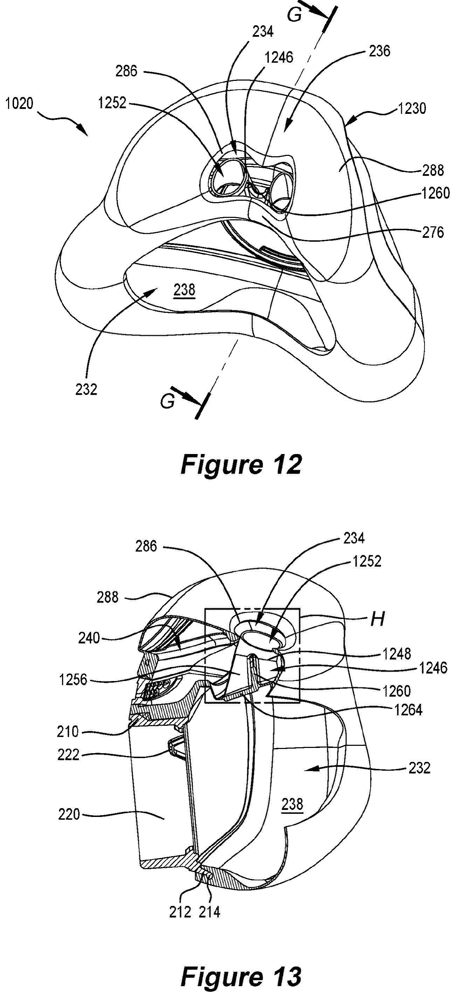

A further variation of the cushion module 20 is shown in Figures 12 to 15 as cushion module 1020. Features of the cushion module 1020 that are the same as the features of the cushion module 20 are denoted by the same reference numerals. Features that are not the same as the features of the cushion module 20 are denoted by like references number with a prefix comprising the number "1".

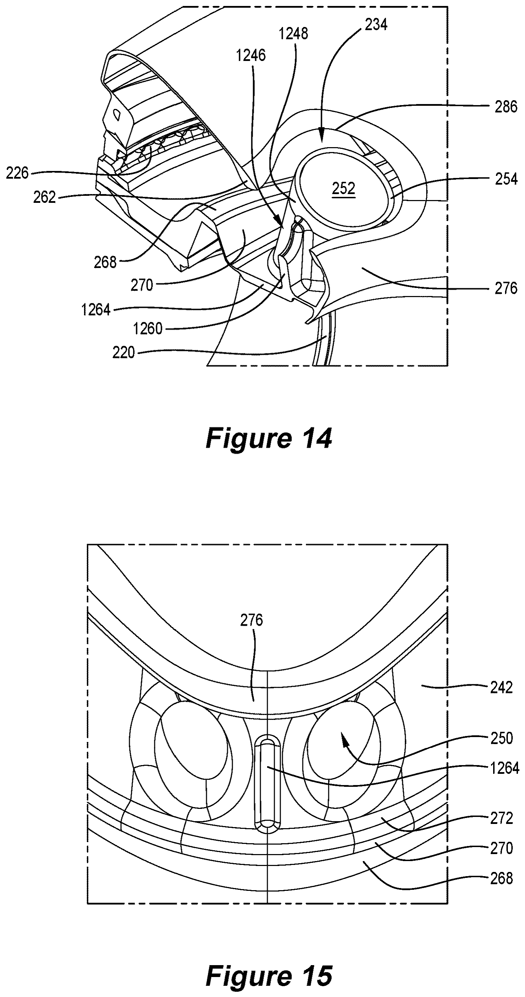

The cushion module 1020 can be used with the same housing 210, frame 30 and conduit connector 40 disclosed in respective of cushion module 10. However, cushion module 1020 differs from the cushion module 20 in that the flow directors 1246 have bodies that have a greater cross-sectional area to reduce the resistance to flow of respiratory gas through them. Additionally, the bodies 1248 are formed with a funnel-shaped lower body portion 1256 (see Figures 13 and 14). The lower body portion 1256 comprises more than 50% of the extent to which the flow director 1246 projects from the dividing wall 242.

The spacing rib 1260 between the flow directors 1246 is formed with a concave bridge connecting the flow directors 1246. This shape reduces the risk of contact with the patient's nose, particularly the patient's septum which is a known high-sensitivity area. The concave shape allows the spacer to extend further up the side of each flow director 1246 and, therefore, contributes a greater stabilising effect on the flow directors 1246. The spacing rib 260 of the cushion module 20 is offset from the centre of the distance that the flow director 246 spans between the wall portion 276 and the second wall 272. In contrast, the spacing rib 1260 is located at the centre or close to the centre of that dimension span. The more central location of the spacing rib 1260 assists to maintain the spacing of the second openings 1252. More specifically, the more central location reduces the tendency of the flow directors 1246 to collapse toward each other when a deformation force is applied to the wall portion 276.

A further difference is that the reinforcement section 1264 is spaced from the wall portion 276 (Figures 14 and 15). Removing the contact point between the reinforcement section 264 and the wall portion 276 (as in the cushion module 20) reduces the localised wall thickness of the wall portion 1276 and, therefore, improves patient comfort. The lower body portion 256 of each flow director 246 sufficiently reinforces the main portion 244 such that deformation forces are directed from the wall portion 276 through the main portion 244 and into the deformation panel 294. The reinforcement of the main portion 244 avoids the need for the reinforcement section 264 to connect with the wall portion 276 or for the reinforcement section 264 to have a force-spreading end 266.

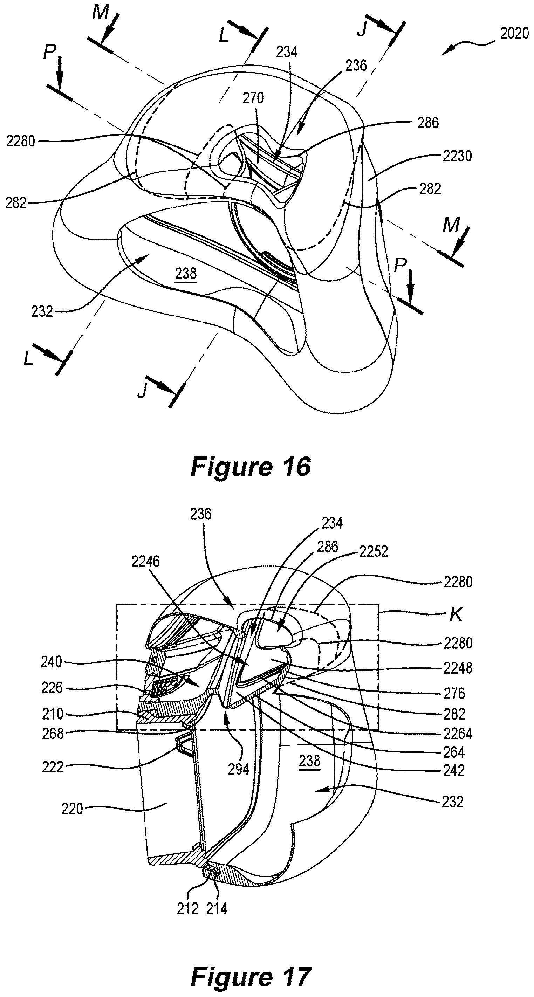

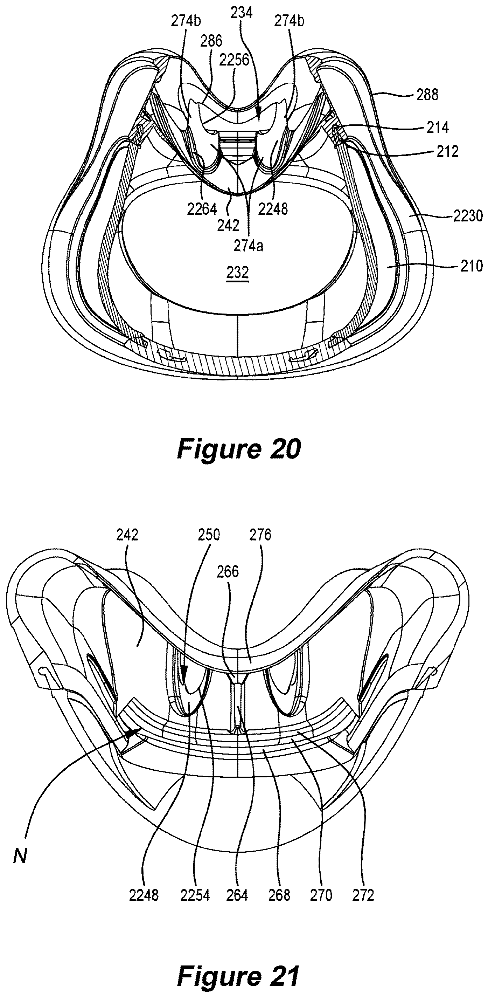

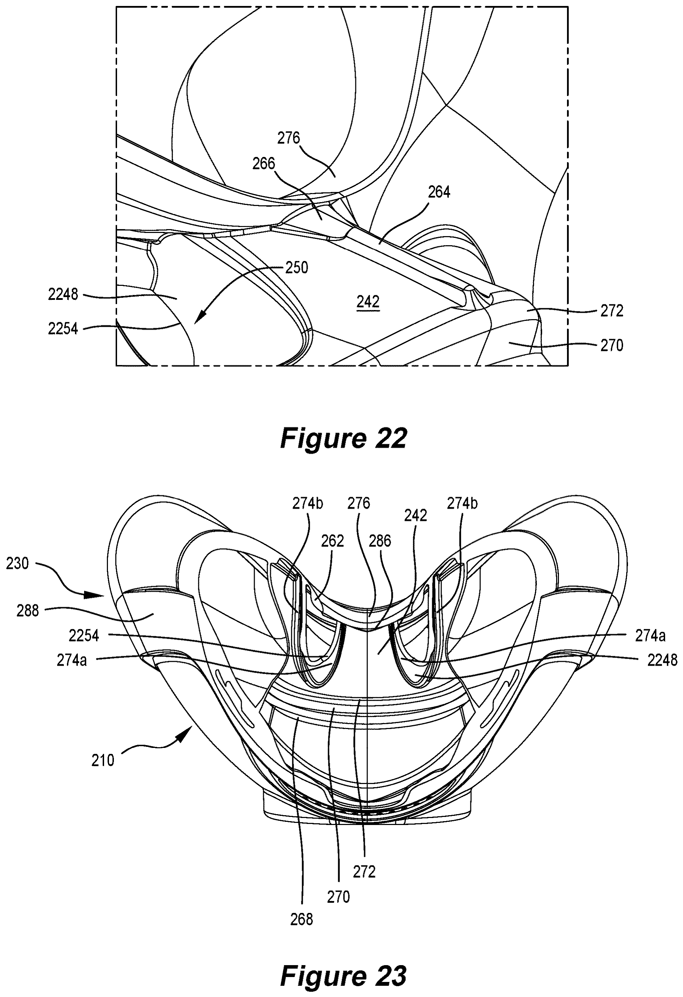

A further variation of the cushion module 20 is shown in Figures 16 to 23 as cushion module 2020. Features of the cushion module 2020 that are the same as the features of the cushion module 20 are denoted by the same reference numerals. Features that are not the same as the features of the cushion module 20 are denoted by like references number with a prefix comprising the number "2".

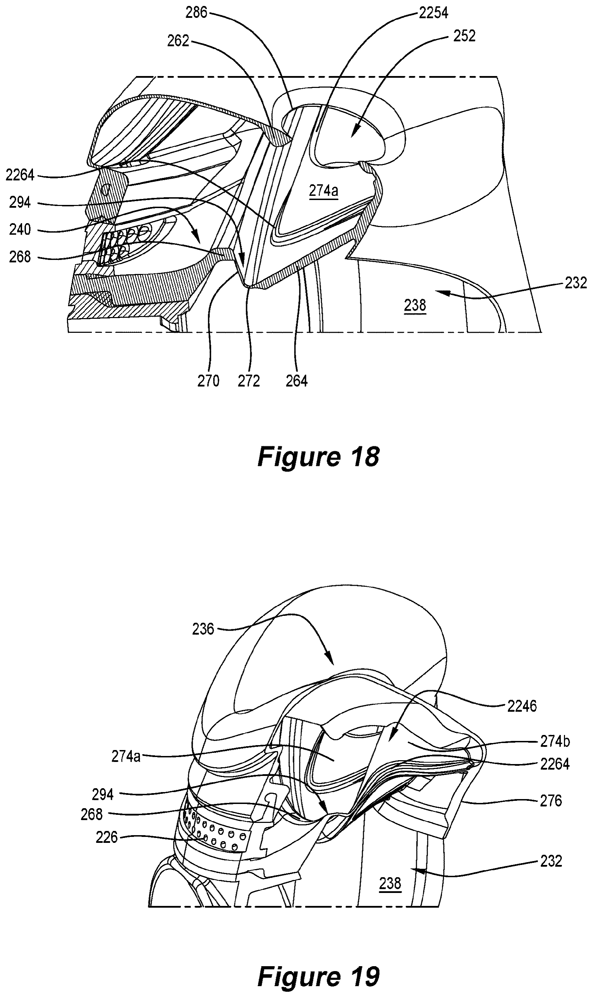

The cushion module 2020 differs from the cushion module 20 in that flow directors 2246 are linked to the outer wall 288 in the second chamber 240, as seen in Figures 19, 20 and 22. This means that respiratory gas flows through the channel that is formed in part by a body 2248 and in part by the outer wall 288. The link where the body 2248 meets the outer wall 288 is notionally shown by meeting line 2280 in Figures 16 and 17. The meeting line 282 between the dividing wall 242 and the outer wall 288 is also shown in Figures 16 and 17 where it can be seen that the meeting line 2280 intersects the meeting line 282.

The flow directors 2246 have an second opening 2252 which is defined partly by the rim 286 of the nare opening 234 and partly by a rim 2254. The rim 2254 extends from the outer wall 288 and is recessed from the nare opening 234, as shown in Figures 17, 18 and 20. The rim 2254 may extend from the rim 286 or may be set-back from the rim 286 so that the rim 2254 and rim 286 are not in direct contact. Accordingly, the second opening 2252 defined by the rims 2254 and 286 are recessed from the nare opening 234. This provide the same respiratory gas flow effects as described above in respect of the cushion module 20, namely that respiratory gas from the first chamber 238 can flow into the nares or into the second chamber 240 via flow directors 2246.

In this embodiment, the flow directors 2246 are spaced further apart than the flow directors 246 and 1246 in the cushion modules 20 and 1020, respectively. The additional spacing places the second opening 2252 in line with the laterally outer side of the patient's nares and provides a wider spacing of the contact points where the bodies 2248 meet the outer wall 288. The wider spacing of the contact points is anticipated to improve patient comfort by shifting the contacts points away from the patient's septum. Furthermore, as the flow directors 2246 are linked to the outer wall, they do not require an equivalent of the spacing rib 260 or 1260 (as in the embodiments described above) because the flow directors are unlikely to collapse laterally inwardly.

The body 2248 of each flow director 2246 is linked to the outer wall 288 between the nare opening 234 and the oral opening 232. More specifically, the body 2248 of each flow director 2246 is linked to the outer wall 288 at or adjacent to the nare opening 234.

The flow directors 2246 are spaced apart and part of the body 2248 joins with the outer wall 288 on a laterally outer side of a lateral rim 286 of the nare opening 234 and another part of the body 2246 joins with a laterally inner side of the lateral rim 286 of the nare opening 234. In this form, each body 2248 has a lateral wall 274a and a medial wall 274b, both of which extend from the wall section 276 and which meet each other away from the wall section 276. Additionally, the lateral wall 274a and the medial wall 274b extend from the dividing wall 242 to meet with the outer wall 288. The lateral wall 274a joins with the outer wall 288 on a laterally outer side of the lateral rim 286 of the nare opening 234 so that the rim 286 of the nare opening 234 forms part of the rim 2254 of the flow director 2246. The medial wall 274b joins with the outer wall 288 on a laterally inward side of the lateral rim 286 of the nare opening 234 so that part of the medial wall 274b forms part of the rim 2254 of the flow director.

The rim 2254 of the second opening 2252 formed by the medial wall 274b is closer to the dividing wall 242 than the rim 286 of the second opening 2252 formed by the lateral rim 286 of the nare opening 234. It follows that the second openings of the flow directors 2246 are defined in part by the nare opening 234. This configuration of the second opening 2252 may have the effect of directing respiratory gas laterally inwardly as it flows from the flow director 2246 into the second chamber 240.

In an alternative embodiment, the flow directors 2246 may be linked to the outer wall 288 in the first chamber 238 and in the second chamber 240. In a further alternative embodiment, the bodies 2248 may be linked to the outer wall 288 on a side of the nare opening 234 that is opposite to the wall portion 276.

Cushion module 2020 includes the reinforcement section 264 with the force-spreading end 266. Accordingly the primary and secondary deformation effects described above in respect of the cushion module 20 apply in the same way to the cushion module 2020.

The cushion module 2020 includes additional reinforcement sections 2264 where the bodies 2248 of flow directors 2246 meet the dividing wall 242, see Figures 17, 18 and 19. The reinforced sections 2264 are thickened fillets where the respective bodies 2248 meet the dividing wall 242. Accordingly, the reinforced sections 2264 extend from the outer wall 288 on one side of the bodies 2248 and return to the outer wall 288 on the other side of the bodies 2248. The location of the reinforcement sections 2264 serves to direct deformation forces applied to the wall portion 276 through the main portion 244 and into the deformation panel 294. The deformation forces are, therefore, directed through the reinforcement sections 2264 and around the base of the flow directors 2246 so they are likely to retain their shape when a deformation force is applied. In this configuration, the reinforcement section 264 does not overlap with the reinforcement sections 2264.

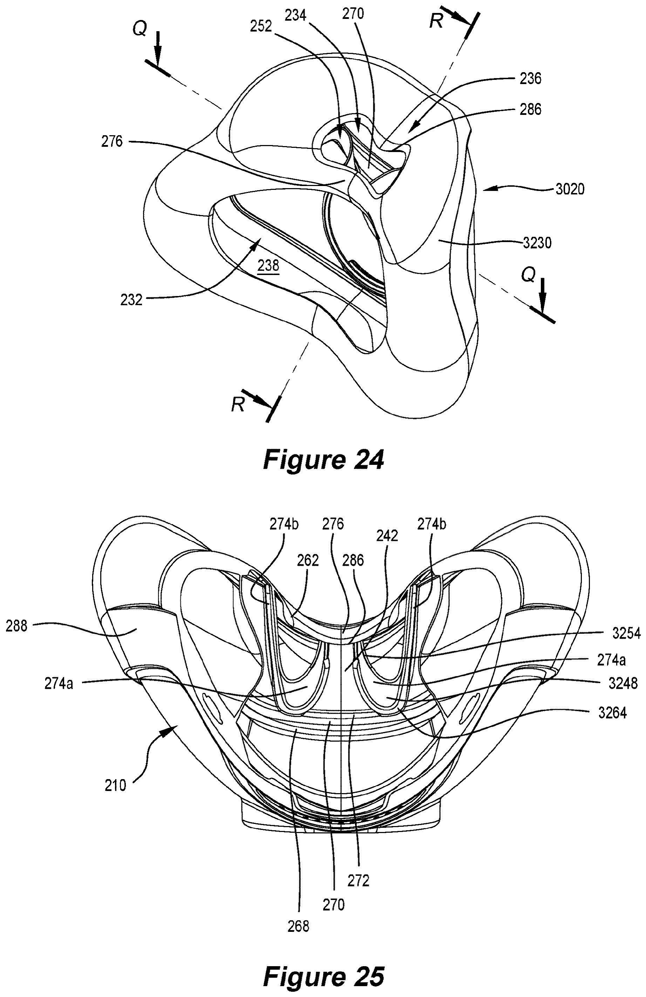

A further variation of the cushion module 20 is shown in Figures 24 to 29 as cushion module 3020. Features of the cushion module 3020 that are the same as the features of the cushion module 20 are denoted by the same reference numerals. Features that are not the same as the features of the cushion module 20 are denoted by like references number with a prefix comprising the number "3".

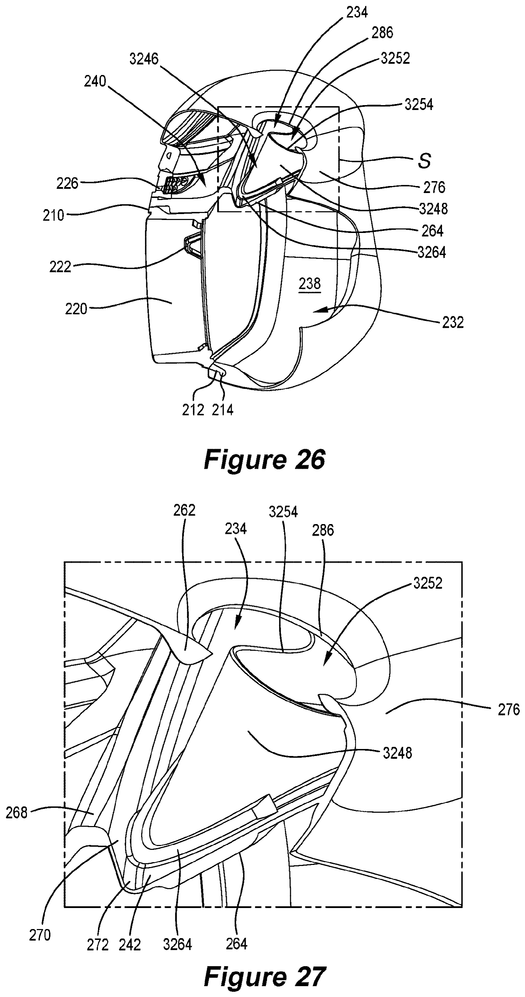

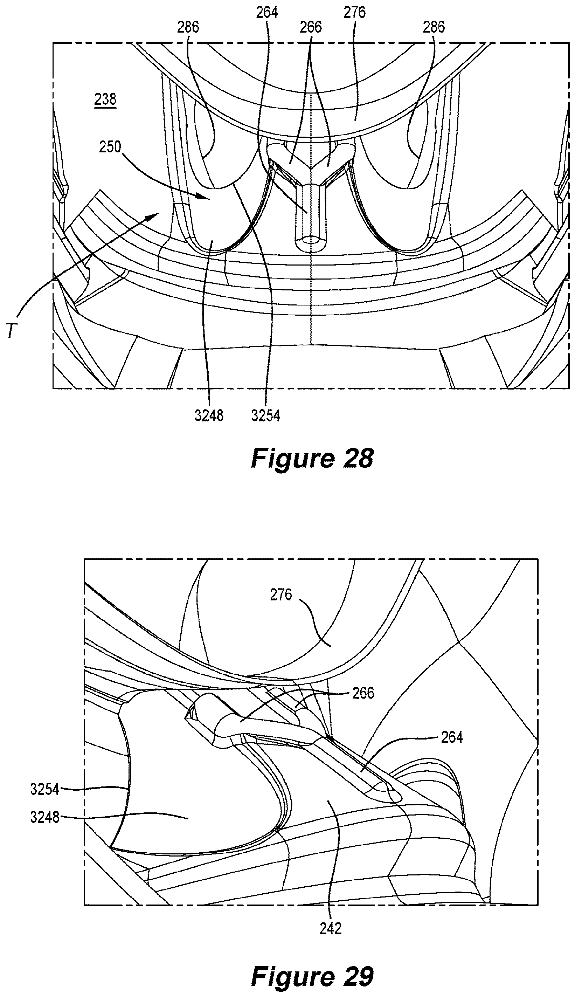

The cushion module 3020 is similar to the cushion module 2020 in that flow directors 3246 are linked to the outer wall 288, as seen in Figures 25 and 29. This means that respiratory gas flows through the channel that is formed in part by a body 3248 and in part by the outer wall 288. The link where the body 3248 meets the outer wall 288 is notionally shown by meeting line 3280 in Figure 24. The meeting line 282 between the dividing wall 242 and the outer wall 288 is also shown in Figure 26 where it can be seen that the meeting line 3280 intersects the meeting line 282.

The cushion module 3020 includes flow directors 3246 that have an second opening 3252 which is defined partly by the rim 286 of the nare opening 234 and partly by a rim 3254. The rim 3254 extends from the outer wall 288 and is recessed from the nare opening 234, as shown in Figures 26 and 27. The rim 3254 is set-back from the rim 286. In this embodiment, the rim 3254 joins the bead 262. In other embodiments, however, the rim may be set-back further by joining with the outer wall 288 adjacent to the bead 262. As shown in Figure 27, the rim 3254 extends away from the outer wall 288. In this way, the second opening 2252 defined by the rims 2254 and 286 are recessed from the nare opening 234. This provide the same respiratory gas flow effects as described above in respect of the cushion module 20, namely that respiratory gas from the first chamber 238 flows into the nares or into the second chamber 240.

The cushion module 3020 further differs from the cushion module 2020 in that the reinforcement section 264 (shown in Figures 28 and 29) has a bifurcated force-spreading end 3266. The force spreading end 3266 is spaced from the wall section 276. This is anticipated to reduce pressure experience by the patient through the wall section 276. The bifurcated force-spreading end 3266 is formed to spread slightly wider than the philtrum and/or septum on most patients. (It will be appreciated that the vast range of facial geometries can be accommodated by a set of patient interfaces that are different sizes). For a given patient interface size, the force spreading end 3266 is formed to spread slightly wider than the philtrum on most patients for whom that patient interface 10 size is suited.) The bifurcation is anticipated to ease pressure on the patient's philtrum applied through the wall portion 276 when the patient interface 10 is fitted and a treatment is applied. Additionally, it will be appreciated that by having two contact points due to the bifurcation the force applied through each contact point can be halved. This means that the wall thickness of the wall section 276 can be reduced because point-loading by the reinforcement section 264 is reduced. In this embodiment, the wall portion 276 has a wall thickness of 0.7 mm. Other embodiments may involve bodies 3248 and the wall section 276 having a different wall thickness.

Additional reinforcement sections 3264 are included where the bodies 3248 meet the dividing wall 242, see Figures 26 and 27. While the reinforcement sections 3264 do not extend to the outer wall 288 in this embodiment, they may extend to the outer wall in other embodiments. The location of the reinforcement sections 3264 serves to direct deformation forces applied to the wall portion 276 through the main portion 244 and into the deformation panel 294. The deformation forces are, therefore, directed through the bifurcated force spreading end 3266 which overlaps with the reinforcement sections 3264. This means that the deformation force is transferred through the reinforcement sections 3264 and around the base of the flow directors 3246, so they are likely to retain their shape when the deformation force is applied.

A further variation of the cushion module 20 is shown in Figures 30 to 40 as cushion module 4020. Features of the cushion module 4020 that are the same as the features of the cushion module 20 are denoted by the same reference numerals. Features that are not the same as the features of the cushion module 20 are denoted by like references number with a prefix comprising the number "4".

In this embodiment, the cushion module 4020 includes a dividing wall 4242, flow directors 4246 and a deformation region that is similar to the deformation region described above in respect of the cushion module 20. All are described in more detail below.

The dividing wall 4242 partitions the cushion module 4020 internally to define the first chamber 238 and the second chamber 240. The dividing wall 4242 separates the first chamber 238 from the second chamber 240 by extending all the way across the internal volume of the cushion module. In other words, the perimeter of the dividing wall 4242 seals with the outer wall 288.

In comparison to the dividing wall 242 of the cushion module 20, the dividing wall 4242 has a flatter, although still curved, profile which joins with laterally positioned reinforced portions 804 of the outer wall 288. The flatter profile reduces movement of the flow directors 4246 toward each other. It follows that the dividing wall 4242 is configured to reduce the collapse or buckling of the flow directors 4246. In each case, the dividing wall 4242 is configured to reduce collapse or buckling of the flow directors 4246 inwardly toward each other.

More specifically, the dividing wall 4242 in this embodiment has a flatter transverse profile than the dividing wall 242. As shown in Figures 30, 31 and 33, the position where the dividing wall 4242 joins the outer wall 288 is indicated by the meeting line 4282. The meeting line 4282 extends below the bias vent holes 226 and across the wall portion 276 and extends around the sides of the cushion module 4020 along a notional line that is contiguous with a line extending across the top of the housing 210.

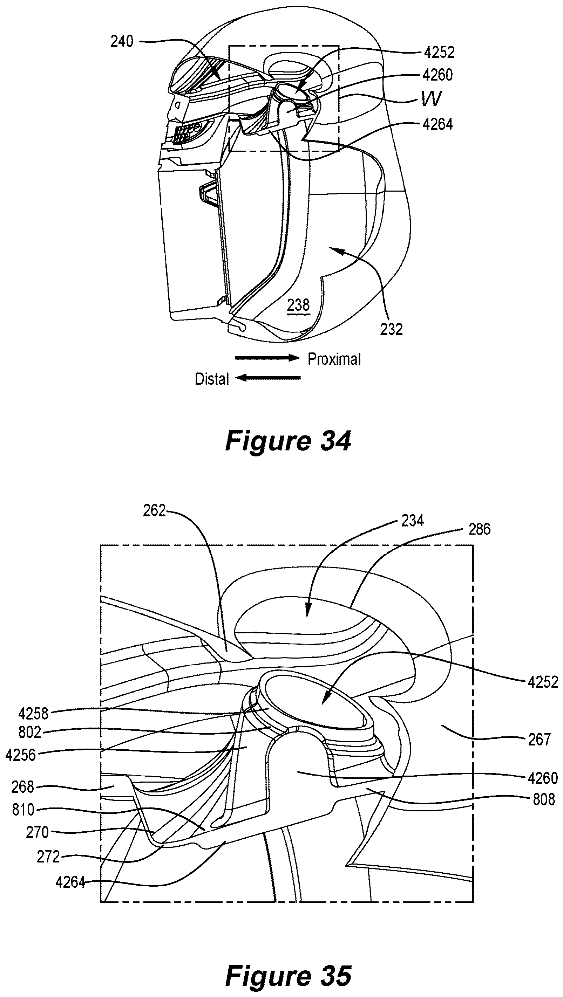

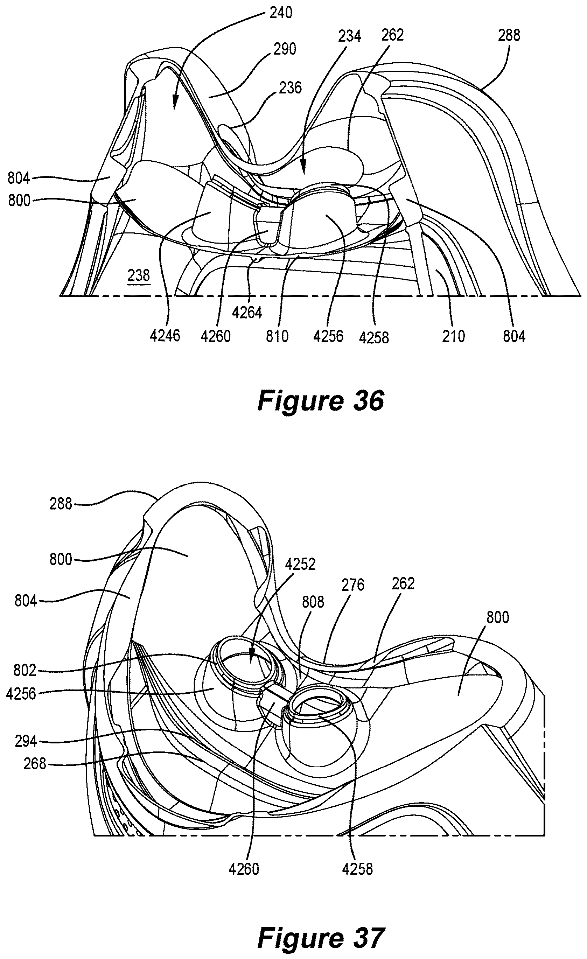

It can be seen in Figure 34 that the dividing wall 4242 includes flow directors 4246 disposed distally of the wall portion 276 and that a deformation panel 294 is disposed distally of the flow directors 4246. The deformation panel 294 is the same as the deformation panel 294 described above in respect of the cushion module 20 and operates in the same way in terms of controlled collapse under the influence of the deformation force applied to the wall portion 276. This means that the deformation panel 294 in the cushion module 4020 is configured to deform in preference to the flow directors 4246 and the main portion 244. The dividing wall 4242 includes lateral side portions 800 which are disposed laterally outwardly of the flow directors 4246 and which join with the outer wall 288. The lateral side portions 800 are proximal of the deformation region. In this embodiment, the deformation region is configured to deform also in preference to the lateral side portions 800. As shown in Figure 36, the lateral side portions 800 join the outer wall 288 at a position that is spaced from the top of the outer wall 288. In this particular embodiment, the level of the lateral side portions 800 where they join with the outer wall 288 is considerably below the level of the top of the outer wall 288. More specifically, the lateral side portions 800 join the outer wall 288 along a line (i.e. the meeting line 4282) that extends at least partly above and partly below the lowest level of the nare opening 234 and the meeting line 4282 does not extend above the highest level of the nare opening 234, having regard to an upright orientation of the patient interface 10.

In another embodiment, however, the lateral side portions 800 may join the outer wall 288 at a level below the lowest level of the nare opening 234. In a further embodiment, part of the lateral side portions 800 join the outer wall 288 along a line that extends between the highest and lowest levels of the nare opening 234 and that does not extend above the highest level of the nare opening 234, having regard to an upright orientation of the patient interface 10. In a further alternative embodiment, part of the lateral side portions 800 join with the outer wall 288 along a line that extends above the highest level of the nare opening 234 having regard to an upright orientation of the patient interface 10. In another alternative embodiment, the lateral side portions 800 join with the outer wall 288 at a level lower than or substantially equal to the uppermost level of the flow directors 4246, having regard to an upright orientation of the patient interface 10.

As a result of the flatter transverse profile of the dividing wall, the inclination of the lateral side portions 800 where they join with the outer wall 288, having regard to an upright orientation of the patient interface 10, is horizontal or is at an acute angle relative to horizontal. More specifically, the inclination of the lateral side portions 800 where they join with the outer wall 288, having regard to an upright orientation of the patient interface 10, is less than 45°, less than 30° or is less than 15° relative to horizontal. In most embodiments, the lateral side portion 800 will join with the outer wall 288 to form an angle in the second chamber 240 that is obtuse with an internal surface of the outer wall.

The seal member 4230 includes reinforced portions 804 (Figure 36) that resist deformation of the seal member 4230 and thereby assist with maintaining the shape of the seal member 4230. The reinforced portions 804 comprise portions which have a wall thickness that is greater than the wall thickness of other portions of the outer wall 288. The lateral side portions 800 join, at least in part, with reinforced portions 804 of the outer wall 288 which have a wall thickness that is greater than the wall thickness of the patient-contact surface 290 of the outer wall 288. For example, the lateral side portions 800 join with the reinforced portion 804 of the seal member 4230 which is over-moulded with the housing 210. Joining of the lateral side portions 800 with this over-moulded reinforced portion 804 of the seal member 4230 assists to retain the shape of the dividing wall 4242 by transferring deformation forces applied to the patient-contact surface 290 through to the reinforced portions 804, such as the over-moulded reinforced portion 804 of the seal member 4230.

As with other embodiments, the cushion module 4020 defines the internal volume which comprises the first chamber 238 and the second chamber 240. The second chamber 240 is located in an upper portion of the internal volume of the seal member 4230. The first resilient region 268 is positioned at a level which is higher than or equal to the bias vent holes 226, having regard to an upright orientation of the patient interface 10. The dividing wall 4242 joins with the wall portion 276 approximately midway between the nare opening 234 and the oral opening 232. Alternatively, the dividing wall 4242 may join with the wall portion 276 in an upper half of the wall portion 276.

Figures 34 to 40 show the flow directors 4246. The flow directors 4246 enable gas flow between the first chamber 238 and the second chamber 240. Each flow director 4246 has a base 806 which joins with the dividing wall 4242 and which defines the first opening 4250 into the first chamber 238, a body 4248 extending from the base 806 and a rim 4254 remote from the base 806. The rim 4254 defines a second opening 4252 which opens into the second chamber 240. As with other embodiments, a gas flow channel 812 extends from the first opening 4250 in the base 806 to the second opening 4252 of the rim 4254.

While this and other embodiments show the body 4248 extending from the dividing wall 4242 into the second chamber 240, it will be appreciated that other configurations of the flow directors 4246 may be adopted. An example of one such configuration involves at least part of the body 4248 extending into the first chamber 238. In other words, the body 4248 extends, in part, downwardly from the dividing wall 4242 and into the first chamber 238 and also extends, in part, upwardly from the dividing wall 4242 and into the second chamber 240. The base 806, in this configuration, joins the body 4248 to the dividing wall 4242. It has an annular form from which the lower body portion 4256 extends into the first chamber 238. The lower body portion 4256 includes the first opening 4250 in the first chamber. The upper body portion 4258 extends into the second chamber 240 from the base 806 and includes the second opening 4252. This configuration may be combined with any of the features described in the embodiments above and in the embodiments to follow. In another embodiments, the flow directors 4246 may not extend from either side of the dividing wall 4242. For example, the dividing wall 4242 may have a wall thickness that is sufficient to include a flow director 4246 between the surfaces of the dividing wall 4242 exposed to the first chamber 238 and to the second chamber 240 respectively.

Figures 34, 35 and 39 show that the body 4248 of each flow director 4246 includes a transition that is intermediate the base 806 and the rim 4254. The transition 802 delineates the lower body portion 4256 from the upper body portion 4258. The lower body portion 4256 extends from the base to the transition. The upper body portion extends from the transition to the rim 4254. In this embodiment, the transition comprises, at least in part, a step 802 and the upper body portion 4258 extends from the step 802 to the rim 4254. The step 802 is formed on the exterior of the flow director 4246. The upper body portion 4258 has a wall thickness that is less than the wall thickness of the lower body portion 4256. Therefore, the step 802 joins the different wall thicknesses of the upper body portion 4258 and the lower body portion 4256. The step 802 extends all the way around the flow director 4246. Accordingly, there is a step-change in wall thickness of the flow directors 4246 from the lower body portion 4256 to the upper body portion 4258. The step-change in wall thickness of the flow directors 4246 occurs on the exterior surface of the flow director 4246 such that the interior surface of the flow director 4246 is smooth. The upper body portion 4258 extends from the step 802. The upper body portion 4258 terminates at the rim 4254 which is spaced from the nare opening 234. The upper body portion 4258 has a wall thickness that is less than the wall thickness of the lower body portion 4256. The thinner wall thickness of the upper body portion 4258 makes it readily deformable and, therefore, there is less discomfort for a patient should the upper body portion 4258 come into contact with the patient's nose. The greater wall thickness of the lower body portion 4256 increases the resistance of the flow director 4246 to collapse or buckling under the influence of a deformation force. The wall thickness is selected to increase the resistance to deformation so that deformation forces applied to the wall portion 276 are transferred through the dividing wall 4242 around the flow directors 4246 and into deformation panel 294. This way, the deformation panel 294 deforms in preference to the flow directors 4246 and, therefore, the flow directors 4246 are more likely to retain their shape and retain a capacity to transfer a desired flowrate of gas between the first chamber 238 and second chamber 240 when a deformation force is applied. In other words the flow directors 4246 are less likely to occlude when a deformation force is applied.

As shown in figures 35 and 36, the step 802 is inclined relative to the dividing wall 4242. The step 802 is further from the dividing wall 4242 on a distal side of the flow director 4246 and is closer to the dividing wall 4242 on a proximal side of the flow director 4246. Additionally, the step 802 is also inclined downwardly in a laterally inward direction toward a vertical mid-plane through the patient interface 10, having regard to an upright orientation of the patient interface 10. This means that the step 802 is further from the dividing wall 4242 on the laterally outer side of the flow director 4246 than the step is from the dividing wall 4242 on the laterally inner side of the flow director 4246. The lateral and proximal inclination of the step 802 provides a substantially constant spacing between the step 802 and the nare opening 234.

The upper body portion 4258 extends from the step 802 by a constant distance about the flow director 4246. This means that the rim 4254 has the same inclination as the step. That is, the rim 4254 and, therefore, the second opening 4252, has an inclination which provides a substantially constant spacing between the rim 4254 and the nare opening 234. However, compared to other embodiments disclosed previously, the rim 4254 is set-back further from the nare opening 234. The increased set-back further facilitates gas flow from the first chamber 238 to the second chamber 240, such as respiratory gas exhaled orally by a patient. It also facilitates gas flow from the nares via the nare opening 234 and into the second chamber 240, such as respiratory gas exhaled via the nares of the patient.

In the illustrated embodiment, the step 802 extends substantially all the way around the flow director 4246. However, the transition may comprise the step 802 in only parts of the flow director 4246. For example, in some embodiments the step 802 may extend around at least the distal side of the flow director 4246. In other embodiments, the step 802 may extend around the distal and lateral outer sides of the flow director 4246.

In this embodiment, the radial wall thickness of the lower body portion 4256 is constant circumferentially around the flow director 4246. The radial wall thickness of the upper body portion 4258 is also constant circumferentially around the flow director 4246. This means that the step 802 has a constant wall thickness change around the flow director 4246 from the lower body portion 4256 to the upper body portion 4258. In other embodiments however, the radial wall thickness of the lower body portion 4256 may vary about the flow director 4246, having regard to the longitudinal axis of the flow director 4246. In further embodiments, the radial wall thickness of the lower body portion 4256 may vary circumferentially around the flow director 4246. Alternatively, the radial wall thickness of the lower body portion 4256 may vary with the spacing of the rim 4254 from the base 806. For example, the radial wall thickness of the lower body portion 4256 may be greater where the rim 4254 is spaced further from the base 806 than where the rim 4254 is spaced less from the base 806. As another example, the radial wall thickness of the lower body portion 4256 is least where the spacing between the rim 4254 and the base 806 is the least and the radial wall thickness increases with increasing spacing between the rim 4254 and the base 806. The reduced radial wall thickness of the upper body portion 4258 relative to the lower body portion 4256 makes it more compliant and, therefore, there is less discomfort experience by a patient in the event their nose contacts the upper body portion 4258.

The dividing wall 4242 further includes a spacing rib 4260 disposed between the bodies 4248 of the flow directors 4246, as shown in figures 34 to 36. The spacing rib 4260 joins with the bodies 4248 at a position spaced from the rim 4254. In this embodiment, the spacing rib 4260 extends from the dividing wall 4242 between the flow directors 4246 and joins with the base 806, lower body portion 4256, the step 802 and the upper body portion 4258, but is spaced from the rim 4254. Spacing the spacing rib 4260 from the rim 4254, and therefore further from the nare opening than the rim 4254 is from the nare opening, reduces the chance that the patient's nose will come into contact with the spacing rib 4254 during use. Spacing rib 4260 is disposed in a plane which intersects the longitudinal axes of the flow directors 4246. Accordingly, the spacing rib 4260 is disposed mid-way between the proximal and distal sides of the flow directors 4246.

As shown in Figure 35, the spacing rib 4260 has an inverted U-shaped profile. The spacing rib 4260 reinforces the flow directors 4246 and makes them more resilient to buckling inwardly or outwardly. Reducing the likelihood of buckling assists to retain the alignment of the flow directors 246 in a direction that, in use, directs a flow of respiratory gas toward the nare opening 234. Furthermore, the position of the spacing rib 4260 and the reinforcement section 4264 means that deflection of the wall portion 276 transferred through the spacing rib 4260 and the reinforcement section 4264 and, therefore, causes the flow directors 4246 to track movement of the wall portion 276 and the nare opening 234.

The spacing rib 4260 has a generally upright orientation, having regard to an upright orientation of the patient interface 10. Given that the dividing wall 4242 is inclined downwardly from the wall portion 276 to the deformation panel 294, the spacing rib 4260 is inclined relative to the dividing wall 4242. That is, the spacing rib 4260 joins the dividing wall 4242 at its proximal side in an acute angle. The spacing rib 4260 includes a fillet-shaped transition joint with the flow directors 4246. This provides a smooth transition 802 from the spacing rib 4260 to the base 806 and to the body 4248 of the flow directors 4246.

The thickness of the spacing rib 4260 in the distal-proximal direction is considerably greater than the wall thickness of the flow directors 4246. More specifically, the spacing rib 4260 has a wall thickness in the distal-proximal direction that is 5 to 20%, 20 to 40% or 40 to 60% of the maximum width dimension of the flow director 4246.

The spacing rib 4260, Figures 35, 36 and 38, overlaps a reinforcement section 4264 which is arranged on an underside of the dividing wall 4242 between the respective first openings 4250 of the flow directors 4246. The reinforcement section 4264 comprises a thickened section of the dividing wall 4242. The reinforcement section 4264 extends in a distal-proximal direction. Furthermore, it is spaced from the wall portion 276 and from the deformation panel 294. The reinforcement section 4264 increases the stiffness of the dividing wall 4242 in the region between the wall portion 276 and the deformation panel 294 and, therefore, contributes to transferring deformation forces applied to the wall portion 276 around the flow directors 4246 and through to the deformation panel 294. Accordingly, the reinforcement section 4264 assists to retain the position of the dividing wall 4242 (and the flow directors 4246) relative to the nare opening 234. In other words, the reinforcement section 4264 assists to brace the dividing wall in position relative to the wall portion 276. This enables a patient treatment to continue with little interference to the flow of respiratory gas (a) through the flow directors 4246, (b) through the nare opening 234 and (c) through the second chamber 240 during deformation of the patient contacting surface 290 of the outer wall 288.

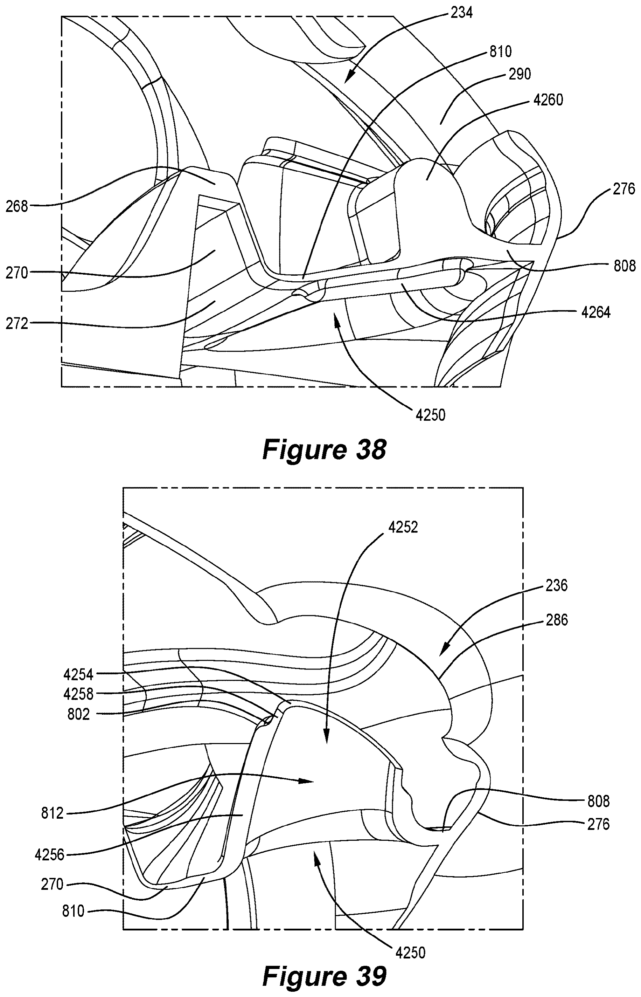

The reinforcement section 4264 is assisted in transferring deformation forces from the wall portion 276 through to the deformation panel 294 by portions 808, 810 of the dividing wall 4242 having a wall thickness that is greater than the wall thickness of other parts of the dividing wall 4242. The portions 808, 810 are respectively proximal and distal of the flow directors 4246. In some embodiments, proximal and distal portions 808, 810 are respectively proximal and distal of the spacing rib 4260. In some embodiments, the proximal and distal portions 808, 810 include the respective portions of the dividing wall 4242 that are respectively proximal and distal of the flow directors 4246. The proximal portion 808 is between the lateral side portions 800 of the dividing wall 4242. Alternatively, the proximal portion may be between the flow directors 4246. The distal portion 810 is between the lateral side portions 800 of the dividing wall 4242. For embodiments that don't include the spacing rib 4260, the proximal and distal portions 808, 810 are respectively proximal and distal of a notional line through the centres of the respective second opening 4252 of the flow directors 4246. The wall thickness of the distal portion is represented by the wall thickness excluding the wall thickness of the reinforcement section 4264. Figure 36 shows a distal portion 810 which has a wall thickness that is greater than the wall thickness of the lateral side portions 800. The distal portion 810 extends from the spacing rib 4260 and around the bases 806 of the flow directors 4246. The distal portion 810 also extends toward the deflection panel but is spaced from the second wall 272 of the deformation panel 294. Accordingly, the distal portion 810 overlaps the reinforcement section 4264. The same applies to the proximal portion 808, as shown in Figures 35 and 38. The proximal portion 808 joins with the spacing rib 4260 and overlaps the reinforcement section 264. The proximal portion 808 extends proximally from the spacing rib 4260 and around the bases 806 of the flow directors 4246. The proximal portion 808 joins with the wall portion 276.

These portions 808, 810, on account of their greater wall thickness compared to other parts of the dividing wall, have a greater resistance to deformation. It follows that deformation forces imparted upon the wall portion 276 are transferred through the dividing wall 4242 to the deformation panel 294 which will deform in preference to the portions 808, 810. The presence of the portions 808, 810 in conjunction with the reinforcement section 276 and the spacing rib 4260 resist undesirable deformation of the dividing wall 4242 by improving the transfer of deformation forces into the deformation panel 294. Therefore, the likelihood of the flow directors 4246 collapsing or buckling on account of the dividing wall 4242 buckling is reduced. There is a limit, however, to the wall thickness of the portions 808, 810 because increasing the wall thickness of the portions has the effect of localising the pressure forces experienced by the patient at the wall portion 276. In other words, increased wall thickness of the portion increases the likelihood of pressure sores. Conversely, making the wall thickness too small will result in buckling of the dividing wall 4242 and possible buckling of the flow directors 4246 because the deformation forces aren't transferred to the deformation panel 294.

The greater wall thickness of the portions 808, 810 means that the reinforcement section 4264 doesn't need to extend all the way to the wall portion 276 or all the way to the deformation panel 294 in order to transfer deformation forces from the wall portion 276 into the deformation panel 294.

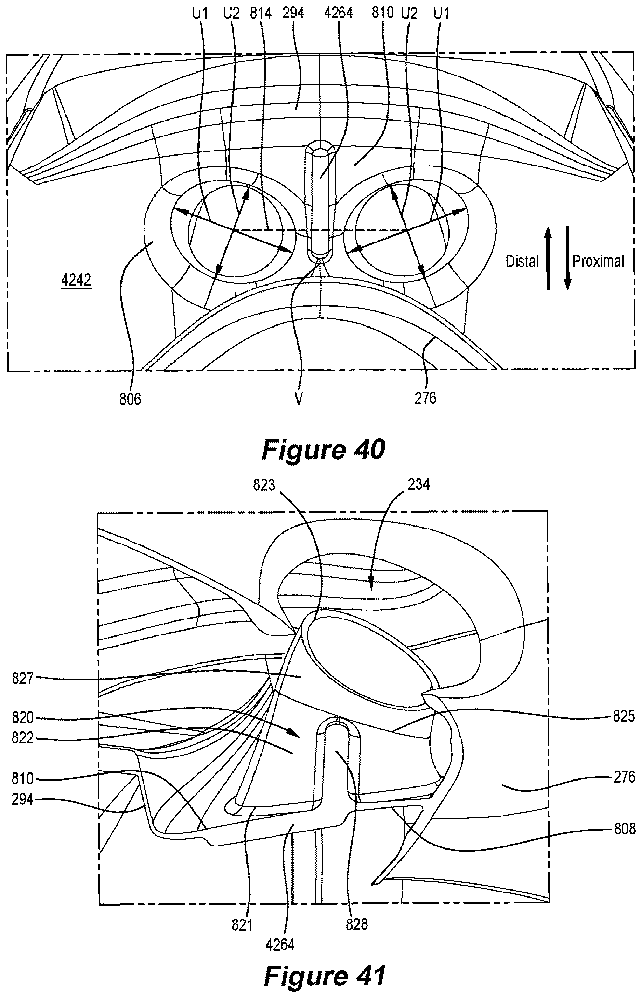

Resistance to flow through the flow directors 4246 is reduced by adopting a larger first opening 4250 and larger second opening 4252 compared to the flow directors 246 of the cushion module 20. The comparison between the first openings 250 of the cushion module 20 in Figure 11 with the first openings 4250 of the cushion module 4020 in Figure 40 provides an example of the size difference between the flow directors 4246 and the flow directors 246. In order to accommodate the larger size of the first openings 4250, the shape and orientation of the first opening 4250 differs to allow the flow directors 4246 to fit between the wall portion 276 and the deformation panel 294. The adopted shape shown in Figure 40 enables the flow directors 4246 to be spaced from the wall portion 276 and the deformation panel 294. This reduces the extent to which the wall portion 276 is stiffened by the base 806. Stiffening of the wall portion 276 can reduce patient comfort by forming a localised pressure point which can lead to patient soreness over a period of time. Spacing the flow directors 4246 from the deformation panel 294 provides additional displacement travel for the deflection panel 204 to deform and, thereby, to absorb deformation forces. That is, limiting the extent of travel in the deformation panel 294 limits the extent of the deformation forces that can be absorbed before other features of the seal member 4230, such as the dividing wall 4242 or the flow directors 4246, begin to buckle under the deformation force. Such uncontrolled buckling is undesirable because it can misalign the flow directors with the nare openings, impede gas flow through the flow directors or even occlude gas flow through the flow directors.

As shown in Figure 40, the first opening 4250 has a shape with a major axis U1 that is longer than an orthogonal minor axis U2. In this embodiment, the first openings 4250 have an elliptical shape. However, it will be appreciated that alternative shapes having a major axis U1 and an orthogonal minor axis U2 may be adopted instead of an elliptical shape. To assist with fitting the first opening 4250 between the wall portion 276 and the deformation panel 294, the major axis U1 is within a plane of the dividing wall 4242 and is oriented at least 45° from a vertical mid-plane through the patient interface 10. Therefore the dimension of the first opening in the proximal-distal direction is reduced compared to if the major axis U1 was parallel to the vertical mid-plane. In the embodiment shown in Figure 40, the major axes U1 of the respective first openings 4250 intersect at a point that is on a proximal side of a notional straight line 814 (Figure 40) that passes through the centres of the first openings 4250. In some embodiments, the major axes U1 of the respective first openings 4250 may intersect at a point that is proximal of patient-contact portion 290 of the outer wall 288. Either way, an angle (V) between major axes U1 where they intersect is less than 180°. The angle V may be in the range of 45° to less than 180°, in the range of 90° to 150° or in the range of 110° to 150°. In other embodiments, the major axes U1 of the respective first openings 4250 may intersect at a point that is on a distal side of the straight line that passes through the centres of the first openings 4250. In this alternative, the angle V between the major axes U1 falls within the ranges as described above.

The orientation of the major and minor axes U1, U2 is retained throughout the flow director 4246. However, the dimension of the flow director 4246 along the major axis U1 and minor axis U2 at the second opening 4252 are different from the dimension of the flow director 4246 along the major axis U1 and the minor axis U2 at the first opening 4250. Specifically in this embodiment, the dimension of the flow directors 4246 along both the major and minor axes U1, U2 at the second opening 4252 is smaller than the dimension of the flow directors 4246 along the respective major and minor axes U1, U2 at the first opening 4250. In other embodiments, the orientation of the major and minor axes U1, U2 at the second opening 4252 are different from the orientation of the major and minor axes U1, U2 at the first opening 4250. Alternatively, the orientation of the major and minor axes U1, U2 may vary through the flow director 4246 from the first opening 4250 to the second opening 4252. In other embodiments, the dimension of flow director 4246 along one of the major axis U1 or the minor axis U2 at the second opening 4252 may be different from the dimension of the flow director 4246 along the corresponding axis at the first opening 4250. For example, the dimension of the flow director 4246 along only one of the major axis U1 or the minor axis U2 at the second opening 4252 is less than the dimension of the flow director 4246 along the corresponding axis at the first opening 4250. Alternatively, the dimension of the flow director 4246 along the major axis U1 or the minor axis U2 or both the major and minor axes U1, U2 varies through the flow director 4246 from the first opening 4250 to the second opening 4252. In other embodiments, the orientation and the dimension of the flow director 4246 along the major and minor axes U1, U2 at the second opening 4252 are different from the orientation and dimension of the flow director 4246 along the major and minor axes U1, U2 at the first opening 4250.

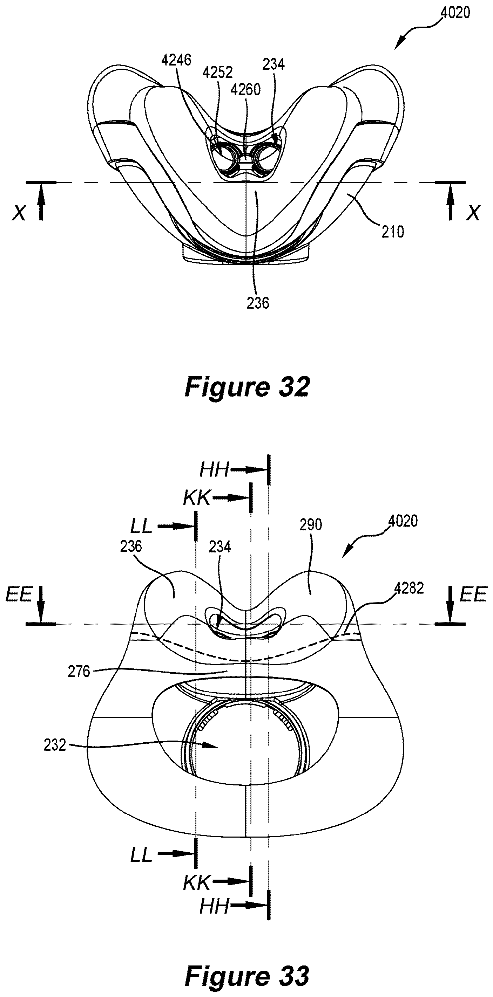

The positioning of the second openings 4252 relative to a vertical mid-plane through the patient interface 10 is selected to direct gas flowing from the first chamber 238 to the second chamber 240 through the nare opening 234 and into the nares of a patient. However, the risk is that if the second openings 4252 are disposed too closely together, gas flow may be directed into the septum of a patient. This can cause patient discomfort, undesirable turbulence and issues delivering sufficient flow to the nares. Accordingly, the second openings 4252 are sufficiently spaced apart to direct gas flow into the nares and to substantially avoid directing gas flow into the septum of the patient. In this embodiment, with the orientation of the major and minor axes U1, U2 and with the spacing of the second openings 4252, the outer wall 288 extends over part of the second opening 4252 when the patient interface 10 is not fitted to a patient. This can be seen in Figures 32 and 40 where laterally outer parts of the rim 286 of the nare opening 234 extend over part of each second opening 4252 when the patient interface 10 is not fitted. That is, part of the outer wall 288 surrounding the nare opening 234 extends over laterally outer parts of the respective second openings 4252 when viewed vertically above the cushion module 4020, having regard to an upright orientation of the cushion module 4020.

In the view shown in Figure 32, at least part of each second opening 4252 is obscured by the outer wall 288 when the patient interface 10 is viewed from vertically above a mid-point between the second openings 4252, having regard to an upright orientation of the patient interface 10 when the patient interface 10 is not fitted. However, the outer wall 288 is configured to enable the nare opening 234 to extend laterally outwardly when fitted to a patient. This mainly results from the nare sealing portion 236 being curved to receive the underside of the patient's nose, when the patient interface 10 is fitted to a patient, so that the curvature reduces when the patient interface 10 is fitted. However, this is assisted by the outer wall 288 being formed of a resilient material. The nare sealing portion 236 has a relatively thin wall thickness, compared with other parts of the outer wall 288, so that the resilient material forming the outer wall 288 enables the nare opening 234 to expand laterally outwardly when fitted to a patient. In other words, the patient-contact surface 290 includes a valley-shaped nare sealing portion 236 that is configured to receive the underside of a patient's nose. A floor of the valley-shape, which includes the nare opening 234, adopts a flatter valley-shape when the patient interface 10 is fitted to a patient. As a result, when the patient interface 10 is fitted to a patient, the laterally outer edges of the nare opening 234 are displaced laterally outwardly of the second openings 4252. The result is that when the patient interface 10 is fitted, a gas stream through the flow directors 4246 will be directed through the nare opening 234 without impinging upon the outer wall 288.

The curvature of the outer wall 288 and the formation of the outer wall from a resilient material contributes to the area, shape or shape and area of the nare opening 234 changing when the patient interface 10 is fitted to a patient. Such change enables a gas stream passing through the flow director to be directed through the nare opening 234 without impinging upon the outer wall 288.

Alternative forms of flow directors, spacing ribs, reinforcement sections, distal portions and proximal portions may be utilised in place of the flow directors, spacing ribs, reinforcement sections, distal portions and proximal portions described above for the cushion module 4020. Some alternative embodiments of these components are discussed below. It will be appreciated that different alternative embodiments may be combined together in the cushion module 4020. For example, any one of the alternative flow directors 246, 1246, 2246, 3246 and 4246 discussed above or any one of the flow directors 820, 830, 840, 850, 860, 870, 880 or 890 described below may be combined in the cushion module 4020 with any one of the range of alternative spacing ribs 260, 1260 discussed above or ay one of the spacing ribs 828, 838, 848, 888, 898 discussed below.

Features of the following embodiments that are the same as the features of the cushion module 4020 are denoted by the same reference numerals.

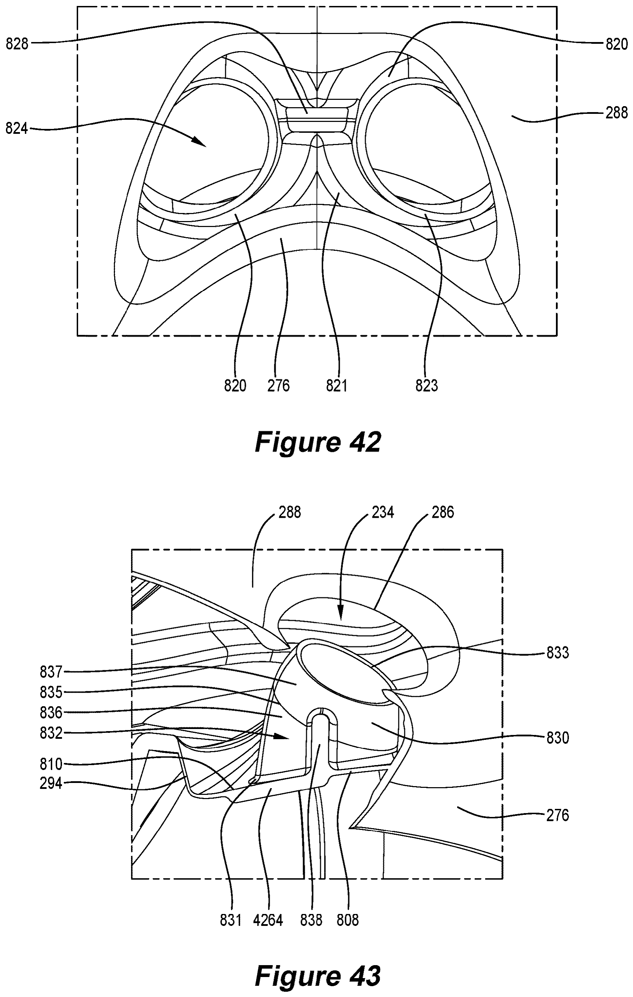

One alternative flow director 820 is shown in Figure 41. The flow director 820 has the same general form as the flow director 4246 in that it includes a base 821, a body 822 extending from the base 821 and a rim 823. The base 821 joins the body 822 to the dividing wall 4242. The base 821 forms a smooth transition surface between the body 822 and in the dividing wall 4242. Like the flow director 4246, part of the outer wall 288 extends over the laterally outer sides of a second opening 824 defined by the rim 823. This is shown in Figure 42. As explained above in respect of the cushion module 4020, the nare opening 234 expands laterally when the patient interface 10 is fitted so that the nare opening 234 extends laterally outwardly so that the outer wall 288 doesn't extend over the second openings 824.

The flow director 820 differs from the flow director 4246 in that it has a differently shaped body 822 and a differently shaped spacing rib 828. The body 822 includes a transition 825 which delineates a lower body portion 826 from an upper body portion 827. The lower body portion 826 has a wall thickness which decreases from the base 821 to the transition 825. The upper body portion 827 has the same wall thickness from the transition to the rim 823. The lower body portion 826 is angled in the proximal direction so the proximal side of the lower body portion 826 extending from the base 821 to the transition 825 is shorter than the distal side of the lower wall portion 826 extending from the base 821 to the transition 825. The upper body portion 827 is angled proximally and laterally inwardly. In other words, the rim 823 is inclined so that it is spaced further from the dividing wall 4242 on the laterally outer distal side of the flow director than the rim 823 is spaced from the dividing wall 4242 on the laterally inner proximal side of the flow director 4246. The transition 825 occupies a plane that is inclined in the same manner as the lower body portion 826. The same angular direction is present in the flow director 4246. The angling of the lower and upper body portions 826, 827 and the lateral positioning of the flow directors 820 facilitates direction of respiratory gas through the one of more nare openings and into the nares from the flow directors 820.

The base 821 of each of the flow directors is joined at a vertical mid-plane through the cushion module 4020 (as seen in Figure 42). The spacing rib 828 spans the gap between the flow directors 820. The spacing rib 828 extends from the bases 821 to the transition 825 on each flow director 820. The top of the spacing rib 828 forms a straight line between the flow directors 820. The spacing rib 828 overlaps with the reinforcement section 4264 on the underside of the dividing wall 4242. The proximal and distal portions 808, 810 of the dividing wall 4242 have a wall thickness that is the same as the wall thickness of the lateral side portions 800.

Another alternative flow director 830 is shown in Figure 43. The flow director 830 has the same general form as the flow director 820 in that it has a base 831, a body 832 and a rim 833. The body includes a transition 835 which delineates a lower body portion 836 from an upper body portion 837. However, the flow director 830 differs in that the lower body portion 836 has a substantially constant wall thickness from the base to the transition. The transition 835 is inclined in the proximal direction so that it is adjacent to the base 831 on the proximal side of the body 832. In another embodiment the transition 835 joins with the base 831 on the proximal side of the body 832. The inclination of the transition 825 means that the lower body portion 836 on the proximal side of the body 832 will exert less pressure on the wall portion 276 in the event that the wall portion 276 is pressed against the flow director 830.

The wall thickness of the upper body portion 837 tapers inwardly from the transition 835 to the rim 833. This is shown in the cross-sectional view in Figure 44. In this particular embodiment, the outer wall of the body 832 tapers inwardly towards an inner wall of the body 832. This provides the body 832 with a curved external profile.

The flow directors 830 include a spacing rib 838 in the same size and shape as the spacing rib 828 of the flow directors 820. However, as the transition 835 is lower on the body 832 than the transition 825 on the body 822, the spacing rib 838 extends from the base 831 to a point between the transition 835 and the rim 833.

A further alternative flow director 840 is shown in Figure 45. The flow director 840 has the same general form as the flow director 820 in that it has a base 841, a body 842 and a rim 843. The body 842 includes a transition 845 which delineates a lower body portion 846 from an upper body portion 847. The transition 845 comprises, in part, a step 844 which is intermediate the base 841 and the rim 843. However, the step 844 joins the upper body portion 847 with the base 841 on the proximal side of the body 842 due to the inclination of the step 844. The proximal side of the flow director 840 is proximal of a notional centre line in the lateral direction. The lower body portion 846 below the step 844 has a first wall thickness and the upper body portion 847 above the step 844 has a second wall thickness. The first wall thickness may vary about the lower body portion 846. The second wall thickness is less than the first wall thickness. The greater wall thickness of the lower body portion 846 provides the flow director 840 with resistance to deformation in the event that a deformation force is applied to the wall portion 276. The resistance to deformation contributes to directing deformation forces applied to the wall portion 276 through the dividing wall and into the deformation panel 294. The lesser wall thickness of the upper body portion 847 makes it more compliant and softer to touch in the event that the rim 843 comes into contact with the patient.

Having regard to the longitudinal axis of the flow director 840, the radial wall thickness of the upper body portion 847 is constant around the flow director 840. However, the radial wall thickness of the lower body portion 846 varies about the flow director. In this embodiment, the radial wall thickness of the lower body portion 846 varies with the spacing of the rim 843 from the base 841. More particularly, the radial wall thickness of the lower body portion 846 is greater where the rim 843 is spaced further from the base 841 than where the rim 843 is spaced less from the base 841. In this embodiment, the wall thickness on the distal side of the lower body portion 846 is greater than the wall thickness on the proximal side of the lower body portion 846. In one alternative embodiment, the radial wall thickness of the lower body portion 846 is least where the spacing between the rim 843 and the base 841 is the least and increases with increasing spacing between the rim 843 and the base 841. In another alternative embodiment, the wall thickness of the lower body portion 846 is constant around the flow director 840.

The step 844 extends at least around the distal side of the flow director 840. As seen in Figure 45, the step 844 comprises an inclined surface which joins the lower body portion 846 with the upper body portion 847. The inclination of the step 844 changes about the flow director 840. More specifically, the inclination is steeper on a proximal side of the body 842 and is less inclined on the distal side of the body 842. As a result, the profile of the step 844 changes depending upon the position around the body 842. This can be seen in Figure 45 where the proximal side of the body 842 has a steep step 844 and the distal side of the body 842 has a less steep step 844. In this particular embodiment, the step 844 extends all the way around the body 842 and joins the base 841 with the upper body portion 847 on proximal side of the flow director 840.

The spacing ribs 828 and 838 associated with flow directors 820 and 830 respectively are the same. However, the spacing rib 848 associated with the flow director 840 has a square or rounded square cross-sectional profile. The spacing rib extends from the dividing wall 4242 and bridges the gap between the flow directors 840. The spacing rib 848 extends up the base 841 and the lower body portion 846 to the step 844. That is, the spacing rib 848 terminates at the top of the lower body portion 846 where the spacing rib 844 meets the step 844. A top surface of the spacing rib 848 is flattened and is inclined so that the distal side of the spacing rib 848 extends further from the dividing wall 4242 than the proximal side of the spacing rib 848 extends from the dividing wall 4242. The inclination of the spacing rib 848 follows the proximal-distal inclination of the step 844 where the spacing rib 848 meets the step 844.

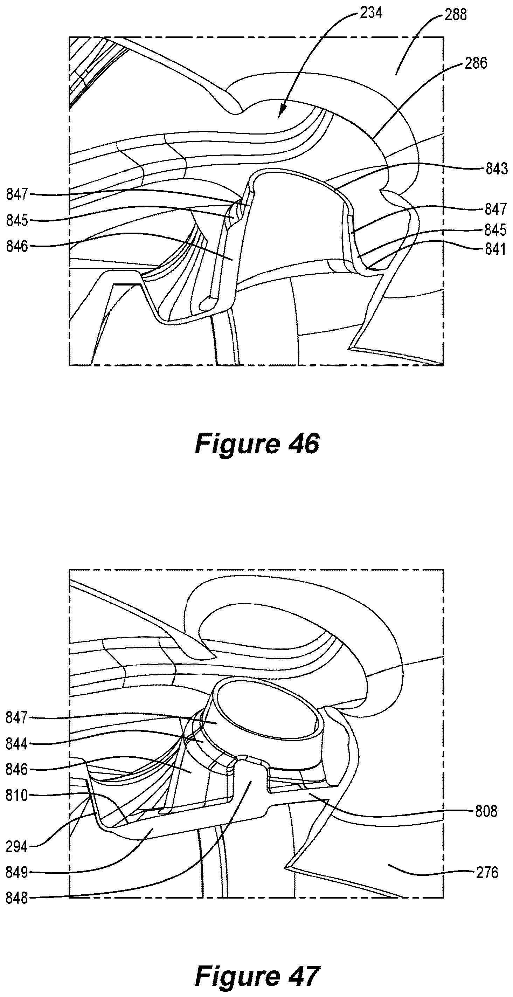

An alternative embodiment of the spacing rib 848 is shown in Figure 47 with the same flow director 840. In this embodiment, the spacing rib 848 is the same as the spacing rib shown in Figure 45 but joins with the base 841, the lower body portion 846 and the step 844. As can be seen in Figure 47, the spacing rib 848 includes a fillet-shaped transition joint with the flow director 840.

As with other embodiments, the spacing rib 848 overlaps with the reinforcement section 849. This arrangement contributes to transferring deformation forces applied to the wall portion 276 to the deformation panel 294. The same configuration is present in the embodiments described above for flow directors 820 and 830. Those flow directors 820, 830 are associated with the same reinforcement section 4264 that is present in the cushion module 4020. However, the effect is the same in that the deformation forces are transferred by the respective lower body portions 826, 836 to associated spacing ribs 828, 838 and then transferred through the reinforcement section 4264 to the deformation panel 294.

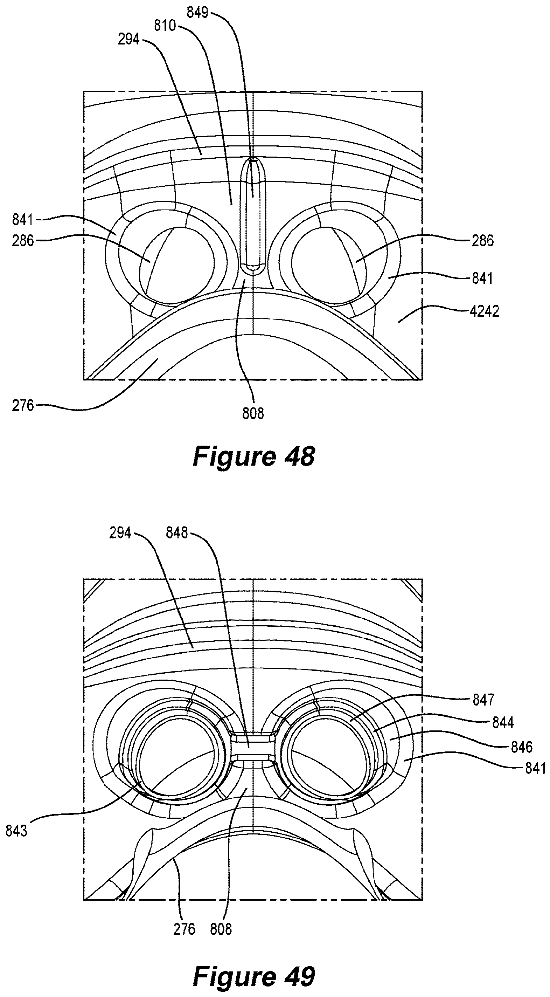

The embodiment of the flow director 840, however, has an alternative reinforcement section 849. While figure 48 shows that the reinforcement section 849 has the same elongate form as the reinforcement section 4264, the reinforcement section 849 extends from an underside of the spacing rib 848 to the deformation panel 294. This differs from the reinforcement section 4264 which extends from the spacing rib 4262 to a position spaced from the deformation panel 294. Extending the reinforcement section 849 to the deformation panel 294 may assist with controlling deformation of the deformation panel 294. More specifically, the reinforcement section 849 aids the rolling action of the second wall 272 below the first resilient section 268.

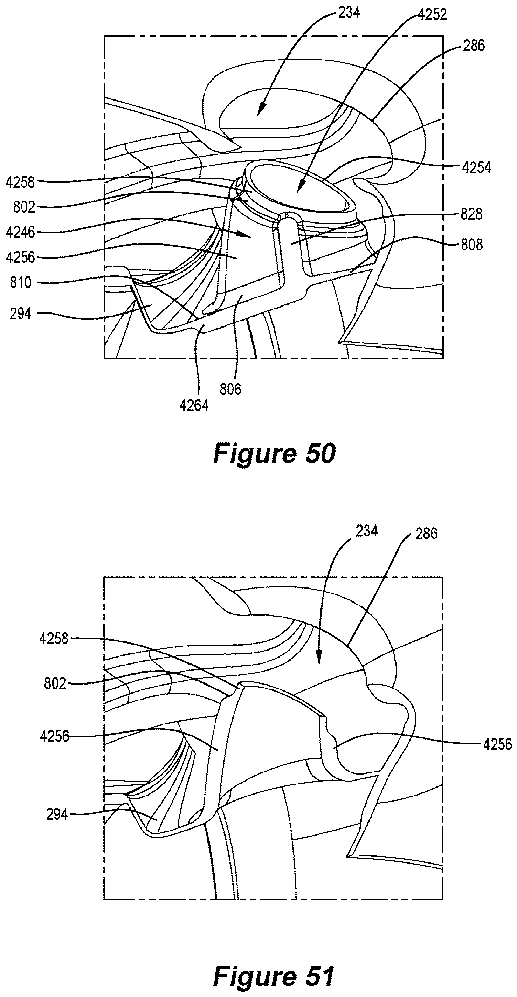

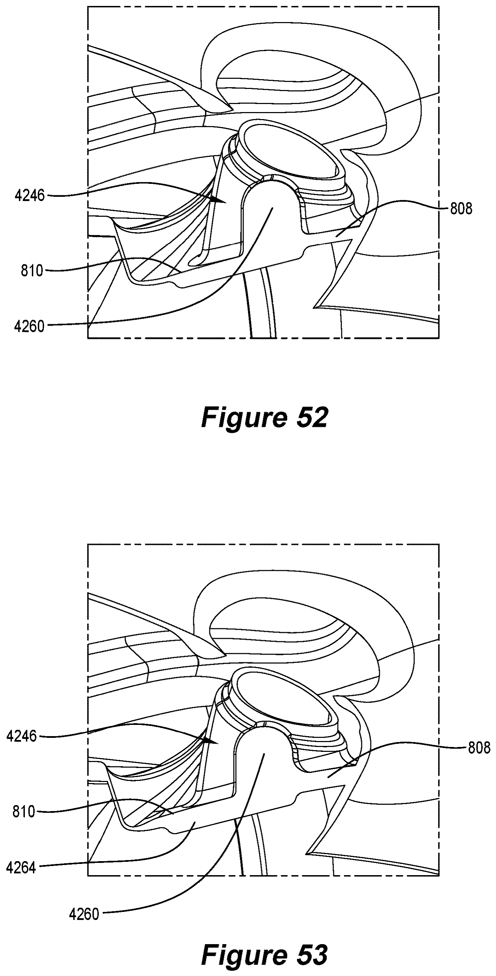

The embodiments of flow directors 820, 830 and 840 are associated with proximal and distal portions 808, 810 of the dividing wall 4242 that have a wall thickness that is the same as the wall thickness of the lateral side portions 800. The wall thickness of the proximal and distal portions 808, 810 may be different in different embodiments. For example, Figures 52 to 54 show the flow director 4246 with its associated spacing rib 4260 as described above in respect of the cushion module 4020. These figures also show different embodiments with different wall thicknesses for the proximal and distal portions 808, 810. In comparison with the flow directors 820, 830 and 840, Figures 50 and 51 show the flow director 4246 with the spacing rib 828 and with the proximal and distal portions 808, 810 having the same wall thickness as the lateral side portions 800. The proximal portion 808 is thickened in the embodiment shown in Figure 52. This thickening of the proximal portion 808 increases the resistance to deformation of the proximal portion 808 and thereby improves the stability of the flow director 4246. The increased stability occurs due to deformation forces being directed through the proximal portion 808 and into the reinforcement section 4264. This encourages controlled deformation of the deformation panel 294 instead of buckling of the proximal portion 808 which may cause the flow director 4246 to buckle or collapse.

A variation of the embodiment shown in Figure 52 is shown in Figure 53 with the same flow director 4246 and the same spacing rib 4260. This embodiment differs in that the proximal portion 808 is further thickened in comparison to the proximal portion 808 shown in figure 52. It also differs in that the distal portion 810 is thickened in comparison to the distal portion 810 shown in Figure 52. Thickening of the proximal and distal portions 808, 810 has the same effect of increasing the stability of the flow directors 4246 as described above for the embodiment shown in Figure 52.

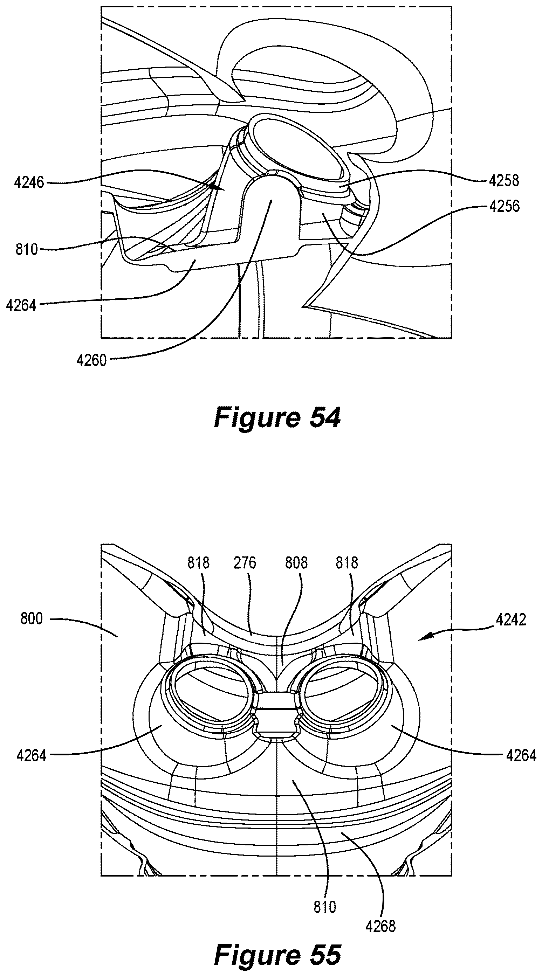

A further variation of the embodiment shown in Figure 52 is shown in Figures 54 and 55 with the same flow director 4246 and the same spacing rib 4260. In this embodiment, the distal portion 810 is thickened in the same way as it is in the embodiment shown in Figure 53. This embodiment differs in that the proximal portion 808 has a wall thickness in an area proximal of the spacing rib 4260 that is the same as the wall thickness of the lateral side portions 800. The proximal portion 808 includes brace members 818 disposed between each flow director 4246 and the outer wall 288. The brace members 818 maintain a spacing between the outer wall 288 and each of the respective flow directors 4246.

Each brace member 818 joins with the outer wall 288 at an outer wall portion between the nare opening 234 and the oral opening 232. In this embodiment, each brace member 818 joins with the base 806 and the lower body portion 4256 only (Figure 54). In other embodiments, the brace member 818 may join with the base 806, the lower body portion 4256 and the step 802. In a further embodiment, the brace member 818 may join with the base 806 only. In each of these embodiments, the brace member may have a lateral width which is 20 to 70% of the lateral width of the flow director 4246.

In this embodiment, the brace member 818 has a trapezoidal profile. However, the base member is not limited to this profile. For example, the brace member 818 may have a polygonal profile, a semi-circular profile, a circle-segment profile or a curved profile.

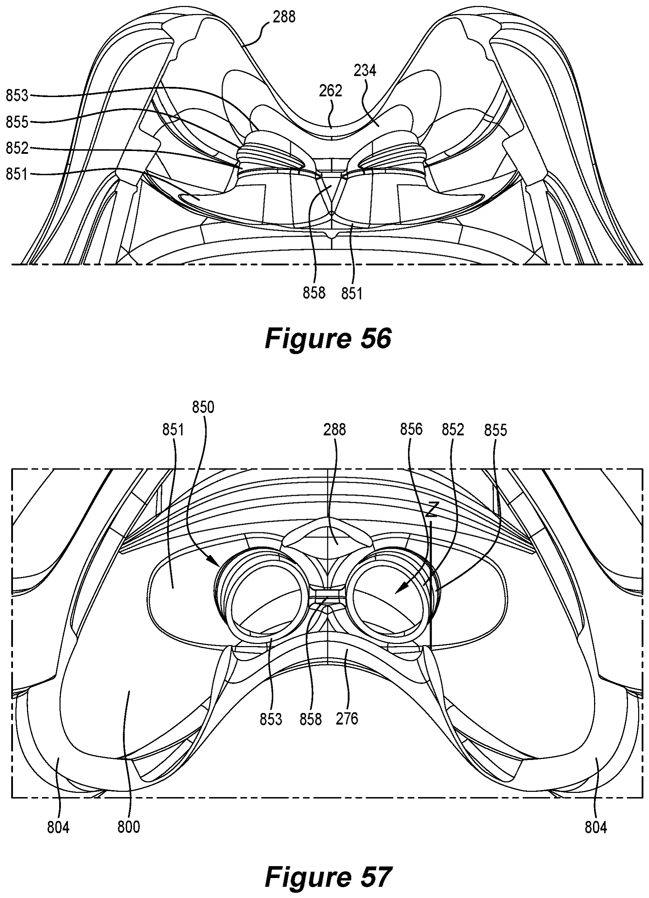

An alternative embodiment of flow directors 850 for the cushion module 4020 is shown in Figures 56 and 57. The flow director 850 has part of the same general form as the flow director 820 in that it has a base 851, a body 852 and a rim 853. The base 851 includes a laterally outwardly-directed thickened pad 854 that decreases in thickness from the body 852 to the laterally outermost part of the base 851. The base 851 wraps around the body 852 at the level where the thickened pad 854 joins the body 852. The body 852 extends upwardly from the base 851 and terminates at the rim 853.

In other words, the base 851 of each flow director 850 is elongate in a lateral direction away from a vertical mid-plane through the patient interface 10, having regard to an upright orientation of the patient interface 10. Furthermore, the body 852 is offset from a lateral centreline (denoted as Z in Figure 57) through the base 851 so that the body 852 is closer to the laterally inward side of the base 851 than the laterally outward side of the base 851. The laterally-outer side of the base 851 extends laterally outwardly further from the body 852 than a laterally inward side of the base 851 extends laterally inwardly from the body 852. This is evident in Figure 57 which shows that the gas flow channel 812 is off-set laterally inwardly from a lateral centreline through the base 851, which centreline is parallel to the vertical mid-plane through the patient interface 10. Accordingly, the gas flow channel 812 is closer to the laterally inward side of the base 851 than the laterally outward side of the base 851. In this embodiment, the base 851 has a footprint which extends laterally beyond a corresponding lateral edge of the nare opening 234. However, the laterally-outer side of the base 851 may extend different distances in different embodiments. In that regard, the laterally-outer side of the base 851 extends laterally outwardly from the body 851 at least two times, at least three times, at least four times or at least five times further than the laterally-inward side of the base 851 extends laterally inwardly from the body 851.

The effect of the thickened pad 854 is to stabilise the body 852. More specifically, the thickened pad 854 increases the resistance of the flow director 850 to buckling or collapse because localised buckling of the dividing wall 4242 is resisted. This improves the lateral stability of the flow directors 850 and contributes to resisting inward deflection of the flow directors 850 toward each other. It follows that the thickened pads assist to maintain the orientation of the flow directors 850 in directing respiratory gas through the nare opening 234 and into the nares of a patient in use.

A further factor which contributes to resisting bucking or collapse of the flow directors is an increase in the wall thickness of the body 852. For reasons of patient comfort, the wall thickness of the body 852 cannot be too thick because the bodies 852 will cause patient discomfort when they come into contact with a patient. Alternatively, the body 852 may include a transition and upper and lower body portions in the form of any one of the bodies 248, 1248, 2248, 3248, 822, 832 or 842. In those alternatives, the transition may take the form of the step 802. The step, in those alternative embodiments, may extend wholly or partly around the body.

Another feature which may be adopted into any of the embodiments disclosed in this specification is a reinforced segment 855 (Figure 56) on the body 852 or on the body of any of the other embodiments. The reinforced segment 855 comprises a rib in this embodiment. The reinforced segment 855 in the form of the rib or otherwise may be used in combination with other features disclosed in this specification to stabilise the flow directors and to transfer deformation forces to the deformation region. Accordingly, it will be understood that the reinforced segment 855 is not exclusive to this embodiment and may be used in combination with other flow directors, such as the flow directors 246, 1246, 2246, 3246, 820, 830, 840 and the flow directors 860 and 870, 880 or 890 (described below).

The reinforced segment 855 shown in Figures 56 and 57 is configured to resist deformation of the shape of the second opening 856 under a deformation force. The reinforced segment 855 may extend wholly or partly around the body 852. In this embodiment, the reinforced segment 855 extends partly around the body 852 on a distal side of the body 852. In other embodiments, however, the reinforced segment 855 may extend around the distal side of the body 852, the proximal side of the body 852 or the lateral side of the body 852 or a combinations of those sides (e.g. the proximal and lateral sides or the lateral and distal sides).

The reinforced segment 852, in this embodiment, is integrally formed with the body 852. It has a wall thickness that is greater than the wall thickness of the other parts of the body 852. In another embodiment, the body 852 is formed of the first material and the reinforced segment 855 is formed of a second material which is different from the first material. Furthermore, the second material is less compliant than the first material such that the reinforced segment 852 reinforces the body 852 by means of structure and material properties.

The reinforced segment 855 is disposed at a set spacing from the rim 853. In alternative embodiments, the reinforced segment 855 may be disposed at a set spacing from the base 851. Alternatively, the reinforced segment 855 may be disposed around the body 852 at a varying spacing from the rim 853. The reinforcing segment 855 may be combined with the body of other flow directors described or claimed in this specification. This includes flow directors having a body that includes a lower body portion and an upper body portion. In those embodiments, the reinforced segment may extend wholly or partly around the lower body portion. Alternatively, the reinforced segment may extend wholly or partly around the upper body portion. In a further alternative the reinforced segment may extend from the lower body portion and at least partly around the upper body portion.

A further embodiment of flow directors 860 which may replace the flow directors 4246 in the cushion module 4020 is shown in Figures 58 to 60. In this embodiment, the flow directors 860 have a base 861 and a body 862 which extends upwardly from the base 861 and terminates in a rim 863. The base 861 of each flow director is a region of increased thickness compared to the wall thickness of the dividing wall 4242. In this embodiment, the bases 861 take the form of an island that extends from the dividing wall 4242 into the second chamber 240. Said another way the base 861 in this embodiment take the form of a localised region of increased wall thickness compared to the wall thickness of the dividing wall 4242. This embodiment differs in that the bases 861 of the flow directors 860 are linked together to form a common block 865 from which the separate bodies 862 extend. The common block 865 has a footprint which extends laterally beyond corresponding lateral edges of the nare opening 234. Aside from gas flow channels 812 extending through the common block 865, the common block 865 is a solid structure. The common block 865 behaves as a monolithic structure so that deformation forces imparted upon the wall portion 276 will be transferred through the common block 865 and into the deformation panel 294. With the deformation forces transferred into the deflection panel 294, the gas channels 812 within the common block 865 are protected from buckling or collapse when a deformation force is applied to the wall portion 276. An advantage of the common block 865 is that the deformation force is spread across a laterally broader region of the deflection panel. This provides more even distribution of the deformation force across the width of the deformation panel 294 and therefore provides a more controlled deformation of the deformation panel 294. A further advantage is that a deformation force applied to one body 862 will be transferred, at least in part, through the common block 865 to the other body 862 (or other bodies 862 depending on the number of bodies 862 incorporated into the cushion module 4020). The force transfer increases the overall resistance of the flow directors 860 to buckling.

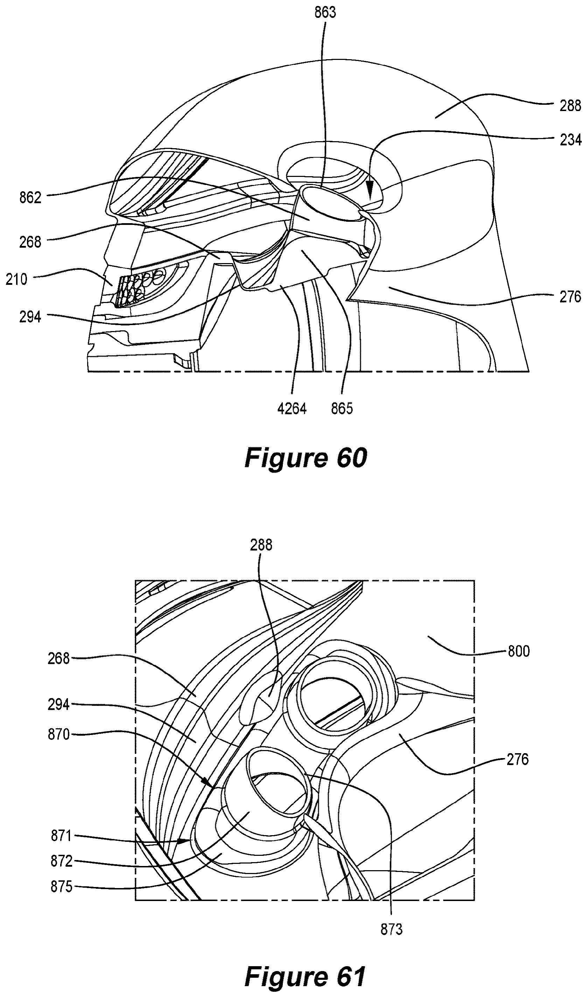

As shown in figure 60, the common block 865 is inclined upwardly from the dividing wall 4242 on the proximal and distal sides of the common block 865 and also on the lateral sides of the common block 865. The inclined sides of the common block 865 may take the shape of a fillet or in other embodiments may be chamfered. The top surface of the common block 865 is inclined relative to the dividing wall 4242. The relative inclination is downward in the proximal direction. That is, the proximal side of the top surface spaced by a distance from the dividing wall 4242 that is less than the spacing of the top surface from the dividing wall 4242 at a distal side of the common block 865.

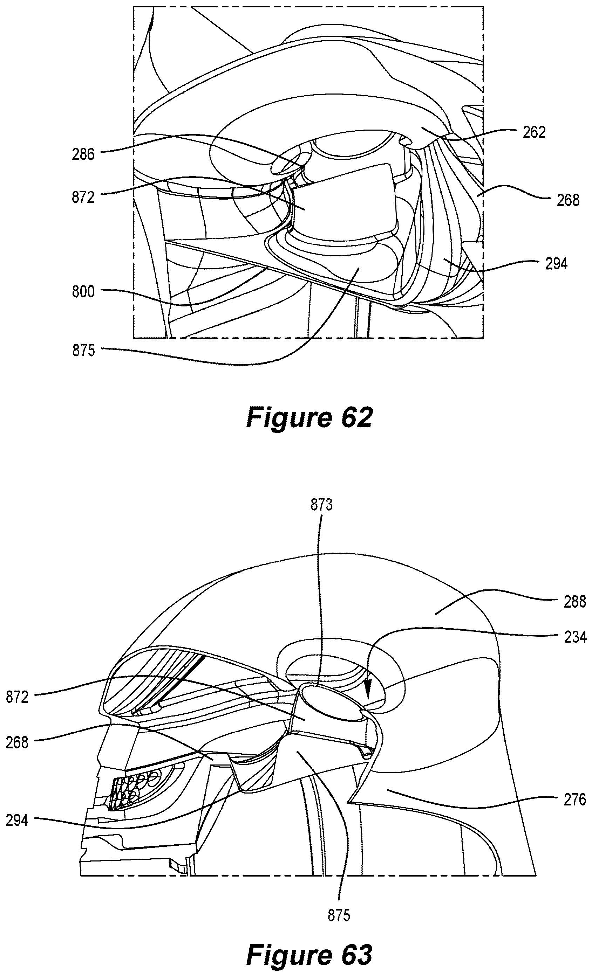

A variation of the flow directors 860 is shown in figures 61 to 63 as a further embodiment of the flow directors 870 which may be substituted for the flow directors 4246 in the cushion module 4020. The flow directors 870 have a base 871, bodies 872 and rims 873 in the same form as the flow directors 860. That is, the base 871 is in the form of a common block 875. However, the flow directors 870 differ from the flow directors 860 in that the perimeter walls of the common block 875 are more steeply inclined. As a result, the distal side of the common block 875 forms the proximal edge of the deformation panel 294. This means that deformation forces transferred through the common block 875 are transferred directly into the deformation panel 294 instead of being transferred through the distal portion 810 or a tapered distal wall of the common block 865.

The bodies 862 and 872 have a constant wall thickness from their respective bases 861, 871 to their rims 863, 873. In alternative embodiments, the bodies 862, 872 may include a transition which delineates upper and lower body portions. The transition may take the form of a step, such as the step 844 in the flow director 840. The step may form the transition between a lower body portion which has a wall thickness that is more than the wall thickness of an upper body portion. For example, the bodies 862, 872 may be replaced with any one of the bodies, 822, 832, 842, including different wall thicknesses for the upper body portion and the lower body portion. The body may also be replaced by the body 852, including the reinforcing segment.

In a further variation, the bases 861 and 871 may be combined with any of the bodies 248, 1248, 2248, 3248.

Other features may be combined with the bodies 862 and 872. Such features include, for example, the reinforced segment 855 from the flow director 850 described above.

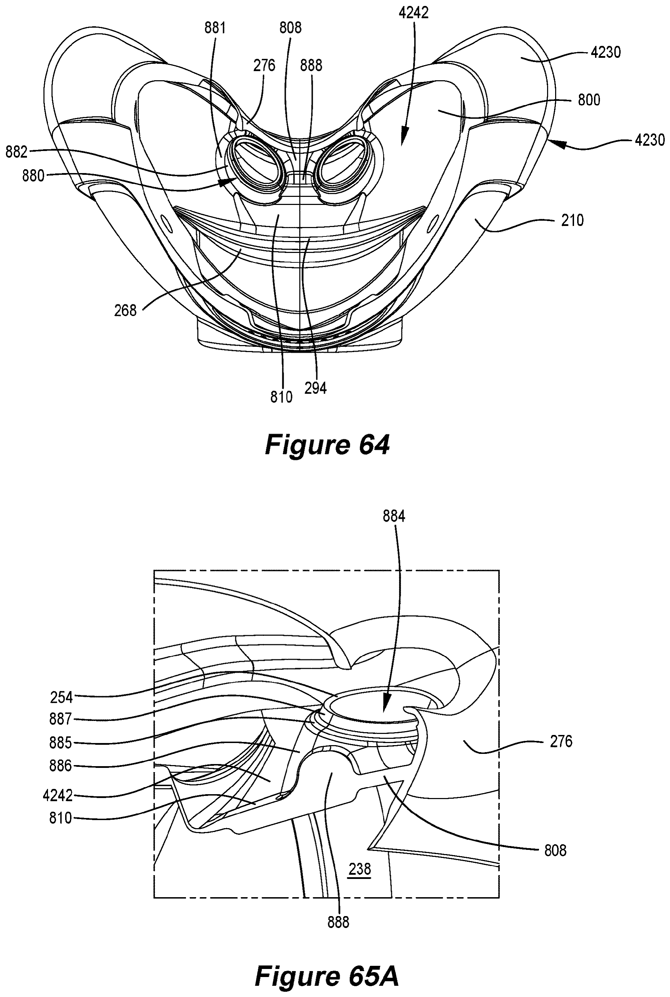

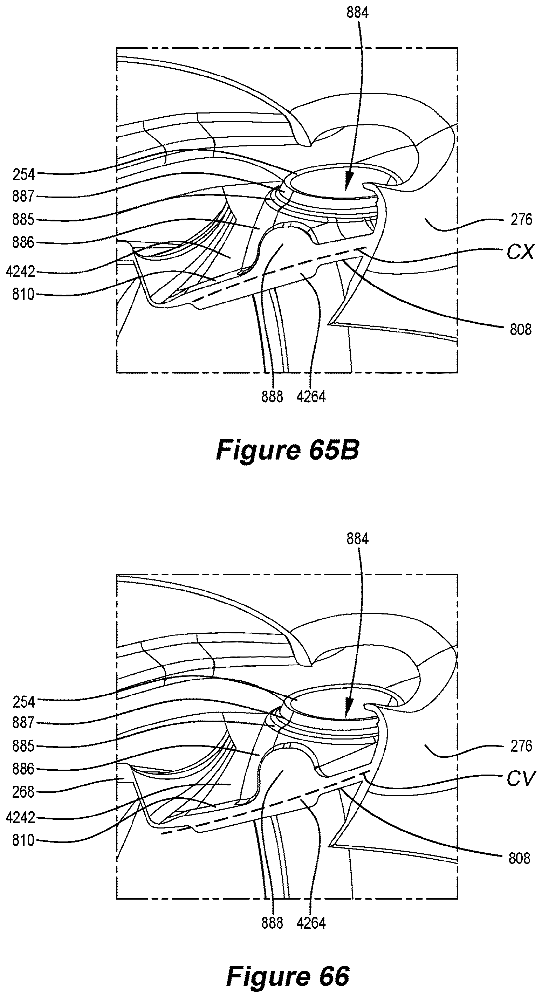

One alternative flow director 880 that can form part of the cushion module 4020 is shown in Figure 64 and Figures 65A and 65B. The flow director 880 has the same general form as the flow director 4246 in that it includes a base 881, a body 882 extending from the base 881 and a rim 883. The base 881 joins the body 882 to the dividing wall 4242. The base 881 forms a smooth transition surface between the body 882 and in the dividing wall 4242. Like the flow director 4246, part of the outer wall 288 extends over the laterally outer sides of a second opening 884 defined by the rim 883. As explained above in respect of the cushion module 4020, the nare opening 234 expands laterally when the patient interface 10 is fitted so that the nare opening 234 extends laterally outwardly so that the outer wall 288 doesn't extend over the second openings 884 in use.

The body 882 includes a transition 885 which delineates a lower body portion 886 from an upper body portion 887. The lower body portion 886 has a wall thickness which decreases from the base 881 to the transition 885. The upper body portion 887 has the same wall thickness from the transition 885 to the rim 883. The lower body portion 886 is angled in the proximal direction so the proximal side of the lower body portion 886 extending from the base 881 to the transition 885 is shorter than the distal side of the lower wall portion 886 extending from the base 881 to the transition 885. The upper body portion 887 is angled proximally and laterally inwardly. The transition 885 occupies a plane that is inclined in the same manner as the lower body portion 886. However, the inclination of the lower body portion 886 and the transition 885 is less that the inclination present in the flow director 4246. The angling of the lower and upper body portions 886, 887 and the lateral positioning of the flow directors 880 facilitates direction of respiratory gas through the one of more nare openings 234 and into the nares from the flow directors 880. The flow director 880 also differs from the flow director 4246 in that the transition 885 is closer to the dividing wall than the transition 802 for the flow director 4246.

The flow directors 880 further differ from the flow directors 4246 in that the base of the or each respective flow director 880 joins with the outer wall 288 at the wall portion 276. In this arrangement, the proximal portion 808 is located between the flow directors 880 and between the spacing rib 888 and the wall portion 276. In other embodiments, the flow directors 888 are spaced from the wall portion 276 so that the proximal portion 808 is positioned between the lateral side portions 800.

The base 821 of each of the flow directors is spaced from a vertical mid-plane through the cushion module 4020 (as seen in Figure 64). A spacing rib 888 spans the gap between the flow directors 880. The spacing rib 888 extends from the bases 881 to a position below the transition 885 on each flow director 880. The top of the spacing rib 888 forms a straight line between the flow directors 880. The spacing rib 828 overlaps with the reinforcement section 4264 on the underside of the dividing wall 4242. The spacing rib 888 is aligned, in the proximal-distal direction, with a distal portion of the rim 883 which defines the second opening.

The proximal portion 808 of the dividing wall 4242 between the spacing rib 888 and the wall portion 276 of the seal member 4230 and between the lateral side portions 800 of the dividing wall 4242 has a wall thickness that is greater than the wall thickness of lateral side portions 800 of the dividing wall 4242. In this embodiment, the wall thickness of the proximal portion 808 is at least 1.5 times the wall thickness of the lateral side portions 800. The wall thickness of the proximal portion 808 may be up to 3 times the wall thickness of the lateral side portions 800. In other embodiments, the wall thickness of the proximal portion 808 may be up to 8 times the wall thickness of the lateral side portions 800.

The wall thickness of the proximal portion 800 may be different at different locations. In other words, the wall thickness of the proximal portion 808 may vary across the proximal portion 808 in the proximal-distal direction or in the lateral direction or in both directions.

In the embodiment shown in Figure 65B, the wall thickness of the proximal portion 808 is greater than the wall thickness of a distal portion 810 of the dividing wall 4242 between the spacing rib 888 and the deformation panel 294 and between the lateral side portions 800 of the dividing wall 4242. Figure 65A shows part of the distal portion 810 in profile which overlaps the reinforcement section 4264. Accordingly, in Figures 65A, the wall thickness of the distal portion is represented by the wall thickness excluding the wall thickness of the reinforcement section 4264.

The dividing wall 4242, in this and other embodiments, has a curved profile in the proximal-distal direction. The curved profile extends across substantially the entire lateral dimension of the main portion 244 of the dividing wall 4242. Optionally, the curved profile extends through the proximal and distal portions of the dividing wall.

The main portion 244 of the dividing wall 4242 is convex in the proximal-distal direction and relative to the nare opening 234. This convex curvature can be seen in the curved alignment of the proximal portion 808 relative to the distal portion 810 in Figures 35, 50, 52, 53, 54 and Figure 65B. The convex curvature is highlighted by a dashed line CX in Figure 65B.

In an alternative embodiment shown in Figure 66, the curved profile is concave in the proximal-distal direction and relative to the nare opening 234. The concave curvature is highlighted by a dashed line CV in Figure 66. In one embodiment, as shown in Figure 66, the proximal portion 808 and the distal portion 810 have profiles that are inclined relative to one another in the proximal-distal direction such that the angle between them on the side of the dividing wall within the second chamber is less than 180°.

In an alternative embodiment, the profile of the proximal portion 808 is curved. The profile of the proximal portion 808 is concave relative to the nare opening 234. It will be appreciated, however, for a convex curvature, the proximal portion 808 will be concave relative to the first chamber 238. Optionally, the profile of the distal portion 810 is curved. For a concave curvature of the dividing wall 4242, the profile of the distal portion 810 may be concave relative to the nare opening 234. However, for a convex curvature, the distal portion 810 will be concave relative to the first chamber 238.

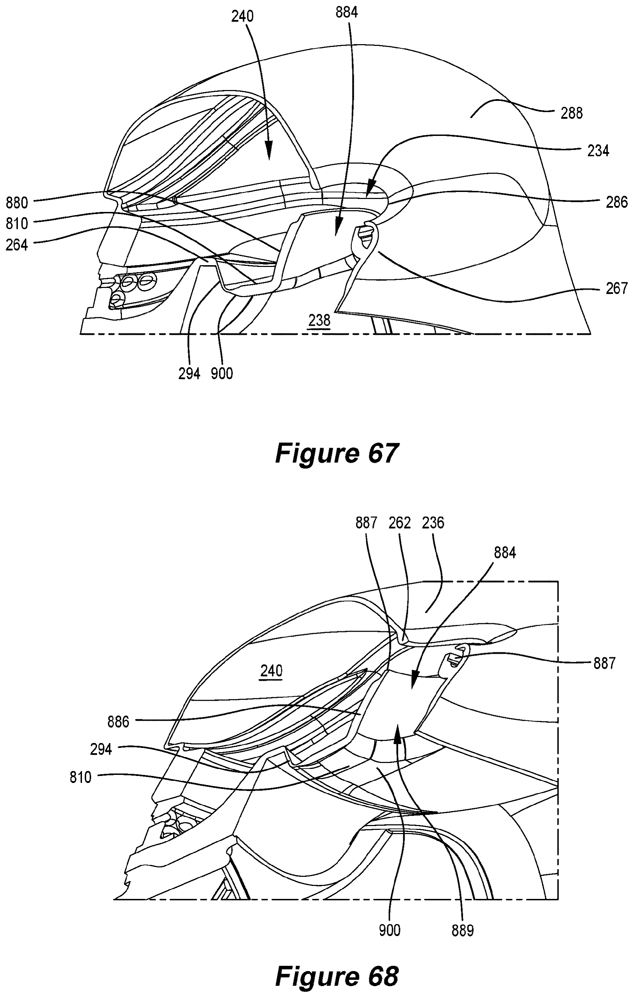

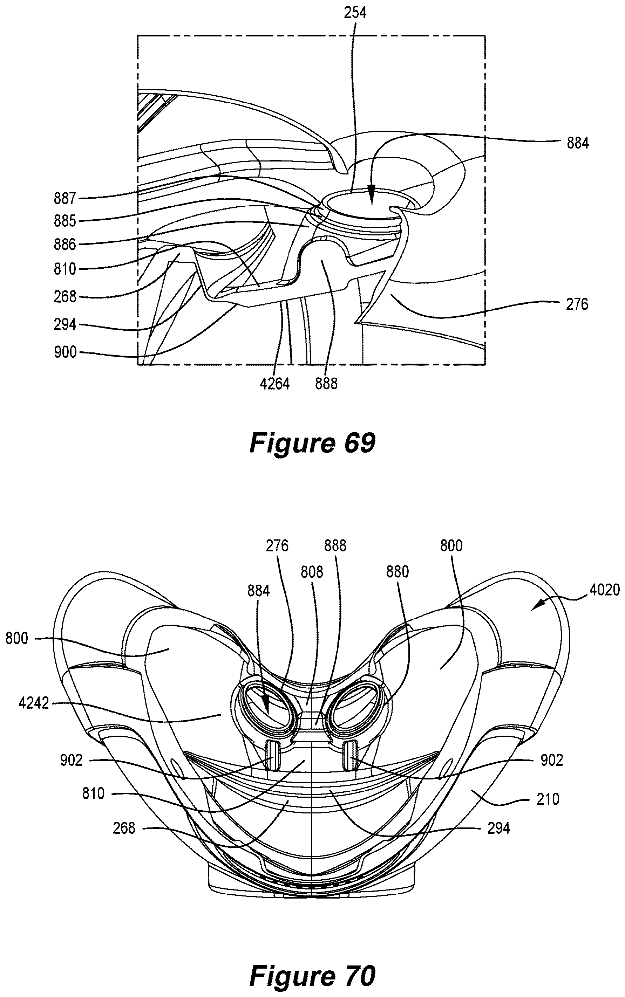

Figures 67 to 69 show a variation of the embodiment shown in Figures 66. The variation involves the distal portion 810 having a wall thickness that is greater than the wall thickness of lateral side portions 800 of the dividing wall 4242. The wall thickness of the distal portion is at least 1.5 times the wall thickness of the lateral side portions. The wall thickness of the distal portion 810 may be up to 3 times the wall thickness of the lateral side portions 800. In other embodiments, the wall thickness of the distal portion 810 may be up to 8 times the wall thickness of the lateral side portions 800.

The wall thickness of the distal portion 810 may vary in the proximal-distal direction. Additionally, the wall thickness of the distal portion 810 may vary in the lateral direction. In this embodiment, the distal portion 810 includes one or more taper regions 900 where the wall thickness of the distal portion 810 decreases in the direction of an adjoining lateral side portion 800 or in the direction of the adjoining deformation panel 294 to a thickness that is the same as the respective adjoining lateral side portion 800 or the adjoining deformation panel 294.

The one or more of the taper regions 900 may be disposed on the side of the dividing wall facing the first chamber 238. This is shown in Figure 68, as taper regions 900 on the distal and lateral sides of the distal portion 810 adjacent to where the distal portion 810 joins the deformation panel 294 and the lateral side portion 800. In this embodiment, the surface of the dividing wall 4242 across where the distal portion 810 meets the lateral side portion 800 includes a continuous curve associated with the general curvature of the dividing wall 4242. In other words, the side of the distal portion 810 exposed to the second chamber 240 doesn't include an adjustment to account for the tapering region 900.

In other embodiments, however, one or more of the tapering regions 900 are disposed on the side of the dividing wall 4242 facing the second chamber 240. In another embodiment, tapering region may be disposed on both sides of the dividing wall 4242.

The spacing rib 888 includes a taper region 900 disposed between the proximal portion 808 and the distal portion 810.

Furthermore, the base 881 of the or each flow director 880 includes a tapering region 900 where the wall thickness of the distal portion 810 decreases to a thickness that matches the wall thickness of the lower body portion 886 of the one or more flow directors 880.

The wall thickness of the distal portion 810 may be the same as the wall thickness of the proximal portion 808. In another embodiment, however, the wall thickness of the distal portion 810 is greater than the wall thickness of the proximal portion 808.

In this and in other embodiments, the distal portion 810 has a lateral width that tapers inwardly in the distal direction. However, the laterally-inward tapering of the distal portion 810 may not be present in alternative embodiments.

In a variation of the embodiment shown in Figure 64, the patient interface 10 includes a tether 902 configured to resist changes in the orientation between the lower body portion 886 of a respective flow director 880 and the dividing wall 4242. The tether 902 links the lower body portion 886 to the distal portion 810 at positions on the lower body portion 886 and on the distal portion 810 spaced from the base 821 of the respective flow director 880. In this particular embodiment, the tether 902 joins with the flow director 800 along the lower body portion 886 and the base 881 and joins with the distal portion 810 from the base 810 to the position spaced from the base 881.

The tether 902 may a variety of different forms. In this embodiment, the tether 902 is a rib extending between the lower body portion 886 of a respective flow director 800 and the distal portion 810.

The tether 902 is positioned on a distal side of the flow director 800. The tether 902 is oriented in the proximal-distal direction.

The tether 902 may be oriented in other directions in alternative embodiments. In further alternative embodiments, the tether 902 may be an extension of the lower body portion 886 in the distal direction and which extension has a wall thickness in the proximal-distal direction that is greater than the wall thickness of other parts of the lower body portion 886. The extension may taper inwardly in the distal direction in a cross-section parallel to the plane of the distal portion.

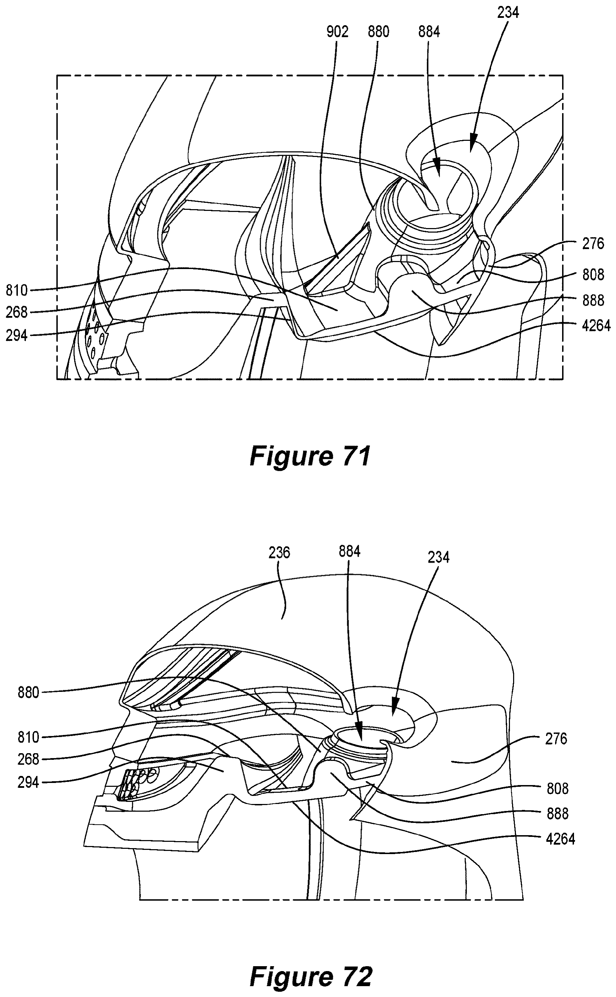

Figures 72 to 74 show a further variation of the embodiment shown in Figure 66. In this embodiment, the patient interface 10 includes one or more ties 904 that are configured to resist movement of the distal portion 810 towards the nare sealing portion 236 of the outer wall 288.

Having regard to Figure 72, the first resilient region 268 includes a support wall 906 and an overhang lip 908 projecting proximally from the support wall 906. The first wall 270 of the deformation panel 296 extends away from the nasal sealing portion and extends proximally from the overhang lip 908 to define a gap 910 between the deformation panel and the support wall 906. When a deformation force is applied to the wall portion 276, the main portion 244 of the dividing wall 4242 travels distally as the deformation panel deforms. In doing so, the distal edge of the main portion 244 travels into the gap 910. Permitting such travel enables the patient interface to absorb a deformation force and substantially avoid buckling of the flow directors 870 and the main portion 244. The tie 904, as shown in Figures 72 to 74 contributes to maintaining the shape of the main portion when subject to a deformation force, for example deformation forces applied to the wall portion 276 or resulting from a gas pressure differentials between the first chamber 238 and the second chamber 240 or from a gas pressure differential between the ambient gas pressure external to the patient interface 10 and the gas pressure within the patient interface 10.

The tie 904 is configured to collapse when subject to a compressive force in the proximal-distal direction, for example, due to a deformation force applied to the wall portion 276. Such collapse permits the travel of travel of the main portion 244 into the gap 910. However, the tie 904 is also configured to inhibit movement of the main portion 244 toward the nare sealing portion 236. In other words, any tendency of the main portion 244 to buckle by moving toward the nare sealing portion 236 is resisted by the tie 904 tethering the main portion to the support wall 906. Such tethering places the tie 904 in tension which resists displacement of the main portion 244 toward the nare sealing portion 236.

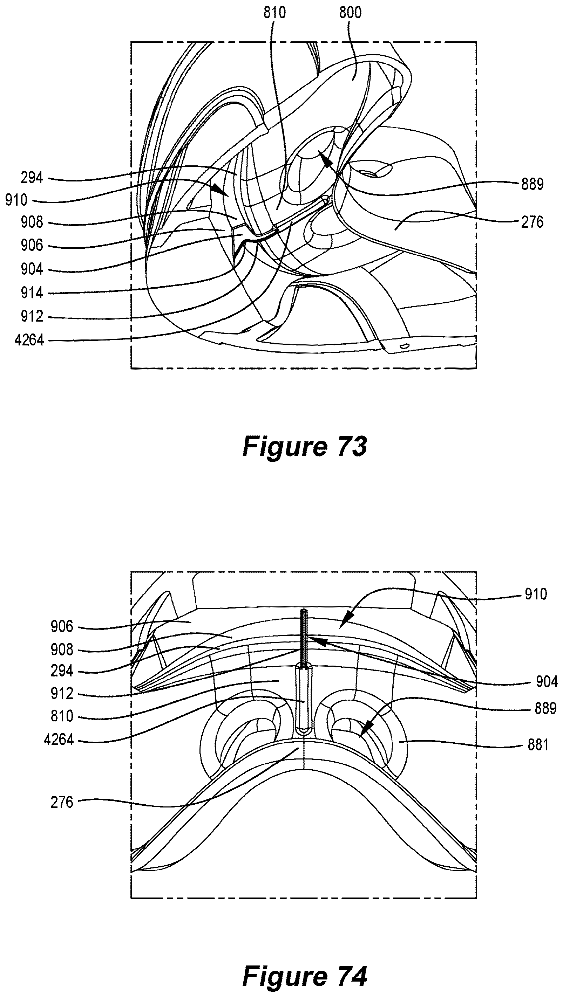

In this embodiment, the tie 904 is configured to limit travel of the dividing wall away from the first resilient region 268. The tie 904 is a panel which connects with the deformation panel 294, the main portion 244 and the first resilient region 268. In this embodiment, the tie 904 connects the distal portion 810 and the first resilient region 268. In terms of connecting with the first resilient region 268, the tie 904 connects with the deformation panel 294, the overhang lip 908 and the support wall 906. As shown particularly in Figures 73 and 74, tie 904 connects the first resilient region 268 and the reinforcement section 4264 associated with the distal portion 810. In other embodiments, however, the tie 904 may connect with the deformation panel 294, the main portion 244 and the support wall 906. That is, the tie 904 may not connect with the overhang lip 908. In other embodiments, the tie may connect with the dividing wall 4242 and the first support wall 906 only.

The tie 904 is configured to collapse under compressive forces in the proximal-distal direction and is configured to resist extension under tensile forces in the proximal-distal direction and in the direction toward the nare sealing portion 236. In this embodiment, the tie 904 is a panel.

The tie 904 has a wall thickness that is the same as the wall thickness of the first wall 270 of the deformation panel 294. Optionally, the tie 904 has a wall thickness that is less than the wall thickness of the first wall 270 of the deformation panel 294.

The tie 904 is perpendicular to the first wall 270 of the deformation panel 294, as shown in Figure 74. In this embodiment, the tie 904 is disposed in the vertical mid-plane through the patient interface 10, having regard to an upright orientation of the patient interface 10. However, other embodiments may include a tie 904 that is oriented obliquely to the proximal-distal direction.

The tie 904 includes a free edge 912 exposed to the first chamber 238. The free edge 912 is a straight line extending between the first resilient region 268 and the deformation panel 294 or the distal portion 810 or the reinforcement section 4264 associated with the distal portion 810.

The tie 904 includes a point of weakness to induce collapse of the tie 904 at that point as the distal portion 810 travels toward the first resilient region 268. Optionally, the point of weakness is a localised reduction in the wall thickness of the tie 904. However, in this embodiment, the point of weakness in the free edge 912 is a notch 914 disposed between the deformation panel 294 and the first resilient region 268. The notch 914 has a V-shape. Other shapes may be selected for the notch 914 provided that they make the notch 914 function as a point of weakness.

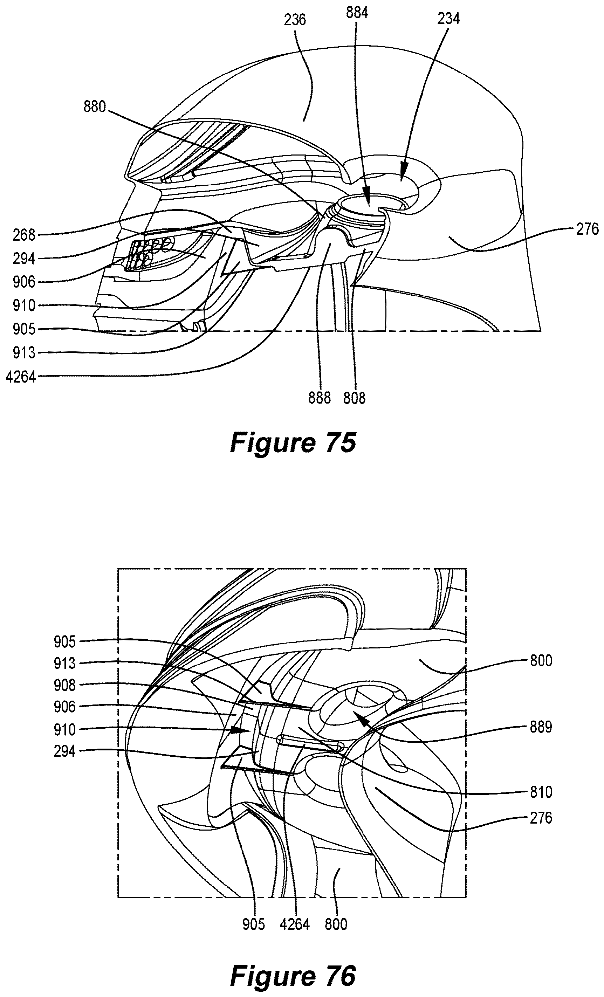

In the embodiment shown in Figures 72 to 74, the patient interface includes one tie 904. However, it will be appreciated that other embodiments may include more than one tie. One embodiment that include more than one tie 904 is shown in Figures 75 to 77.

In that embodiment, the patient interface 10 includes two ties and each tie is aligned in the proximal-distal direction with the distal portion. However, the ties 904 may be further laterally spaced apart. For example, the patient interface may include two ties 904 and each tie 904 may be aligned in the proximal-distal direction with a respective lateral side portion.

For the embodiment shown in Figures 75 to 77, the two ties 904 are parallel to each other in the proximal-distal direction. However, the two ties 904 may diverge from one another in the distal direction. Alternatively, the two ties 904 may converge toward one another in the distal direction.

The free edge 914 each tie 904 extends to a respective flow director 880. Alternatively, the free edge 914 of each tie 904 merges with a respective flow director 880. The free edge 914 of each ties 904 merges with the base 881 of the respective flow director 880. However, one tie 904 of the multiple ties 904 may extend to or merge with one of the respective flow directors 880.

The dividing wall 4242 includes a reinforcing structure 916 extending across the distal portion 810 and the lateral side portions 800 of the dividing wall 4242 and adjacent to the deformation panel 294. The reinforcing structure 816 is configured to resist deformation of the distal portion 810 and the lateral side portions 800. In this embodiment, the reinforcing structure 916 is a lateral reinforcing rib extending across the distal portion 810 and the lateral side portions 800 at a position that is on a distal side of the or each flow director 880. The additional wall thickness associated with the reinforcing structure 916 provides resilience to deformation of the dividing wall 4242 along the line of the reinforcing structure 916. This contributes to preferential deformation of the dividing wall 4242 in the deformation panel 294 and, therefore, contributes to retaining the shape of the dividing wall 4242 when it is subject to deformation forces applied through the wall portion 276.

The reinforcing structure 916 is disposed with a spacing between the reinforcing structure 916 and the deformation panel 294 and, optionally, the spacing is constant along the reinforcing structure 916. In this embodiment, the spacing varies along the reinforcing structure. The spacing between the reinforcing structure 916 and the deformation panel 294 is greater across the distal portion 810 than the spacing between the reinforcing structure 916 and the deformation panel 294 across the lateral side portions 800. The spacing between the reinforcing structure and the deformation panel decreases in the lateral direction. The decrease in the spacing may be continuous, stepped or discontinuous. Furthermore, the spacing may vary differently in other embodiments. In this embodiment, the spacing between the reinforcing structure 916 and the deformation panel 294 is greatest in a central location.

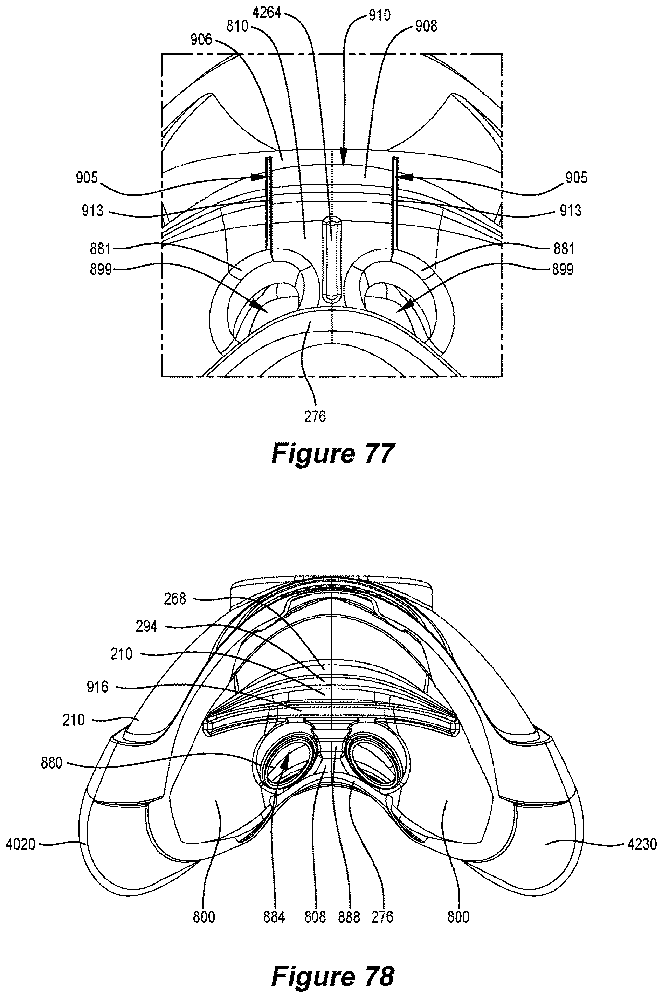

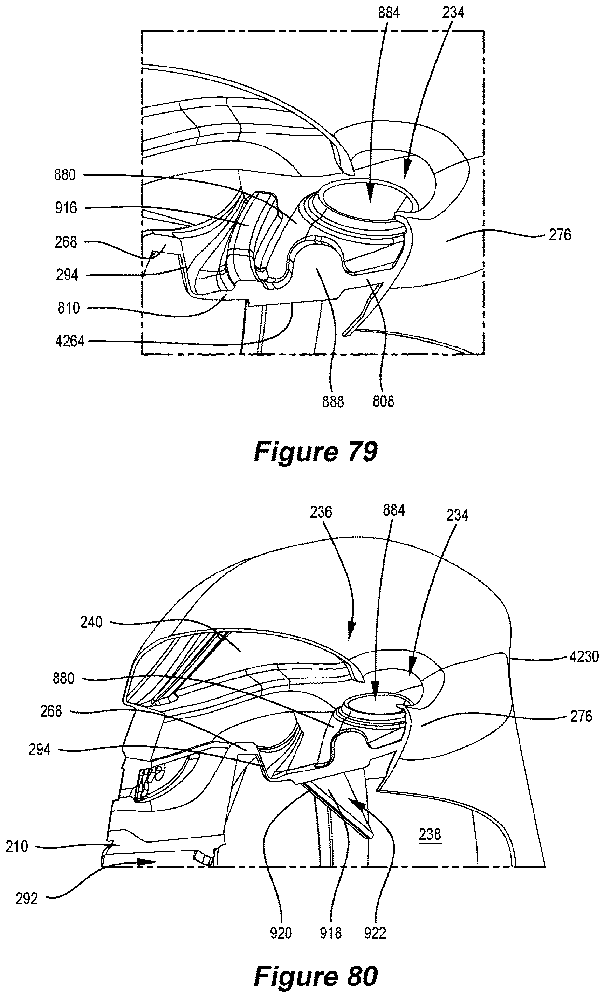

In the embodiment shown in Figures 78 and 79, the reinforcing structure 916 is a lateral reinforcing rib and is on the side of the dividing wall 4242 exposed to the second chamber 240. In other embodiments, the reinforcing structure 916 may be a lateral reinforcing rib and may be on the side of the dividing wall 4242 exposed to the first chamber 238.

In other embodiments, the reinforcing structure 916 includes a first lateral reinforcing rib on the side of the dividing wall 4242 exposed to the second chamber 240 and includes a second lateral reinforcing rib on the side of the dividing wall 4242 exposed to the first chamber 238. The first and second lateral reinforcing ribs may be aligned on each side of the dividing wall 4242 in the proximal-distal direction. Alternatively, the first and second reinforcing ribs are off-set on each side of the dividing wall in the proximal-distal direction. Alternatively, the first and second reinforcing ribs may be aligned in the proximal-distal direction on each side of the dividing wall 4242 at one or more locations and may be off-set on each side of the dividing wall 4242 in the proximal-distal direction at other locations.

Other embodiments may be variations on the configuration for the first and second reinforcing ribs described above. For example, the spacing from the deformation panel 294 for the first reinforcing rib is different to the spacing from the deformation panel 294 for the second reinforcing rib. In another example, the spacing from the deformation panel 294 for the first reinforcing rib is constant and the spacing from the deformation panel 294 for the second reinforcing rib varies. In another example, the spacing from the deformation panel 294 for the second reinforcing rib is constant and the spacing from the deformation panel 294 for the first reinforcing rib varies.

In the embodiment shown in Figures 78 and 79, the reinforcing structure 916 has a profile that is consistent along the length of the reinforcing structure 916. In other embodiments, the reinforcing structure 916 has a profile that varies along the length of the reinforcing structure 916. Alternatively, the reinforcing structure 916 may have a profile that tapers in height above the dividing wall 4242 in the lateral direction. The reinforcing structure may have a polygonal profile, a rectangular profile, a trapezoidal profile, a semi-circular profile or a circle-segment profile.

The reinforcing structure 916 is spaced from the or each respective flow director 880, in this embodiment. However, the reinforcing structure 916 may be linked to the or each respective flow director 880.

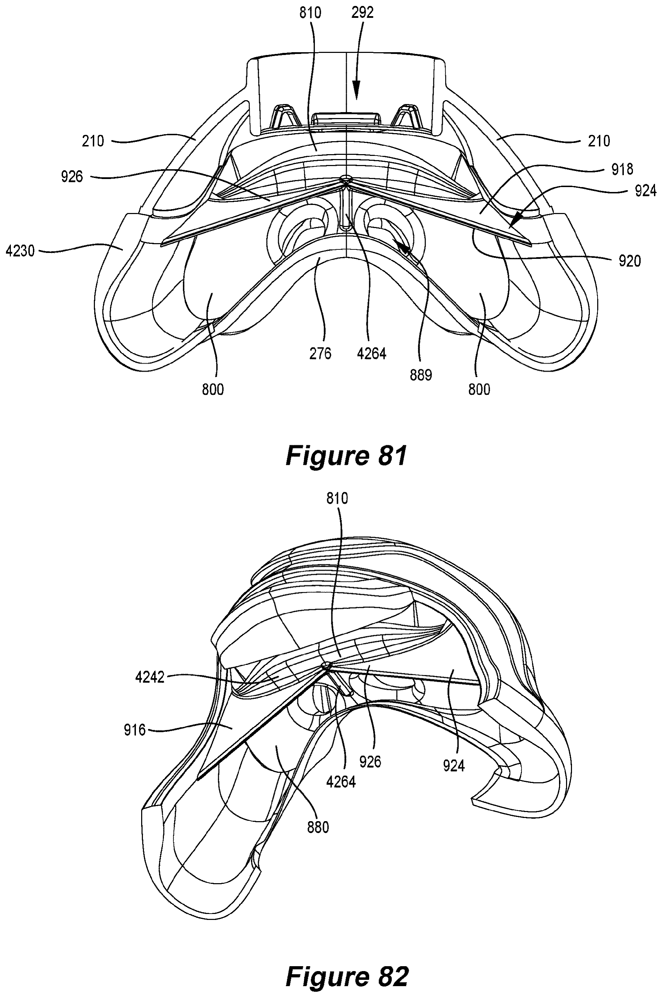

The patient interface 10 includes one or more deformation region ties 918 (Figures 80 to 82) that are configured to inhibit inversion of the dividing wall 4242. The or each respective deformation region tie 918 extends between the side of the dividing wall 4242 that is exposed to the first chamber 238 and the outer wall 288 of the seal member 4230. As shown particularly in Figures 81 and 82, seal member includes two deformation region ties 918. Each deformation region tie 918 extends from the outer wall to the distal portion 810. While the following description is in the context of the seal member having two deformation region ties 918, it will be appreciated that the seal member 4230 may have only one deformation region tie 918. So the following description of different configurations of deformation region ties 918 will be understood to apply equally to embodiments of the patient interface 10 which include only one deformation region tie 918.

The deformation region ties 918 connect with the dividing wall 4242 between the deformation panel 294 and the flow directors 880 or a respective one of the flow directors 880. In the embodiment shown in Figures 80 to 82, the deformation region ties 918 are spaced from the deformation panel 294. The deformation region ties 918 are spaced from the flow directors 880. Optionally, each respective deformation region tie 918 may merge with a respective one of flow directors. For example, each respective deformation region tie 918 may merge with the base 881 of a respective flow director 880.

As seen in Figures 81 and 82, each respective deformation region tie 918 extends from the dividing wall 4242 and the outer wall 288 and has a free edge 920 in the first chamber 238. The free edge 920 is a straight line. In this embodiment, the deformation region ties 918 have a flat, planar structure.

The patient interface 10 includes two deformation region ties and an angle between a proximal surface 922 of each respective deformation region tie 918 on the proximal side is greater than 140°. Alternatively, the angle between the proximal surface 922 of each respective deformation region tie 918 on the proximal side is greater than 160°.

In the embodiment shown in Figures 81 and 82, the two deformation region ties 918 extend to the same position on the distal portion 810. The two deformation region ties 918 extend to the reinforcement section 4264. However in alternative embodiments, the two deformation region ties 918 extend to toward each other to positions on the distal portion 810 that are spaced apart. In another embodiment, the deformation region ties 918 may extend to spaced apart positions on the distal portion, but the deformation region ties 918 may not extend toward each other.

The deformation region ties 918 include a first portion 924 that connects with the outer wall and a second portion 926 that extends to the main portion 244. In alternative embodiments, one connection point is with the main portion 244 and the other connection point is with the outer wall 288 at a level below the distal portion 810, having regard to an upright orientation of the patient interface. In such embodiments, the deformation region tie 918 may be a cord or wire or cable. In a further alternative embodiment, the first portion 924 has a flat planar structure and the second portion 926 is curved so that an angle, on the proximal side, between tangent lines at the respective positions to which the second portions 926 extend is between 140° to 180°.

The deformation region ties 918 are arranged to permit travel of the main portion 244 of the dividing wall 4242 when a deformation force is applied to the wall portion 276 and are arranged to inhibit deformation or inversion of the main portion toward the nare sealing portion 236. Accordingly, the location of the deformation region ties 918 within the first chamber 238 enables them to act as tensile resistors to deflection or inversion of the main portion 244 toward the nare sealing portion 236. The broad angle between the proximal surfaces 922 reduces the extent to which the deformation region ties 918 impart tensile or compressive influence on the travel of the main portion 244. The deformation region ties 918 may be configured to exert a substantially constant tensile force on the dividing wall 4242 through the range of travel of the dividing wall 4242 under the influence of a deformation force applied to the wall portion 276. This is assisted by orienting the flat, panel form of the deformation region ties 918 to be substantially orthogonal to the direction of travel of the main portion 244.

The tensile resistance imparted by the deformation region ties 918 is attributable to the connection with the outer wall being lower than the connection point with the main portion 244. In the embodiment shown in Figures 80 to 82, the deformation region ties 918 extends to a level below a lowermost level of the dividing wall 4242, having regard to an upright orientation of the patient interface. The deformation region ties 918 extend to a level below an uppermost level of the oral opening, having regard to an upright orientation of the patient interface. Furthermore, the deformation region ties 918 extends from the distal portion 810 to the outer wall 288 at a position that is located distally of the proximal most portion of the one or more nare openings 234.

The free edge 920 extends from the outer wall 288 at a position that is located proximally of the reinforcement section 4264. Alternatively, the free edge 920 extends from the outer wall 288 at a position that is located distally of the one or more oral openings 232. Alternatively, the free edge 920 extends from the outer wall 920 at a position that is located distally of the spacing rib 888.

In alternative embodiments, the free edge 920 may be curved. The curve may be a convex or concave curve.

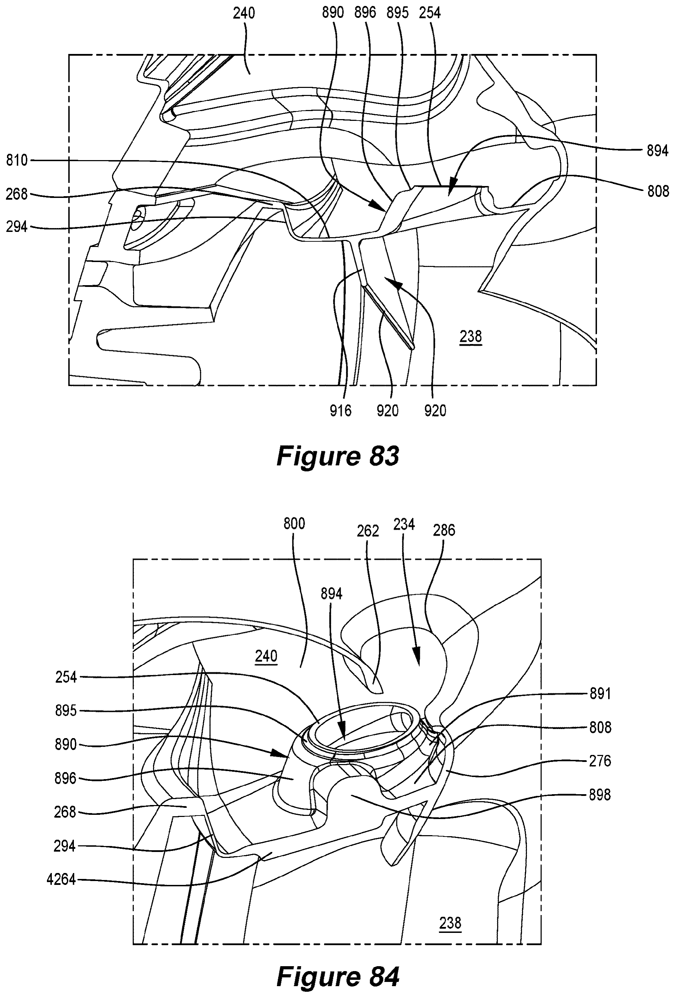

The embodiment shown in Figures 80 to 82 involves the deformation region ties 918 having the same wall thickness throughout. An alternative embodiment shown in Figure 83 involves deformation region ties 918 of the same flat, panel formed described above, but differs in terms of the wall thickness. Specifically, the deformation region tie 918 in Figure 83 is a panel having a wall thickness that decreases from the dividing wall to the free edge.

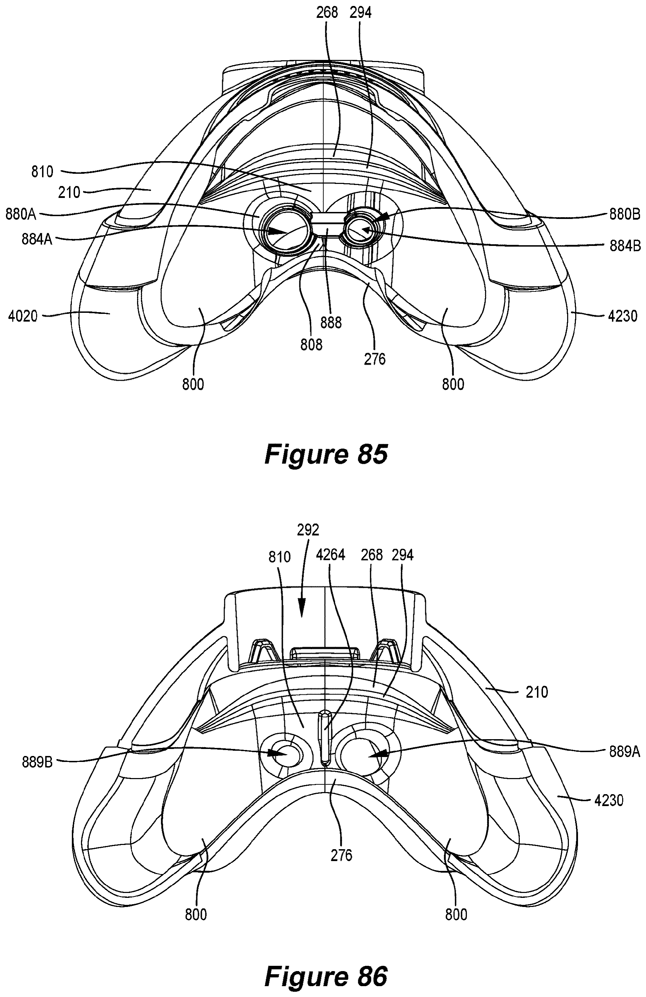

An alternative flow director 880 configuration for the cushion module 4020 is shown in Figures 85 to 87. In this embodiment, the flow directors 880 are the same as the flow directors 880 described above in relation to Figures 64 and 65A. In that embodiment, the flow directors 880 are the same size and, therefore, provide the same gas flow rate. The flow director 880 in Figures 85 to 87 differ in that they are configured to provide a gas flow rate through one flow director 880 that is greater than a gas flow rate through another flow director 880. In other words, one of the respective flow directors 880 is configured with a first resistance to gas flow and another of the respective flow directors 880 is configured with a second resistance to gas flow and the first resistance to gas flow is lower than the second resistance to gas flow.

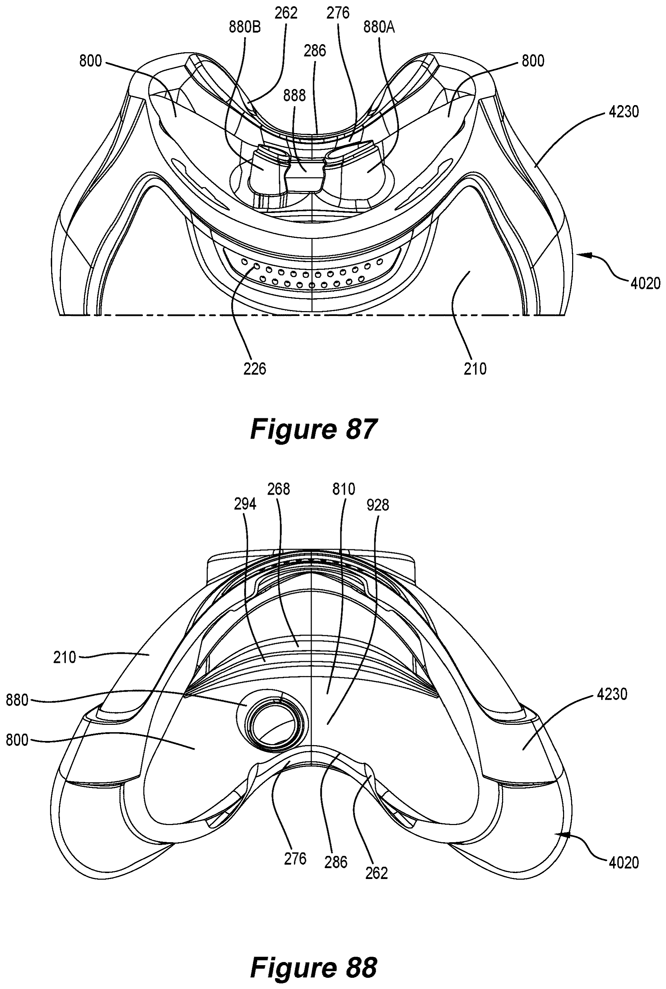

It can be seen from Figures 86 and 87 that the first opening 889A and the second opening 884A of the one of the respective flow directors 880A is larger than the first opening 889B and the second opening 884B respectively of the other of the respective flow director 880B. In alternative embodiments, first opening 889A or the second opening 884A of the flow director 880A is larger than the respective first opening 889B or the second opening 884B of the flow director 880B.

Expressed in another way, the asymmetry in the flow rate between the flow directors 880A and 880B is a result of different opening sizes of the flow directors 880A and 880B. For example, a ratio of the area of the first opening 889A of the flow director 880A to the area of first opening 889B of the other flow director 880B is in the range of 1:1 to 1:0.1. Alternatively, a ratio of the area of the second opening 884A of the flow director 880A to the area of second opening 884B of the other flow director 880B is in the range of 1:1 to 1:0.1. Further alternatively, a ratio of the combined area of the first opening 889A and the second opening 884A of the flow director 880A to the combined area of the first opening 889B and the second opening 884B of the other flow director 880B is in the range of 1:1 to 1:0.1. Alternatively, a ratio of the area of the second opening of the one of the respective flow directors to the area of the second opening of the another of the respective flow directors is in the range of 1:0.5 to 1:0.2. Alternatively, the ratio of the area of the second opening of the one of the respective flow directors to the area of the second opening of the another of the respective flow directors is 1:0.33. In a further alternative, the two flow directors 880A and 880B have the same shape and the ratio of the dimension of the flow director 880A to the dimensions of the flow director 880B is in the range of 1:0.5 to 1:0.2. The ratio of the dimension of the flow director 880A to the dimensions of the flow director 880B may be 1:0.33.

Despite the size difference between the flow directors 880A and 880B, the orientation and location of the flow directors 880A and 880B and the spacing rib 888 remains the same as for the flow directors 880 in the embodiment shown in Figure 64.

The flow director 880A with the larger area of the first and second openings 889A, 884B, has a lower resistance to gas flow and, therefore has a higher gas flow than the gas flow through the flow director 880B. The higher gas flow through the flow director 880A directs more gas flow to one nostril of the patient than to the other nostril. In other words, there is asymmetric flushing flow provided to both nostrils. It is anticipated that the higher gas flow to the one nostril will encourage one-way flushing of dead space in the nasal cavity by a stream of respiratory gas flowing in the one nostril and out the other nostril. While flushing flow is provided to both nares from the respective flow directors 880A and 880B, less flow enters the nare associated with flow director 880B with the result that there is asymmetric flushing flow through the nares and the nasal cavity. The outflow through the other nostril is thought to be facilitated by the relatively lower gas flow rate through the flow director 880B associated with that nostril. That is, the lower gas flow rate directed to the other nostril provides less resistance to gas flow out of the same nostril. It is thought that the one-way flushing of dead space in the nasal cavity may improve anatomical dead space flushing when a patient's mouth is closed. It is also though that the one-way flushing will still be effective at least to some extent when the patient's mouth is open.

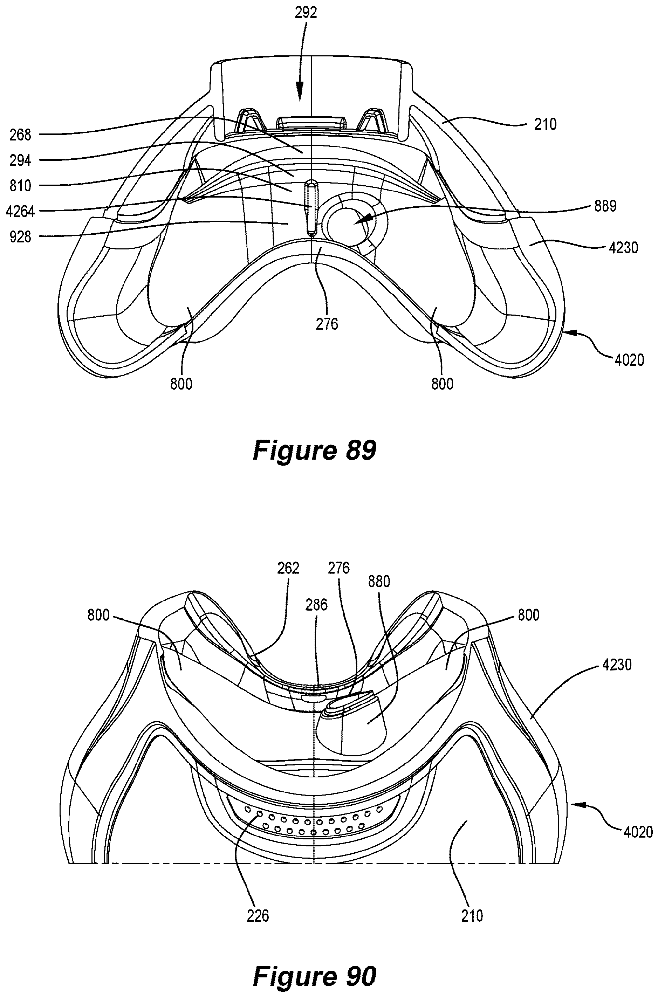

An alternative to having two flow directors 880A and 880B of different sizes involves the patient interface including only one flow director 880, as shown in Figures 88 to 90.

In this embodiment, the one flow director 880 is configured to deliver a flushing flow of respiratory gas to one nare. That is, the flow director 880 is configured to direct a flow of gas through the nare opening and into one of the patient's nares. The one flow director 880 is configured to enable flushing of dead space in the nasal cavity by delivering the flushing flow to the one nare. It is anticipated that delivering a flushing flow of gas via the one flow director 880 to one nare is likely to cause one-way flushing of the dead space in the nasal cavity with the flushing flow flowing through the nasal cavity and exiting through the other nare into the second chamber 240.

While the flow director 880 shown in Figures 88 to 90 has the form of the flow director 880 described above in relation for Figures 64, 65A and 65B, the flow director may be in the form of any one of the flow directors disclosed above. That is, the flow director may be in the form of any one of the flow directors 246, 1246, 2246, 3246, 4246, 820, 830, 840, 850, 860 or 870.

In Figures 88 to 90, the second opening 884 of the flow director 880 is off-set laterally from a mid-plane between the lateral sides of the patient interface 10. Also, the second opening 884 of the flow director 880 is spaced laterally from the mid-plane. Similarly, the first opening 889 of the flow director 880 is off-set laterally from a mid-plane through the patient interface 10. Also, the first opening 889 of the flow director 880 is spaced laterally from a mid-plane through the patient interface 10.

The dividing wall 4242 on the opposite side of the mid-plane to the flow director 880 includes a first lateral portion 928 that is adjacent to the mid-plane. The first lateral portion 928 coincides with where the second of a pair of flow directors 880 would be. The first lateral portion 928 has a first wall thickness. The dividing wall 4242 further includes one of the lateral side portions 800 extending laterally outwardly from the first lateral portion 928. The lateral side portion 800 has a second wall thickness and the first wall thickness of the first lateral portion 928 is the same as the second wall thickness. In an alternative embodiment, the first wall thickness of the first lateral portion 928 is greater than as the second wall thickness.

In accordance with that alternative embodiment, a transition 930 between the first lateral portion 928 and the lateral side portion 800 is laterally spaced from the mid-plane by a distance that is substantially the same as the distance that the laterally-most positioned part of the flow director 880 is spaced from the mid-plane. Alternatively, the transition 930 between the first lateral portion and the lateral side portion 800 is laterally spaced from the mid-plane by a distance that is greater than the distance that the laterally-most positioned part of the flow director 880 is spaced from the mid-plane. In a further alternative embodiment, the transition 930 between the first lateral portion and the lateral side portion 800 is laterally spaced from the mid-plane by a distance that is less than the distance that the laterally-most positioned part of the flow director 880 is spaced from the mid-plane.

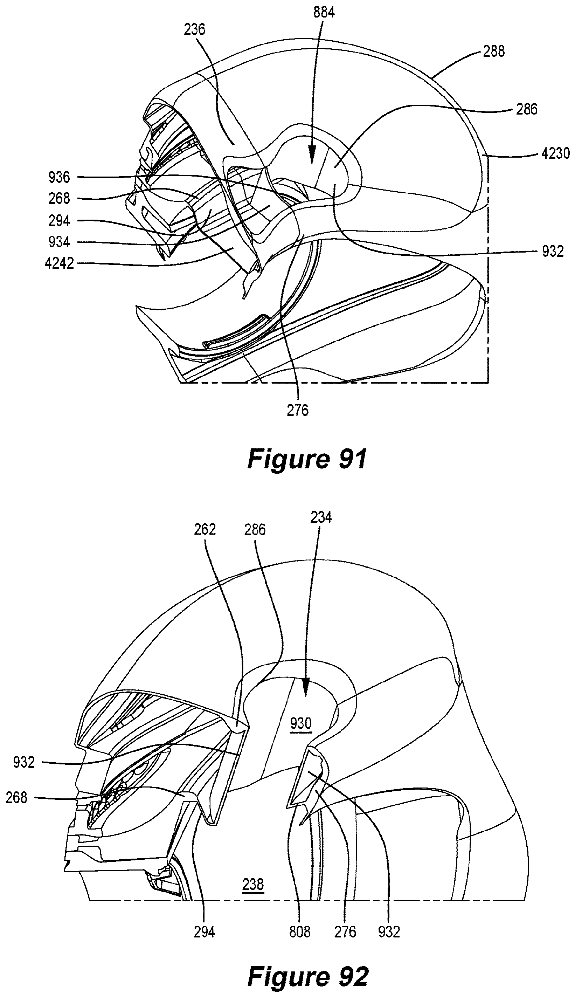

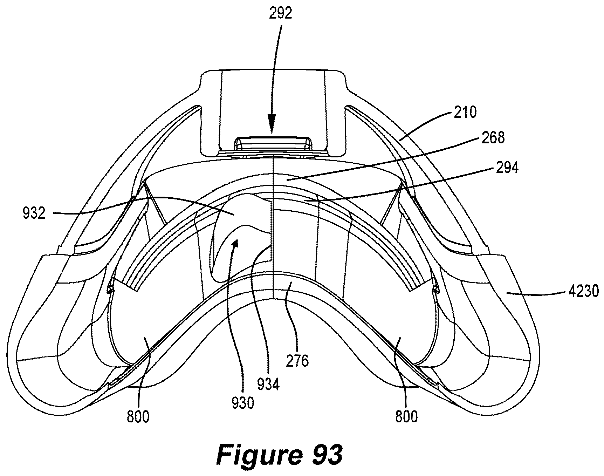

Figures 91 to 93 show a variation of the embodiment shown in Figures 88 to 90. In Figures 91 to 93, the patient interface 10 includes the nare opening 234 that seals about the nares of a patient and includes a channel 930 extending from the first chamber 238 to the nare opening 234 and which channel is configured to align with one of the patient's nares. The remainder of the nare opening 234 is configured to align with the other of the patient's nares. This embodiment is anticipated to operate in the same way as the embodiment described above with only one flow director (Figures 88 to 90). That is, the channel 934 and the nare opening 234 are configured to deliver a flushing flow of gas to a first nare when the interface is fitted to a patient and which flushing flow causes flushing of the nasal cavity and causes gas flushed through the nasal cavity to flow through the other nare and through the remainder of the nare opening 234 and into the second chamber 240. In this way, this embodiment is anticipated to provide one-way flushing of the nasal cavity.

In this embodiment, the channel 930 is configured to enable gas from the other of the patient's nares to flow into the second chamber 240. Additionally, the channel 930 is configured to enable gas from the channel 930 to flow into the second chamber 240.

The channel 930 may be configured to deliver gas flow only to one of the patient's nares. However, in this embodiment, the channel 930 is configured to deliver gas flow to both of the patient's nares. The gas flow to the patient's nares comprise first and second gas flows from the channel 930 and the first and second gas flows are unequal. The first gas flow is greater than the second gas flow. The first gas flow to a first nare is the flushing gas flow. While both nostrils are clear, the flushing gas flow supplied to one nostril enables one-way anatomical deadspace flushing of the nasal cavity and upper throat as described above. In the event, that the nostril to which the flushing gas flow is supplied becomes blocked, the second gas flow supplies the patient with respiratory gas to continue the respiratory cycle.

The split in gas flow from the channel 930 is enable by the configuration for the channel 930. The channel 930 is defined in part by a perimeter wall 932 extending between the dividing wall 4242 and the nare opening 234 and in part by a partition wall 934 extending between the dividing wall and a position recessed from the nare opening 234 to form the channel 930 with the perimeter wall 932. The partition wall terminates at the outlet end of the channel in a partition edge 936. While the partition edge 936 links the rim 386 of the nare opening 234 on the proximal and distal sides of the rim 286, the partition edge 936 has a curved shape which is recessed from the nare opening 234. The recessed position of the partition wall 934 from the nare opening 234 enables gas from the channel 234 to flow into the second chamber 240 when the patient interface 10 is fitted to a patient. The partition wall 934 is recessed from the nare opening to avoid contact with the patient. That is, the partition wall 934 is recessed sufficiently from the nare opening 234 so that there is a gap for gas flow between the end of the partition wall 934 and the patient's septum when the patient interface 10 is fitted to a patient.

The channel 934 is tapered from the dividing wall 4242 to the nare opening 234 to accelerate gas as it travels through the channel 934. In other embodiments, the profile of the channel 934 is constant from the dividing wall 4242 to the nare opening 234.

Another alternative flow director 890 is shown in Figures 83 and 84. The flow director 890 has the same general form as the flow director 880 in that it includes a base 891, a body 892 extending from the base 891 and a rim 893. The base 891 joins the body 892 to the dividing wall 4242. The base 891 forms a smooth transition surface between the body 892 and in the dividing wall 4242. Like the flow director 880, part of the outer wall 288 extends over the laterally outer sides of a second opening 894 defined by the rim 893. As explained above in respect of the cushion module 4020, the nare opening 234 expands laterally when the patient interface 10 is fitted so that the nare opening 234 extends laterally outwardly so that the outer wall 288 doesn't extend over the second openings 894 in use.

The body 892 includes a transition 895 which delineates a lower body portion 896 from the rim 893. Unlike the flow director 880, the flow director 890 doesn't include an upper body portion. The lower body portion 896 has a wall thickness which decreases from the base 891 to the transition 895. The lower body portion 896 is angled in the proximal direction so the proximal side of the lower body portion 896 extending from the base 891 to the transition 895 is shorter than the distal side of the lower body portion 896 extending from the base 891 to the transition 895. The transition 895 occupies a plane that is inclined in the same manner as the lower body portion 896. However, the inclination of the lower body portion 896 and the transition 895 is less that the inclination present in the flow director 4246. The angling of the lower body portion 896 and the lateral positioning of the flow directors 890 facilitates direction of respiratory gas through the nare opening 234 and into the nares from the flow directors 890. The flow director 890 also differs from the flow director 4246 in that the transition 895 is closer to the dividing wall 4242 than the transition 802 for the flow director 4246.

The lower body portion 896 has a wall thickness and the rim 893 has another wall thickness that is less than the wall thickness of the lower body portion so that the transition 895 has a tapering wall thickness.

In this embodiment, the spacing rib 898 joins with the lower body portion 896 and the transition 895 at a position spaced from the rim 893. The spacing rib 898 is disposed distally of a notional line connecting the centres of the second openings 894 of the respective flow directors 890. The base 891 or lower body portion 896 of the flow directors 890 joins with the wall portion 276.

An inner wall of the flow director 890 tapers inwardly from the first opening 899 toward the second opening 894 to form the inwardly tapered channel 930 between the first opening 899 and the second opening 894. In this embodiment, the wall thickness of the rim 893 is greater than the wall thickness of the lateral side portion 800, the distal portion 810 or the proximal portion 808 of the dividing wall.

In this embodiment, the patient interface 10 includes one or more deformation region ties 918 that extends between the side of the dividing wall 4242 that is exposed to the first chamber 238 and the outer wall 288 of the seal member 4230, the main portion 244 of the dividing wall 4242 has a concave profile in the proximal-distal direction relative to the nare opening 234, and the or each respective flow director 890 comprises a base 891, a lower body portion 896, a rim 893 which forms a second opening 894 that opens into the second chamber 240 and a transition 895 extending between the lower body portion 896 and the rim 893.

For all of the embodiments of flow directors described above, the area of the second opening is in the range of 20 to 160 mm² or 40 to 140 mm² or 40 to 100 mm² or 40 to 80 mm² or 40 to 60 mm² or 30 to 80 mm² or 30 to 60 mm² or 20 to 60 mm² The only exception to this range is for the embodiment shown in Figures 85 to 87 and particularly in respect of the flow director 880B having the smaller size of the two asymmetrically sized flow directors 880A and 880B. The area of the second opening 884B of that smaller flow director may be less than the above ranges.

For all of the embodiments of flow directors described above, the gas velocity through the flow director is in the range of 1 to 40 m/s. It will be appreciated that the velocity is likely to differ for a given gas pressure supplied to the patient interface, but it is expected to fall within the range stated above. The anticipated gas velocity is based on the patient interface being fitted to a patient and without occlusion of the first or second chambers 238, 240 or the flow directors 246, 1246, 2246, 3246, 4246, 820, 830, 840, 850, 860, 870, 880 or 890.

As mentioned previously, the patient interface 10 further includes the frame 30 and the conduit connector 40.

The frame 30 includes the central body portion 302 that includes one or more channels for conveying respiratory gas from a gas source to the cushion module 20 and therefore to the patient. The frame 30 includes side wings 304 extending from the central body portion 302. Each side-wing 74 includes a pair of openings 308 are arrange to co-operate with headgear (such as resilient straps) for fitting the patient interface to a patient. The headgear operates by pulling the mask 10 into contact with the patient's face to form a substantially air-tight seal when respiratory gas at elevated gas pressure is delivered to the patient via the mask 10. One opening 308 on each side wing 304 includes a bar 306 for connecting with a headgear clip to allow for easier connection to and disconnection from headgear.

While this embodiment includes the frame 30, the headgear connection points may be integrated with or connected to the housing 210 in alternative embodiments. If so, the frame 30 is not necessary and could be omitted from such embodiments.

The frame 30 further includes a connector sleeve 310 that includes four arcuate fingers 312. The connector sleeve 312 has an inner wall 314 which includes a concave profile having the shape of shape of a spherical segment. The outer wall of the connector sleeve 310 is shaped to fit within the sleeve 220 of the housing 210. The arcuate fingers 312 are spaced by detents which are shaped to fit with the key formations 222. The location of the key formations 222 and the detents ensures that the frame aligns correctly with the housing 210 when they are fitted together.

Each arcuate finger 312 has an end with an arcuate flange portion 318 which forms a snap-fit with a radially inwardly projecting lip 298 of the sleeve 220. The snap-fit holds the frame 30 to the housing 210. The snap-fit may be releasable or may be a permanent fit between the frame 30 and the housing 210.

Having regard to the comments above regarding variations on the general form shown in Figure 1, one such variation of the general form, and which is applicable to the aspects and embodiments described below, is where the housing 210 and the frame 30 are formed integrally. In other words, the patient interface 10 may include a unitary structure that performs the same function of the housing 210 and frame 30. While the housing 210 and the frame 30 are described as being separate components of the patient interface 10, the description should be read as including the option of an integrally formed component that functions in the same way as the housing 210 and frame 30. Alternatively, the frame 30 may be connected to the housing 210 by any conventional means, such as with adhesives or welding. For example, the frame 30 may be permanently connect to the housing 210 by ultrasonically welding the housing 210 and the frame 30.

The conduit connector 40 includes an elbow 60 and a socket insert 50 which couples the elbow 60 to the frame 30. The conduit connector 40 further includes a swivel connector 80 which connects to a conduit that delivers respiratory gas from a source, such as a ventilator, a humidifier or a wall source. The swivel connector 80, elbow 60 and socket insert 50 form a flow path for respiratory gas into the cushion module 20, 1020, 2020, 3020 from a conduit.

The socket insert 50 has an outer wall 502 that includes a convex spherical segment. The shape of the spherical segment fits with the shape of the concave spherical segment of the inner wall 314 of the frame 30. The outer wall 502 is joined at one end to an inner wall 504. The inner wall 504 includes an inner surface 506 that includes radially inwardly projecting shoulder 508. The socket insert 50 snap-fits with the frame 30 such that the outer wall 502 seats in the concave segment of the inner wall 314 of the frame 30 (Figure 4). This connection forms a ball and socket style connection which allows the socket to rotate within the spherical segment of the inner wall 314 of the frame 30.

The elbow 60 includes a first conduit portion 602 and a second conduit portion 608. Longitudinal axes of the first conduit portion 602 and the second conduit portion 608 are set at an oblique angle. Accordingly, respiratory gas flowing through the elbow 60 undergoes a change of direction from the first conduit portion 602 to the second conduit portion 608. The conduit portion 602 includes a radially projecting flange 606. The elbow 60 is connected to the socket insert 50 by snap fitting the flange 606 with the shoulder 508. In other embodiments, however, the elbow 60 may connected to the socket insert 50 by welding or by adhesive, in which case the shoulder 508 and the flange may be omitted.

The second conduit portion 608 includes an inlet 610 for respiratory gas. The inlet 610 is at the distal end of the second conduit portion 608. The second conduit portion 608 further includes a structure (see Figure 5) that co-operates with an anti-asphyxiation valve 70 (Figures 2, 4 and 5) to permit ambient air into the patient interface if the source of respiratory gas fails or the conduit for conveying the gas from the source to the patient interface 10 becomes obstructed. More specifically, the second conduit portion 608 includes an opening 612. The opening is in a lower portion of the second conduit portion 608. A spine 614 is disposed adjacent the opening 612 and supports a panel 616 that is spaced from the opening 612. The spacing of the panel from the opening creates a gap 618 through which ambient air can access the opening.

The anti-asphyxiation valve 70 includes a valve seat 702 and a valve seal 714. The valve seat 702 includes a sealing surface 704 against which the valve seal 714 seals the valve 70. The valve seat 702 further includes a sleeve 708 with a radially outwardly projecting bead 710. The bead 710 is at the end of the sleeve 708. The valve seat 702 further includes a spigot 712 for coupling with the valve seal 714.

The valve seal 714 includes a flap 720 which can transition between an open position in which the elbow 60 is open to flow of respiratory gas from a source and a closed position in which the elbow 60 is closed to flow of respiratory gas from a source. In the open position, access of ambient air to the inside of the elbow 60 is inhibited and in the closed position, access of ambient air to the inside of the elbow 60 is permitted. In this embodiment, the flap 720 is formed of a flexible material. The flap 720 is joined to a lug 716 by a hinge 722. The hinge 722 comprises a section of flexible material with a reduced wall thickness. The lug 716 is configured to assist with locating the valve seal 714 within the end of the second conduit portion 608. Additionally, the lug 716 includes a recess 718 which is adapted to receive the spigot 712. Mating of the spigot 712 within the recess 718 correctly orients the valve seal 714 on the valve seat 702.

When pressurised respiratory gas is supplied from a source, it flows through the elbow 60 and into the cushion module 20, 1020, 2020, 3020. The elevated pressure of the respiratory gas causes the flap to swing about the hinge 722 to cover the opening 612 in the second conduit portion. This represents the "open position" described above in that the flap 720 prevents ambient air from entering the elbow 60 via the gap 618 and the opening 612. In the event that the source of respiratory gas fails or the conduit connecting to the source becomes obstructed, the anti-asphyxiation valve 70 closes because the air pressure in the elbow 60 equalises with the air pressure outside the elbow 60 so that the flap 720 transitions to the "closed position" described above owing to the inherent resilience in the flexible material which forms the hinge 722. In the closed position, the opening 612 is revealed to the interior of the elbow 60 so that the natural breathing cycle of the patient will draw air into the elbow 60 and the cushion module 20, 1020, 2020, 3020 via the one or more openings as shown by the flow arrows in Figure 5.

The valve seat 702 includes a radially projecting step which is configured to couple with the elbow 60. In particular, the step 724 is configured to fit with the end of the second conduit portion 608. The coupling may comprise a snap-fit connection or may comprise a permanent fixing, such as welding or fixing with adhesive.

The valve seat 702 couples with a swivel connector 80 which is configured to connect with a conduit from a respiratory gas source. The swivel connector 80 includes a radially inwardly projecting shoulder 82 which is co-operable with the step 724 of the valve seat 714 to connect the valve seat 714 to the swivel connector 80. The connection is a snap-fit connection. In other embodiment, however, the connection may comprise a permanent fixing, such as welding or fixing with adhesive.

Those skilled in the art of the present invention will appreciate that many variations and modifications may be made to the preferred embodiment without departing from the spirit and scope of the present invention.

In the claims which follow, and in the preceding description, except where the context requires otherwise due to express language or necessary implication, the word "comprise" and variations such as "comprises" or "comprising" are used in an inclusive sense, i.e. to specify the presence of the stated features but not to preclude the presence or addition of further features in various embodiments of the apparatus and method as disclosed herein.

In the foregoing description of preferred embodiments, specific terminology has been resorted to for the sake of clarity. However, the invention is not intended to be limited to the specific terms so selected, and it is to be understood that each specific term includes all technical equivalents which operate in a similar manner to accomplish a similar technical purpose. Terms such as "front" and "rear", "inner" and "outer", "above", "below", "upper" and "lower", "underside" and "topside", "vertical" and "horizontal" and the like are used as words of convenience to provide reference points and are not to be construed as limiting terms. These terms when used in reference to the patient interface throughout the specification, including the claims, refer to orientations relative to the normal operating orientation, i.e. when the interface is fitted to a patient and the patient's head is upright.

Throughout the description and claims, terms such as "join", "link" and "connection" should not be construed as requiring two separate components being linked together. Those terms should be interpreted in context, including the option of meaning an intersection of integrally formed features. For example, the dividing wall in the above embodiments joins with the outer wall, but they are integrally formed in those embodiments.

Furthermore, invention has been described in connection with what are presently considered to be the most practical and preferred embodiments, it is to be understood that the invention is not to be limited to the disclosed embodiments, but on the contrary, is intended to cover various modifications and equivalent arrangements included within the spirit and scope of the invention. Also, the various embodiments described above may be implemented in conjunction with other embodiments, for example, aspects of one embodiment may be combined with aspects of another embodiment to realize yet other embodiments. Further, each independent feature or component of any given assembly may constitute an additional embodiment.

## Claims

1. A non-invasive patient interface which is configured to seal about the mouth and nares of a patient, the patient interface including:
an outer wall defining an interior volume of the patient interface, the outer wall having one or more oral openings which communicate gas with the mouth and one or more nare openings which communicate gas with the nares;
a dividing wall that separates the interior volume into a first chamber having the one or more oral openings and a second chamber having the one or more nare openings; and
wherein the dividing wall includes one flow director which enables gas to flow into the second chamber from the first chamber and the flow director is configured to cause one-way flushing of dead space in the nasal cavity by a stream of respiratory gas flowing in one nostril and out the other nostril and the flow director is off-set laterally from a mid-plane between lateral sides of the patient interface, having regard to an upright orientation of the patient interface, and is configured to deliver a flow of respiratory gas to one nare.

2. The patient interface defined in claim 1, wherein the one flow director is configured to deliver an accelerated flow of respiratory gas to create a flushing flow.

3. The patient interface defined in claim 1 or claim 2, wherein the one flow director has a first opening to the first chamber and a second opening to the second chamber, the first and second openings enable gas to flow into the second chamber from the first chamber and which one flow director is configured to direct the gas flow through the one or more nare openings.

4. The patient interface defined in claim 3, wherein the first opening of the one flow director is off-set laterally from a mid-plane between lateral sides of the patient interface, having regard to an upright orientation of the patient interface.

5. The patient interface defined in claim 3 or claim 4, wherein the second opening of the one flow director is spaced laterally from a mid-plane between lateral sides of the patient interface, having regard to an upright orientation of the patient interface, and is spaced from the one or more nare openings.

6. The patient interface defined in any one of the preceding claims, wherein the one flow director has a base which joins with the dividing wall and which defines the first opening from the first chamber, a body extending from the base, a rim remote from the base and which rim defines the second opening which opens into the second chamber and a gas flow channel extending from the first opening in the base to the second opening.

7. The patient interface defined in claim 6, wherein the rim is contoured so that at least a portion of the rim has a substantially consistent spacing from the one or more nare openings.

8. The patient interface defined in claim 6 or claim 7, wherein the body includes a lower body portion and an upper body portion and the upper body portion includes a wall thickness that is less than a wall thickness of the lower body portion.

9. The patient interface defined in any one of claims 6 to 8, wherein the body has a conical profile, tapering profile or a stepped profile in cross-section generally orthogonal to the dividing wall where the one flow director is located.

10. The patient interface defined in any one of the preceding claims, wherein the dividing wall includes a preferential deformation region which decouples one portion of the dividing wall from another portion of the dividing wall such that the two portions can move relative to each other.

11. The patient interface defined in any one of the preceding claims, wherein the patient interface has an inlet to the first chamber for respiratory gas and an exhaust vent that is configured to communicate respiratory gas from the second chamber to externally of the patient interface.

12. The patient interface defined in any one of the preceding claims, wherein the patient interface further includes a patient-engagement surface and the dividing wall includes one or more deformation resistant sections configured to reduce the extent to which the one flow director deform due to deformation forces applied to the patient-engagement surface.

13. The patient interface defined in claim 12, wherein the one or more of the deformation resistant sections comprise portions of the dividing wall that have a wall thickness that is greater than the wall thickness of other portions of the dividing wall.

14. The patient interface defined in claim 12 or claim 13, wherein the one or more deformation resistant sections are in the region of the dividing wall that includes the one flow director.

15. The patient interface defined in any one of the preceding claims, wherein the outer wall is provided by a cushion module including a resilient seal member and a housing and wherein the seal member and the housing together form the first and second chambers.
